(19) Europäisches Patentamt / European Patent Office / Office européen des brevets

(11) **EP 4 721 764 A1**

(12) **EUROPEAN PATENT APPLICATION**
published in accordance with Art. 153(4) EPC

(43) Date of publication:
**08.04.2026 Bulletin 2026/15**

(21) Application number: **24814604.5**

(22) Date of filing: **31.05.2024**

(51) International Patent Classification (IPC):
***A61K 47/54** (2017.01)* ***C12N 15/113** (2010.01)*
***A61K 31/713** (2006.01)* ***C12N 15/11** (2006.01)*
***C07K 14/47** (2006.01)*

(52) Cooperative Patent Classification (CPC):
**A61K 31/713; A61K 47/54; C07K 14/47; C12N 15/11; C12N 15/113**

(86) International application number:
**PCT/CN2024/096731**

(87) International publication number:
**WO 2024/245410 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.05.2023 CN 202310628559**
**25.10.2023 CN 202311386740**

(71) Applicant: **Tuojie Biotech (Shanghai) Co., Ltd.**
**Shanghai 201203 (CN)**

(72) Inventors:
- **LI, Yunfei**
  **Shanghai 201203 (CN)**
- **ZHANG, Zhen**
  **Shanghai 201203 (CN)**
- **LIN, Xiaoyan**
  **Shanghai 201203 (CN)**
- **WANG, Yanhui**
  **Shanghai 201203 (CN)**

(74) Representative: **Dragotti & Associati S.P.A.**
**Via Nino Bixio, 7**
**20129 Milano (IT)**

Remarks:
The complete document including Reference Table(s) and the Sequence Listing(s) can be downloaded from the EPO website

(54) **DSRNA TARGETING LPA AND USE THEREOF**

(57) A dsRNA targeting LPA and the use thereof. A double-stranded ribonucleic acid (dsRNA) targeting LPA and a preparation method therefor. A pharmaceutical composition, cell or kit comprising the dsRNA. The dsRNA can interfere with the expression of LPA, and can prevent and/or treat related diseases.

EP 4 721 764 A1

## Description

The present disclosure claims priority to Chinese Patent Application No. 202310628559.8 filed on May 31, 2023 and Chinese Patent Application No. 202311386740.9 filed on October 25, 2023, which are incorporated herein by reference in their entirety.

## TECHNICAL FIELD

The present disclosure pertains to the field of biopharmaceutics and particularly relates to an LPA-targeting dsRNA, use thereof, and a preparation method therefor.

## BACKGROUND

Lipoprotein(a) [Lp(a)], first discovered by Norwegian geneticist Berg in 1963, was identified as a unique lipoprotein (Berg K. A new serum type system in man-the Lp system. Acta Pathol Microbiol Scand 1963; 59: 369-82). Lp(a) is composed of two parts, lipid and protein. The lipid part is mainly LDL-like particles located in the core, and the protein part is located in the periphery and is composed of apolipoprotein(a) [apo(a)] and apoB100 linked by a disulfide bond. Apo(a) is expressed predominantly in the liver and only in human beings and non-human primates and is characterized by the presence of three internal disulfide-stabilized tricyclic structural domains (Kringle). In the human lineage, the amplification and differentiation of the Kringle IV domain in apo(a) results in ten different types of KIV domains, with further amplification of Kringle IV type 2 (KIV-2) producing copy number variation (CNV) within multiple alleles (1-40 copies) and the other Kringle IV encoding domains (KIV-1 and KIV-3 to KIV-10) being present only as a single copy (Schmidt K, Noureen A, Kronenberg F, et al. Structure, Function, and Genetics of Lipoprotein(a), [J]. Journal of Lipid Research, 2016, 57(8):1339). All Kringles are transcribed and translated, and therefore KIV-2 CNV leads to the size polymorphism of apo(a) encoded; its expression is inversely proportional to the number of KIV-2 domains present, and the Lp(a) content in plasma increases significantly when the KIV-2 copy number is low.

Patients with elevated Lp(a) have a 2-3 times higher risk of suffering from cardiovascular events than normal people, and the cardiovascular events caused include atherosclerotic cardiovascular disease, lower extremity arterial disease, aortic stenosis, etc. (EnAs EA, Varkey B, Dharmarajan T S, et al. Lipoprotein(a): An independent, genetic, and causal factor for cardiovascular disease and acute myocardial infarction[J]. Indian Heart Journal, 2019, 71(2)). Lp(a) may lead to undesirable atherosclerotic cardiovascular disease (ASCVD) by two mechanisms: in one aspect, since apo(a) has been shown to inhibit fibrinolysis in vitro, it may promote thrombosis at a place where there is plaque rupture or turbulence at a place where there is vessel stenosis, leading to vessel occlusion or promoting thrombosis; in another aspect, LDL-like particles may promote intimal cholesterol deposition and inflammation or the formation of oxidized phospholipids, leading to atherosclerotic stenosis or aortic stenosis (Albert Youngwoo Jang, Seung Hwan Han, I1 Suk Sohn, et al. Lipoprotein(a) and Cardiovascular Diseases[J]. Circulation Journal, 2020, 84: 867-874). However, even at very high levels of Lp(a), the cholesterol level is below the traditional LDL threshold; therefore, the LDL-like particles may be less pathogenic in this aspect.

The 2016 Chinese Guidelines on Prevention and Treatment of Dyslipidemia in Adults defines an Lp(a) level of >30 mg/dL as abnormal, and based on this standard, about 30% of patients with a history of cardiovascular events in China have abnormal Lp(a). The National Lipid Association recommended an Lp(a) of ≥50 mg/dL as an elevated level in 2019, and based on this standard, 20% of the global population has an elevated level of Lp(a). Although the elevated level of Lp(a) is common, no targeted therapeutic agents are available, and no drugs for targeted lowering of Lp(a) have been approved for clinical use to date. Structurally similar to a plurality of lipoproteins, the Lp(a) protein is difficult to become a direct target of small-molecule and macromolecule drugs. However, mRNA transcribed from the Lp(a) gene has high specificity, and the regulation mechanism after siRNA transcription can be used to specifically degrade the mRNA, thereby inhibiting the expression of the Lp(a). An siRNA targeting the apo(a) gene (LPA) is therefore designed to attenuate its expression, thereby lowering the Lp(a) level in serum, which in turn reduces cardiovascular adverse events.

## SUMMARY

The present disclosure provides a double-stranded ribonucleic acid comprising a sense strand and an antisense strand; the sense strand and the antisense strand form a double-stranded region (preferably, the sense strand and the antisense strand are reverse complementary); the double-stranded ribonucleic acid targets the LPA gene or an expression product thereof.

In some embodiments, the sense strand and/or the antisense strand of the dsRNA comprise(s) at least one modified nucleotide.

The present disclosure provides an LPA-targeting double-stranded ribonucleic acid (dsRNA) comprising a sense

strand and an antisense strand that form a double-stranded region, wherein:

the unmodified nucleotide sequence of the sense strand comprises at least 17 contiguous nucleotides that differ from the nucleotide sequence of SEQ ID NO: 1 by no more than 3 nucleotides, and
the unmodified nucleotide sequence of the antisense strand comprises at least 19 contiguous nucleotides that differ from the nucleotide sequence of SEQ ID NO: 2 by no more than 3 nucleotides, wherein in the direction from the 5' end to the 3' end,
the nucleotides at positions 7, 8, and 9 of the sense strand are 2'-fluoro-modified nucleotides, and the nucleotides at the other positions are 2'-methoxy-modified nucleotides;
the nucleotides at positions 2 and 14 of the antisense strand are 2'-fluoro-modified nucleotides, the nucleotides at positions 4, 6, 10, 12, 16, and 18 are independently 2'-methoxy-modified or 2'-fluoro-modified nucleotides, and the nucleotides at the other positions are 2'-methoxy-modified nucleotides;
the number of 2'-fluoro-modified nucleotides in the antisense strand is 2-7 (e.g., 2, 3, 4, 5, 6, or 7).

In some embodiments, the unmodified nucleotide sequence of the sense strand of the dsRNA comprises or is selected from the group consisting of the nucleotide sequence set forth in SEQ ID NO: 1, and the unmodified nucleotide sequence of the antisense strand comprises or is selected from the group consisting of the nucleotide sequence set forth in SEQ ID NO: 2, wherein in the direction from the 5' end to the 3' end,

the nucleotides at positions 7, 8, and 9 of the sense strand are 2'-fluoro-modified nucleotides, and the nucleotides at the other positions are 2'-methoxy-modified nucleotides;
the nucleotides at positions 2 and 14 of the antisense strand are 2'-fluoro-modified nucleotides, the nucleotides at positions 4, 6, 10, 12, 16, and 18 are independently 2'-methoxy-modified or 2'-fluoro-modified nucleotides, and the nucleotides at the other positions are 2'-methoxy-modified nucleotides;
the number of 2'-fluoro-modified nucleotides in the antisense strand is 2-7 (e.g., 2, 3, 4, 5, 6, or 7).

In some embodiments, the sense strand and the antisense strand are provided, wherein:

the sense strand comprises 5'-GCUCCUUAUUGUUAUACGA-3', and
the antisense strand comprises 5'-UCGUAUAACAAUAAGGAGCUG-3'.

In some other embodiments, the sense strand and the antisense strand are provided, wherein:

the unmodified nucleotide sequence of the sense strand is 5'-GCUCCUUAUUGUUAUACGA-3', and
the unmodified nucleotide sequence of the antisense strand is 5'-UCGUAUAACAAUAAGGAGCUG-3';
the unmodified sequences refer to unmodified nucleotide sequences.

In some embodiments, the nucleotide at position 7 of the 5' end of the antisense strand of the dsRNA is a modified nucleotide, wherein the modified nucleotide is a 2'-methoxy-modified nucleotide.
In some embodiments, the nucleotide at position 7 of the 5' end of the antisense strand of the dsRNA is a modified nucleotide, wherein the modified nucleotide comprises a chemical modification represented by formula (I), (I-1), or (I-2) or a pharmaceutically acceptable salt thereof:

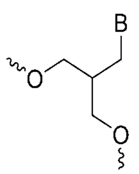 (I), 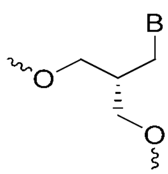 (I-1), or 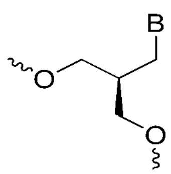 (I-2),

wherein B is the same as the base of the nucleotide at position 7 of the 5' end of the antisense strand when unmodified; in some specific embodiments, B is adenine.
In some embodiments, the chemical modification represented by formula (I'), (I'-1), or (I'-2) is selected from the group consisting of:

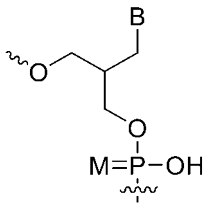 (I'), 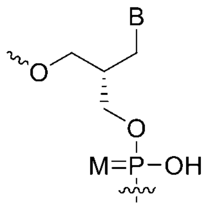 (I'-1), and 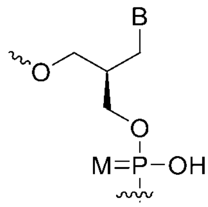 (I'-2),

wherein M is O or S; B is the same as the base of the nucleotide at position 7 of the 5' end of the antisense strand when unmodified; in some specific embodiments, B is adenine. In some specific embodiments, M is S. In some specific embodiments, M is O.

In some embodiments, the nucleotide at position 1 of the 5' end of the antisense strand of the dsRNA is a modified nucleotide, wherein the modified nucleotide is a 2'-methoxy-modified nucleotide.

In some embodiments, the nucleotide at position 1 of the 5' end of the antisense strand of the dsRNA is a modified nucleotide, wherein the modified nucleotide is a nucleotide comprising a chemical modification represented by formula (IV):

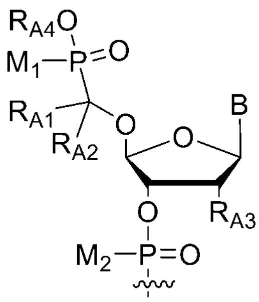

(IV)

wherein:

$R_{A1}$ and $R_{A2}$ are each independently selected from the group consisting of hydrogen and deuterium;
$M_1$ and $M_2$ are each independently selected from the group consisting of -SH and -OH;
B is selected from the group consisting of bases, hydrogen, and deuterium; $R_{A3}$ is selected from the group consisting of hydrogen, deuterium, hydroxy, halogen, alkyl (e.g., C1, C2, C3, C4, C5, or C6 alkyl, including but not limited to methyl, ethyl, and isopropyl), and alkoxy (e.g., C1 alkoxy, C2 alkoxy, C3 alkoxy, C4 alkoxy, C5 alkoxy, or C6 alkoxy, including but not limited to methoxy, ethoxy, propoxy, and isopropoxy), and the hydroxy, alkyl, and alkoxy are each optionally substituted with one or more deuterium atoms; $R_{A4}$ is selected from the group consisting of hydrogen, deuterium, and alkyl (e.g., C1, C2, C3, C4, C5, or C6 alkyl, including but not limited to methyl, ethyl, and isopropyl), and the alkyl is optionally substituted with one or more deuterium atoms; provided that formula (IV) comprises at least one deuterium atom.

In some embodiments, $R_{A3}$ is selected from the group consisting of hydrogen and deuterium.

In some other embodiments, $R_{A3}$ is selected from the group consisting of halogen (e.g., fluorine, chlorine, or bromine).

In some other embodiments, $R_{A3}$ is selected from the group consisting of alkyl (e.g., C1, C2, C3, C4, C5, or C6 alkyl, including but not limited to methyl, ethyl, and isopropyl), and the alkyl is optionally substituted with one or more deuterium atoms.

In some other embodiments, $R_{A3}$ is selected from the group consisting of alkoxy (e.g., C1 alkoxy, C2 alkoxy, C3 alkoxy, C4 alkoxy, C5 alkoxy, or C6 alkoxy, including but not limited to methoxy, ethoxy, propoxy, and isopropoxy), and the alkoxy is optionally substituted with one or more deuterium atoms.

In some embodiments, the 5'-end chemical modification represented by formula (IV) is:

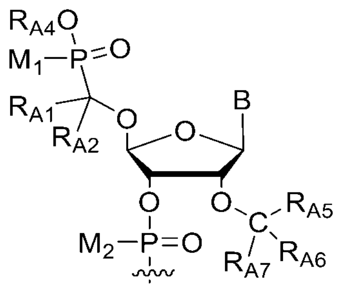

(IV-1) ,

wherein $R_{A5}$, $R_{A6}$, and $R_{A7}$ are each independently selected from the group consisting of hydrogen and deuterium; $R_{A1}$, $R_{A2}$, $M_1$, $M_2$, and B are as defined in formula (IV), provided that formula (IV-1) comprises at least one deuterium atom.

In some embodiments, $R_{A5}$, $R_{A6}$, and $R_{A7}$ are all deuterium.
In some embodiments, $R_{A5}$ is deuterium, and $R_{A6}$ and $R_{A7}$ are hydrogen.
In some embodiments, $R_{A5}$ and $R_{A6}$ are deuterium, and $R_{A7}$ is hydrogen.
In some embodiments, $R_{A1}$ is hydrogen, and $R_{A2}$ is deuterium.
In some embodiments, $R_{A1}$ and $R_{A2}$ are deuterium.
In some embodiments, $R_{A1}$ and $R_{A2}$ are hydrogen.
In some embodiments, the 5'-end chemical modification represented by formula (IV) is selected from the group consisting of:

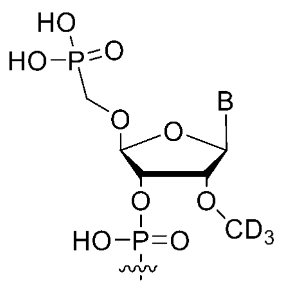 , 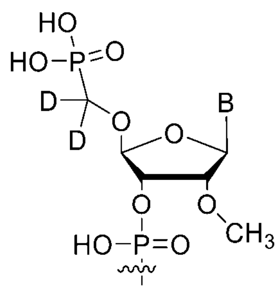 , 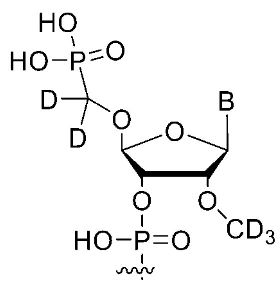 ,

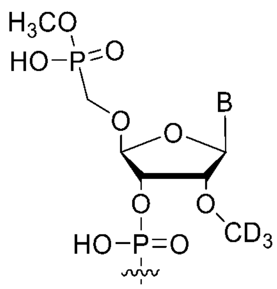 , 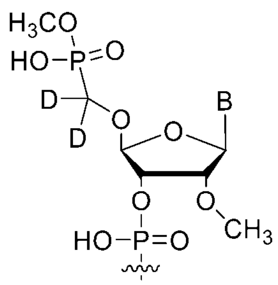 , 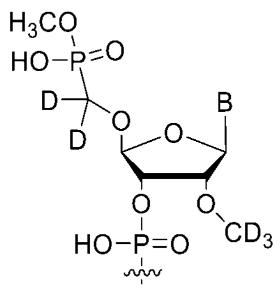 ,

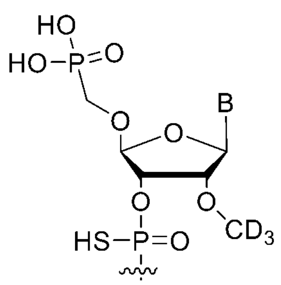 , 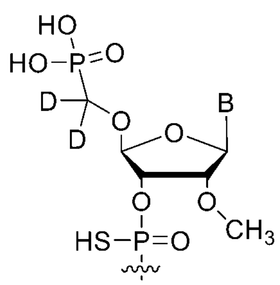 , 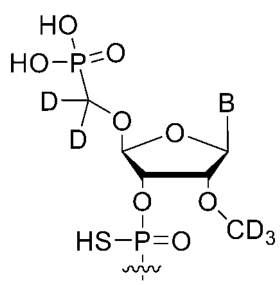 ,

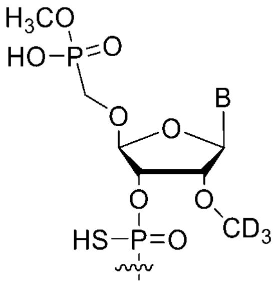 ,
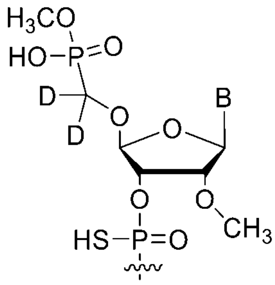 ,
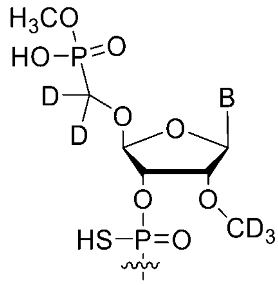 ,
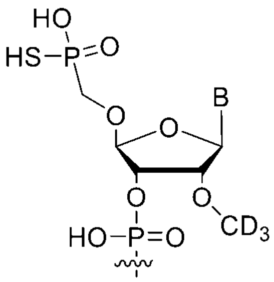 ,
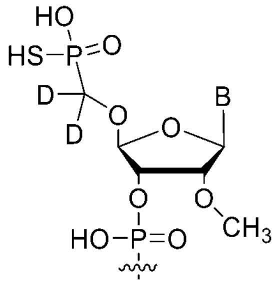 ,
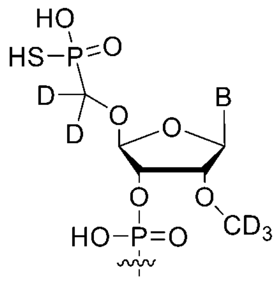 ,
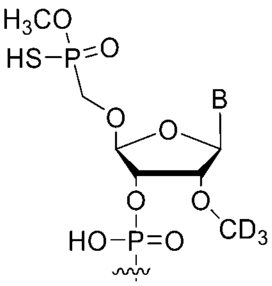 ,
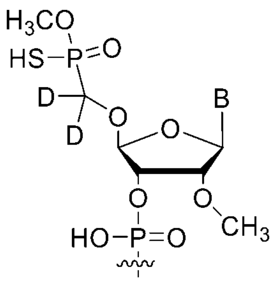 ,
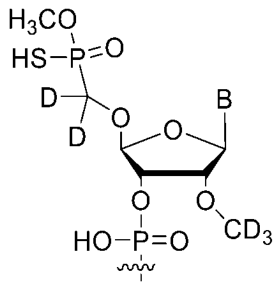 ,
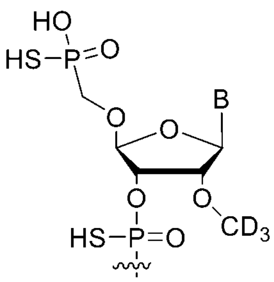 ,
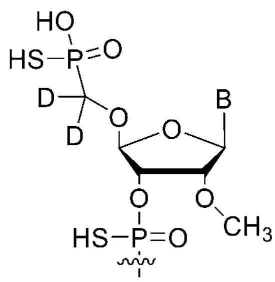 ,
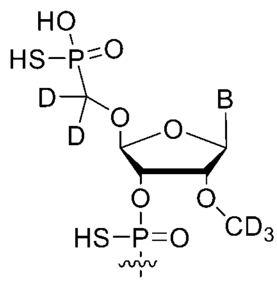 ,
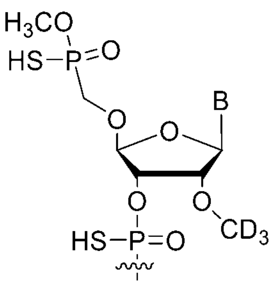 ,
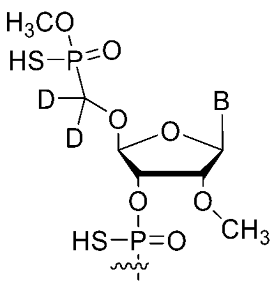 , and
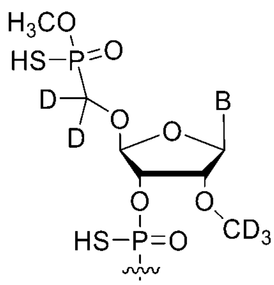 ,

wherein B is selected from the group consisting of bases and hydrogen.

In some embodiments, B is selected from the group consisting of bases; in some specific embodiments, B is selected from the group consisting of adenine, guanine, cytosine, uracil, and thymine.

In some specific embodiments, B is a base at a corresponding position of the modified nucleotide of the antisense strand.

In some specific embodiments, B is selected from the group consisting of uracil.

In some embodiments, the 5'-end chemical modification represented by formula (IV) is selected from the group consisting of:

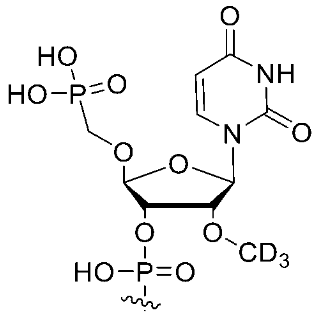
,
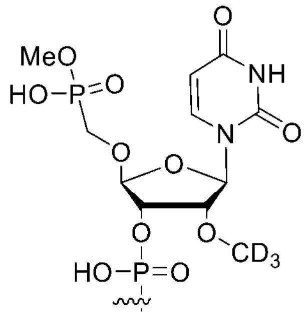
,
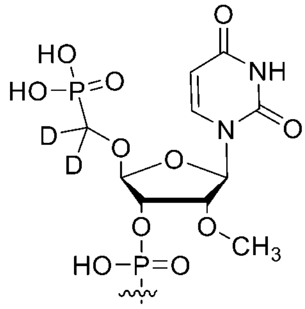
,

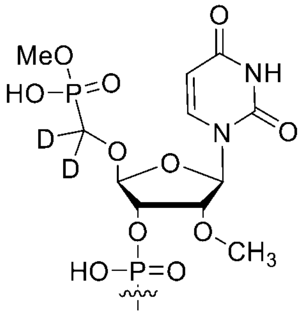
,
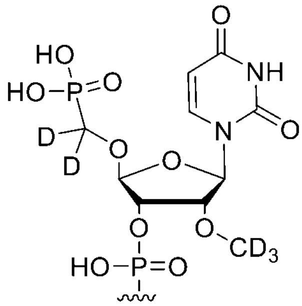
,
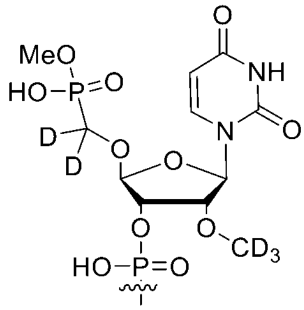
,

and a structure in which uracil is replaced with adenine, guanine, cytosine, or thymine.

In some embodiments, in the direction from the 5' end to the 3' end, the nucleotide at position 1 of the 5' end of the antisense strand of the dsRNA is a 5'-end chemically modified nucleotide represented by formula (IV). In some specific embodiments, the 5'- end chemically modified nucleotide represented by formula (IV) is

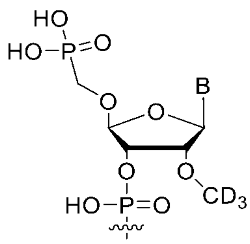

or

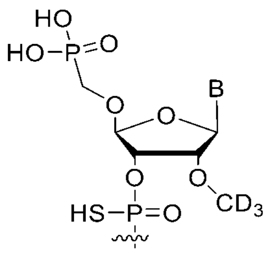
,

wherein B is a base at a corresponding position of the nucleotide at position 1 of the 5' end of the antisense strand.

In some embodiments, the nucleotide at position 1 of the 5' end of the antisense strand of the dsRNA is a modified nucleotide, wherein the modified nucleotide is a chemically modified nucleotide represented by formula (II):

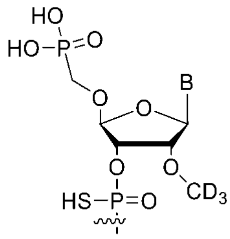

(II),

wherein B represents a base at a corresponding position of the nucleotide at position 1 of the 5' end of the antisense strand. In some specific embodiments, B represents uracil.

In some embodiments, the antisense strand is at least partially reverse complementary to a target sequence to mediate RNA interference. In some embodiments, there are no more than 5, no more than 4, no more than 3, no more than 2, or no more than 1 mismatch between the antisense strand and the target sequence; in some embodiments, the antisense strand is fully reverse complementary to the target sequence.

In some embodiments, the sense strand is at least partially reverse complementary to the antisense strand to form a double-stranded region; in some embodiments, there are no more than 5, no more than 4, no more than 3, no more than 2, or no more than 1 mismatch between the sense strand and the antisense strand; in some embodiments, the sense strand is fully reverse complementary to the antisense strand.

In some embodiments, the sense strand and the antisense strand each independently comprise 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24, or 19 to 23 nucleotides.

In some specific embodiments, the sense strand and the antisense strand each independently have a length of 19, 20, 21, 22, or 23 nucleotides.

In some embodiments, the sense strand and the antisense strand are identical or different in length; the sense strand is 19-23 nucleotides in length, and the antisense strand is 19-26 nucleotides in length. In some embodiments, the length ratio of the sense strand to the antisense strand may be 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, the length ratio of the sense strand to the antisense strand is 19/21, 21/23, or 23/25. In some embodiments, the length ratio of the sense strand to the antisense strand is 19/21.

In some embodiments, the dsRNA comprises one or two blunt ends.

In some embodiments, the dsRNA comprises an overhang comprising 1 to 4 unpaired nucleotides, e.g., 1, 2, 3, or 4 unpaired nucleotides.

In some embodiments, the dsRNA comprises an overhang at the 3' end of the antisense strand.

In some embodiments, the sense strand of the dsRNA comprises or is a nucleotide sequence represented by the following formula: $5'\text{-}N_aN_aN_aN_aN_aN_aN_bN_bN_bN_aN_aN_aN_aN_aN_aN_aN_aN_aN_{a\text{-}}3'$, wherein $N_a$ is a 2'-methoxy-modified nucleotide, and $N_b$ is a 2'-fluoro-modified nucleotide.

In some embodiments, the antisense strand of the dsRNA comprises or is a nucleotide sequence represented by the following formula:

$$5'\text{-}N_a'N_b'N_a'X'N_a'X'N_a'N_a'N_a'X'N_a'X'N_a'N_b'N_a'X'N_a'X'N_a'N_a'N_a'\text{-}3',$$

wherein each X' is independently $N_a'$ or $N_b'$; $N_a'$ is a 2'-methoxy-modified nucleotide, and $N_b'$ is a 2'-fluoro-modified nucleotide.

In some embodiments, the antisense strand of the dsRNA comprises or is a nucleotide sequence represented by the following formula:

$$5'\text{-}N_a'N_b'N_a'N_b'N_a'N_b'N_a'N_a'N_a'N_b'N_a'N_b'N_a'N_b'N_a'N_b'N_a'N_a'N_a'N_a'N_a'\text{-}3',$$

$$5'\text{-}N_a'N_b'N_a'N_b'N_a'N_b'N_a'N_a'N_a'N_b'N_a'N_a'N_a'N_b'N_a'N_b'N_a'N_b'N_a'N_a'N_a'\text{-}3',$$

$$5'\text{-}N_a'N_b'N_a'N_b'N_a'N_b'N_a'N_a'N_a'N_a'N_a'N_b'N_a'N_b'N_a'N_b'N_a'N_b'N_a'N_a'N_a'\text{-}3',$$

$$5'\text{-}N_a'N_b'N_a'N_a'N_a'N_b'N_a'N_a'N_a'N_b'N_a'N_b'N_a'N_b'N_a'N_b'N_a'N_b'N_a'N_a'N_a'\text{-}3',$$

$$5'\text{-}N_a'N_b'N_a'N_a'N_a'N_b'N_a'N_a'N_a'N_a'N_a'N_a'N_a'N_b'N_a'N_b'N_a'N_a'N_a'N_a'N_a'\text{-}3',$$

$5'\text{-}N_a'N_b'N_a'N_b'N_a'N_b'N_a'N_a'N_a'N_a'N_a'N_a'N_a'N_b'N_a'N_b'N_a'N_a'N_a'N_a'N_a'\text{-}3'$,

$5'\text{-}N_a'N_b'N_a'N_a'N_a'N_b'N_a'N_a'N_a'N_b'N_a'N_a'N_a'N_b'N_a'N_b'N_a'N_a'N_a'N_a'N_a'\text{-}3'$,

$5'\text{-}N_a'N_b'N_a'N_a'N_a'N_b'N_a'N_a'N_a'N_a'N_a'N_b'N_a'N_b'N_a'N_b'N_a'N_a'N_a'N_a'N_a'\text{-}3'$,

$5'\text{-}N_a'N_b'N_a'N_a'N_a'N_a'N_a'N_a'N_a'N_a'N_a'N_a'N_a'N_b'N_a'N_a'N_a'N_a'N_a'N_a'N_a'\text{-}3'$,

$5'\text{-}N_a'N_b'N_a'N_a'N_a'N_b'N_a'N_a'N_a'N_a'N_a'N_a'N_a'N_b'N_a'N_a'N_a'N_a'N_a'N_a'N_a'\text{-}3'$,

$5'\text{-}N_a'N_b'N_a'N_a'N_a'N_a'N_a'N_a'N_a'N_a'N_a'N_a'N_a'N_b'N_a'N_b'N_a'N_a'N_a'N_a'N_a'\text{-}3'$,

or

$5'\text{-}N_a'N_b'N_a'N_a'N_a'N_a'N_a'N_a'N_a'N_a'N_a'N_8'N_a'N_8'N_a'N_a'N_a'N_a'N_a'N_a'N_a'\text{-}3'$,

wherein $N_a'$ is a 2'-methoxy-modified nucleotide, and $N_b'$ is a 2'-fluoro-modified nucleotide.

In some embodiments, the antisense strand of the dsRNA comprises or is a nucleotide sequence represented by the following formula: $5'\text{-}N_a'N_b'N_a'X'N_a'X'W'N_a'N_a'X'N_a'X'N_a'N_b'N_a'X'N_a'X'N_a'N_a'N_a'\text{-}3'$, wherein each X' is independently $N_a'$ or $N_b'$; $N_a'$ is a 2'-methoxy-modified nucleotide, and $N_b'$ is a 2'-fluoro-modified nucleotide; W' represents a nucleotide comprising a chemical modification represented by formula (I), (I-1), or (I-2) or a pharmaceutically acceptable salt thereof. In some embodiments, the antisense strand of the dsRNA comprises or is a nucleotide sequence represented by the following formula:

$5'\text{-}N_a'N_b'N_a'N_b'N_a'N_b'W'N_a'N_a'N_b'N_a'N_b'N_a'N_b'N_a'N_b'N_a'N_a'N_a'N_a'N_a'\text{-}3'$,

$5'\text{-}N_a'N_b'N_a'N_b'N_a'N_b'W'N_a'N_a'N_b'N_a'N_a'N_a'N_b'N_a'N_b'N_a'N_b'N_a'N_a'N_a'\text{-}3'$,

$5'\text{-}N_a'N_b'N_a'N_b'N_a'N_b'W'N_a'N_a'N_a'N_a'N_b'N_a'N_b'N_a'N_b'N_a'N_b'N_a'N_a'N_a'\text{-}3'$,

$5'\text{-}N_a'N_b'N_a'N_a'N_a'N_d'W'N_a'N_a'N_d'N_a'N_8'N_a'N_d'N_a'N_b'N_a'N_8'N_a'N_a'N_a'\text{-}3'$,

$5'\text{-}N_a'N_b'N_a'N_a'N_a'N_b'W'N_a'N_a'N_a'N_a'N_a'N_a'N_8'N_a'N_b'N_a'N_a'N_a'N_a'N_a'\text{-}3'$,

$5'\text{-}N_a'N_b'N_a'N_b'N_a'N_b'W'N_a'N_a'N_a'N_a'N_a'N_a'N_b'N_a'N_b'N_a'N_a'N_a'N_a'N_a'\text{-}3'$,

$5'\text{-}N_a'N_b'N_a'N_a'N_a'N_b'W'N_a'N_a'N_b'N_a'N_a'N_a'N_b'N_a'N_b'N_a'N_a'N_a'N_a'N_a'\text{-}3'$,

$5'\text{-}N_a'N_b'N_a'N_a'N_a'N_b'W'N_a'N_a'N_a'N_a'N_b'N_a'N_b'N_a'N_b'N_a'N_a'N_a'N_a'N_a'\text{-}3'$,

$5'\text{-}N_a'N_b'N_a'N_a'N_a'N_a'W'N_a'N_a'N_a'N_a'N_a'N_a'N_b'N_a'N_a'N_a'N_a'N_a'N_a'N_a'\text{-}3'$,

$5'\text{-}N_a'N_b'N_a'N_a'N_a'N_b'W'N_a'N_a'N_a'N_a'N_a'N_a'N_b'N_a'N_a'N_a'N_a'N_a'N_a'N_a'\text{-}3'$,

or

$5'\text{-}N_a'N_b'N_a'N_a'N_a'N_a'W'N_a'N_a'N_a'N_a'N_a'N_a'N_d'N_a'N_8'N_a'N_a'N_a'N_a'N_a'\text{-}3'$,

wherein $N_a'$ is a 2'-methoxy-modified nucleotide, and $N_b'$ is a 2'-fluoro-modified nucleotide; W' represents a nucleotide comprising a chemical modification represented by formula (I), (I-1), or (I-2) or a pharmaceutically acceptable salt thereof.

In some embodiments, the antisense strand of the dsRNA comprises or is a nucleotide sequence represented by the following formula: $5'\text{-}V'N_b'N_a'X'N_a'X'N_a'N_a'N_a'X'N_a'X'N_a'N_b'N_a'X'N_a'X'N_a'N_a'N_a'\text{-}3'$, wherein each X' is independently $N_a'$ or $N_b'$; $N_a'$ is a 2'-methoxy-modified nucleotide, and $N_b'$ is a 2'-fluoro-modified nucleotide; V' represents a chemically modified nucleotide represented by formula (II).

In some embodiments, the antisense strand of the dsRNA comprises or is a nucleotide sequence represented by the following formula:

$5'\text{-}V'N_b'N_a'N_a'N_a'N_b'N_a'N_a'N_a'N_a'N_a'N_d'N_a'N_8'N_a'N_8'N_a'N_a'N_a'N_a'N_a'\text{-}3'$,

or

$$5'\text{-}V'N_b'N_a'N_a'N_a'N_a'N_a'N_a'N_a'N_a'N_a'N_d'N_a'N_8'N_a'N_a'N_a'N_a'N_a'N_a'N_a'\text{-}3',$$

wherein $N_a'$ is a 2'-methoxy-modified nucleotide, and $N_b'$ is a 2'-fluoro-modified nucleotide; V' represents a chemically modified nucleotide represented by formula (II). In some embodiments, the chemical modification represented by formula (I), (I-1), or (I-2) or the pharmaceutically acceptable salt thereof is selected from the group consisting of:

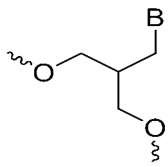,
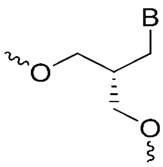, and
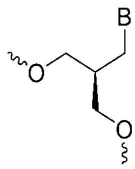,

wherein B represents the base at position 7 of the 5' end of the antisense strand; in some specific embodiments, B is adenine.

In some embodiments, the chemical modification represented by formula (I'), (I'-1), or (I'-2) or the pharmaceutically acceptable salt thereof is selected from the group consisting of:

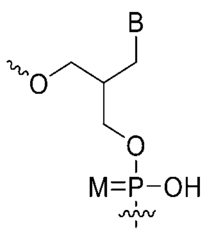,
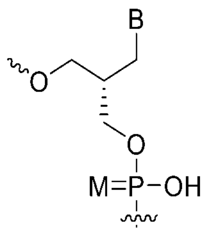, and
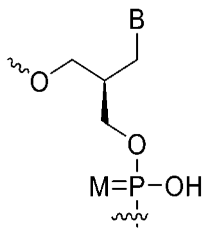,

wherein M is O or S; B represents the base at position 7 of the 5' end of the antisense strand; in some specific embodiments, B is adenine.

In some embodiments, M is S. In some specific embodiments, M is O.

In some embodiments, the chemically modified nucleotide represented by formula (II) is selected from the group consisting of:

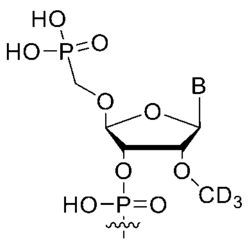
and
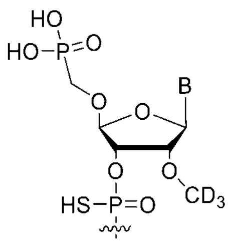,

wherein B represents a base at a corresponding position of the nucleotide at position 1 of the 5' end of the antisense strand. In some specific embodiments, B represents uracil.

In some embodiments, the chemically modified nucleotide represented by formula (II) is selected from the group consisting of:

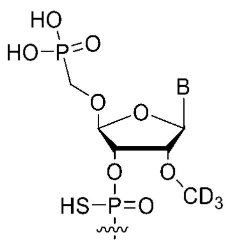,

wherein B represents a base at a corresponding position of the nucleotide at position 1 of the 5' end of the antisense strand. In some specific embodiments, B represents uracil.

In some embodiments, at least one phosphodiester group in the sense strand and/or the antisense strand of the dsRNA is a phosphodiester group with a modification group.

In some embodiments, at least one phosphodiester group in the sense strand and/or the antisense strand of the dsRNA is a phosphorothioate diester group.

In some embodiments, the phosphorothioate diester group is present at at least one of the following positions:

between the first and second nucleotides of the 5' end of the sense strand;
between the second and third nucleotides of the 5' end of the sense strand;
between the first and second nucleotides of the 3' end of the sense strand;
between the first and second nucleotides of the 5' end of the antisense strand;
between the second and third nucleotides of the 5' end of the antisense strand;
between the first and second nucleotides of the 3' end of the antisense strand; and
between the second and third nucleotides of the 3' end of the antisense strand.

In some embodiments, the sense strand and/or the antisense strand comprise(s) a plurality of phosphorothioate diester groups, and the phosphorothioate diester groups are present:

between the first and second nucleotides of the 5' end of the sense strand; and
between the second and third nucleotides of the 5' end of the sense strand; and
between the first and second nucleotides of the 3' end of the sense strand; and
between the first and second nucleotides of the 5' end of the antisense strand; and
between the second and third nucleotides of the 5' end of the antisense strand; and
between the first and second nucleotides of the 3' end of the antisense strand; and
between the second and third nucleotides of the 3' end of the antisense strand.

In some embodiments, the sense strand and/or the antisense strand comprise(s) a plurality of phosphorothioate diester groups, and the phosphorothioate diester groups are present:

between the first and second nucleotides of the 5' end of the sense strand; and
between the second and third nucleotides of the 5' end of the sense strand; and
between the first and second nucleotides of the 5' end of the antisense strand; and
between the second and third nucleotides of the 5' end of the antisense strand; and
between the first and second nucleotides of the 3' end of the antisense strand; and
between the second and third nucleotides of the 3' end of the antisense strand.

In some embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in any one of SEQ ID NO: 16, SEQ ID NO: 20, SEQ ID NO: 22 to SEQ ID NO: 33, and SEQ ID NO: 35.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 16.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 20.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 22.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 23.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 24.

In some specific embodiments, the sense strand of the dsRNA comprises or is selected from the group consisting of the nucleotide sequence set forth in SEQ ID NO: 6, and the antisense strand comprises or is selected from the group

consisting of the nucleotide sequence set forth in SEQ ID NO: 25.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 26.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 27.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 28.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 29.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 30.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 31.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 32.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 33.

In some specific embodiments, the sense strand of the dsRNA comprises or is selected from the group consisting of the nucleotide sequence set forth in SEQ ID NO: 6, and the antisense strand comprises or is selected from the group consisting of the nucleotide sequence set forth in SEQ ID NO: 35.

In some embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in any one of SEQ ID NO: 9 to SEQ ID NO: 20 and SEQ ID NO: 33 to SEQ ID NO: 35.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 9.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 10.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 11.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 12.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 13.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 14.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 15.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 16.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 17.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 18.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 19.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 20.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 33.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 34.

In some specific embodiments, the sense strand of the dsRNA comprises or is selected from the group consisting of the nucleotide sequence set forth in SEQ ID NO: 7, and the antisense strand comprises or is selected from the group consisting of the nucleotide sequence set forth in SEQ ID NO: 35.

In some embodiments, the dsRNA is as follows:
the nucleotide sequence of the sense strand comprises SEQ ID NO: 6, and the nucleotide sequence of the antisense strand comprises SEQ ID NO: 16.

In some embodiments, the dsRNA is as follows: the nucleotide sequence of the sense strand is set forth in SEQ ID NO: 6, and the nucleotide sequence of the antisense strand is set forth in SEQ ID NO: 16. The present disclosure further provides a dsRNA conjugate comprising any one of the dsRNAs described above and a targeting ligand linked to the dsRNA.

In some embodiments, the dsRNA and the targeting ligand are linked covalently or non-covalently.

In some embodiments, the targeting ligand targets the liver. In some embodiments, the targeting ligand binds to an asialoglycoprotein receptor (ASGPR). In some embodiments, the targeting ligand comprises a galactose cluster or a galactose derivative cluster, wherein the galactose derivative is selected from the group consisting of N-acetyl-galactosamine, N-trifluoroacetylgalactosamine, N-propionylgalactosamine, N-n-butyrylgalactosamine, and N-isobutyrylgalactosamine.

In some embodiments, the targeting ligand is linked to the 3' end of the sense strand of the dsRNA.

In some embodiments, the targeting ligand is linked to an end of the dsRNA by a phosphodiester group, a phosphorothioate diester group, or a phosphonic acid group. In some embodiments, the targeting ligand is linked to an end of the dsRNA by a phosphodiester group.

In some embodiments, the targeting ligand is indirectly linked to an end of the dsRNA by a phosphodiester group, a phosphorothioate diester group, or a phosphonic acid group. In some embodiments, the targeting ligand is indirectly linked to an end of the dsRNA by a phosphodiester group.

In some embodiments, the targeting ligand is directly linked to an end of the dsRNA by a phosphodiester group, a phosphorothioate diester group, or a phosphonic acid group. In some embodiments, the targeting ligand is directly linked to an end of the dsRNA by a phosphodiester group.

In some embodiments, the targeting ligand is directly linked to an end of the sense strand of the dsRNA by a phosphodiester group or a phosphorothioate diester group. In some embodiments, the targeting ligand is linked to an end of the sense strand of the dsRNA by a phosphodiester group.

In some embodiments, the targeting ligand is directly linked to the 3' end of the sense strand of the dsRNA by a phosphodiester group or a phosphorothioate diester group. In some embodiments, the targeting ligand is directly linked to the 3' end of the sense strand of the dsRNA by a phosphodiester group.

In some embodiments, to promote entry of the dsRNA into a cell, a lipophilic group such as cholesterol may be introduced to an end of the sense strand of the dsRNA, and the lipophilic group may be covalently bonded to a small interfering nucleic acid; for example, cholesterol, lipoprotein, vitamin E, or the like is introduced to the end to facilitate the crossing of the cell membrane consisting of a lipid bilayer and the interaction with the mRNA in the cell. Meanwhile, the dsRNA may also be modified by non-covalent bonding, for example, binding to a phospholipid molecule, a polypeptide, a cationic polymer, or the like via a hydrophobic bond or an ionic bond to increase stability and biological activity.

In some embodiments, the targeting moiety of the targeting ligand consists of one or more targeting groups or targeting moieties, and the targeting ligand assists in directing the delivery of a therapeutic agent linked thereto to the desired target location. In some cases, the targeting moiety may bind to a cell or cellular receptor and initiate endocytosis to promote entry of the therapeutic agent into the cell. The targeting moiety may comprise a compound with affinity for a cellular receptor or a cell surface molecule or an antibody. Various targeting ligands comprising targeting moieties may be

linked to therapeutic agents and other compounds to target the agents at cells and specific cellular receptors.

In some embodiments, the types of the targeting moieties include carbohydrates, cholesterol and cholesterol groups, or steroids. Targeting moieties capable of binding to cellular receptors include saccharides such as galactose, galactose derivatives (e.g., N-acetyl-galactosamine, N-trifluoroacetylgalactosamine, N-propionylgalactosamine, N-n-butyrylgalactosamine, and N-isobutyrylgalactosamine), mannose, and mannose derivatives).

Targeting moieties that bind to asialoglycoprotein receptors (ASGPRs) are known to be particularly used for directing the delivery of oligomeric compounds to the liver. Asialoglycoprotein receptors are extensively expressed on liver cells (hepatocytes). The targeting moieties of cellular receptors targeting ASGPRs include galactose and galactose derivatives. Specifically, clusters of galactose derivatives, including clusters consisting of 2, 3, 4, or more than 4 N-acetyl-galactosamine (GalNAc or NAG) molecules, can promote the uptake of certain compounds in hepatocytes. The GalNAc cluster coupled to the oligomeric compound is used for directing the composition to the liver, where the N-acetyl-galactosamine saccharide can bind to the asialoglycoprotein receptors on the liver cell surface. It is believed that the binding to the asialoglycoprotein receptors will initiate receptor-mediated endocytosis, thereby promoting entry of the compound into the interior of the cell.

In some embodiments, the targeting ligand may comprise 2, 3, 4, or more than 4 targeting moieties.

In some embodiments, each targeting moiety independently comprises a galactosamine derivative that is N-acetyl-galactosamine. Other sugars that may be used as targeting moieties and that have affinity for asialoglycoprotein receptors may be selected from the group consisting of galactose, galactosamine, N-formyl-galactosamine, N-acetyl-galactosamine, N-propionyl-galactosamine, N-n-butyryl-galactosamine, N-isobutyrylgalactosamine, etc.

In some embodiments, the targeting ligand in the present disclosure comprises N-acetyl-galactosamine as a targeting moiety:

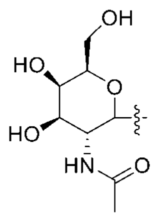
(H-2)

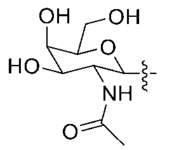
(H-2)

.

In some embodiments, the targeting ligand comprises three terminal galactosamines or galactosamine derivatives (such as N-acetyl-galactosamine), and each has affinity for asialoglycoprotein receptors. In some embodiments, the targeting ligand comprises three terminal N-acetyl-galactosamines (GalNAc or NAG) as targeting moieties.

In some embodiments, the targeting ligand comprises four terminal galactosamines or galactosamine derivatives (such as N-acetyl-galactosamine), and each has affinity for asialoglycoprotein receptors. In some embodiments, the targeting ligand comprises four terminal N-acetyl-galactosamines (GalNAc or NAG) as targeting moieties.

The terms commonly used in the art when referring to three terminal N-acetyl-galactosamines include tri-antennary, tri-valent, and trimer.

The terms commonly used in the art when referring to four terminal N-acetyl-galactosamines include tetra-antennary, tetra-valent, and tetramer.

In some embodiments, the targeting ligand provided by the present disclosure is the compound represented by formula (III-1) or a pharmaceutically acceptable salt thereof:

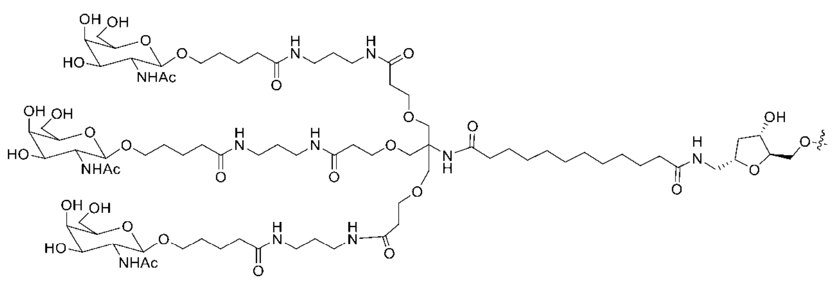
(III-1).

In some embodiments, the targeting ligand provided by the present disclosure is the compound represented by formula (III-2) or a pharmaceutically acceptable salt thereof:

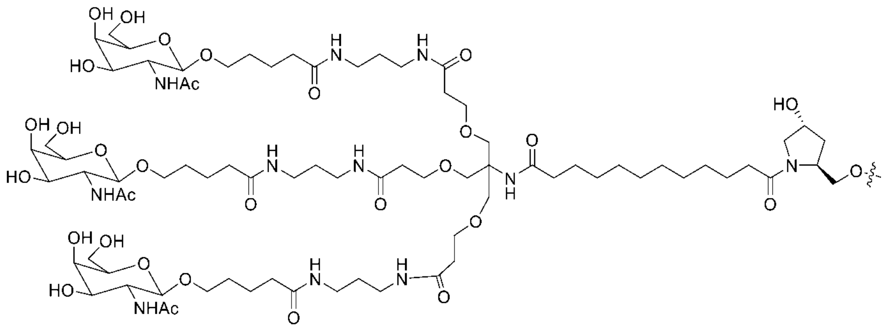

(III-2).

In some embodiments, the N-acetyl-galactosamine moiety in the above targeting ligands may be replaced with N-trifluoroacetylgalactosamine, N-propionylgalactosamine, N-n-butyrylgalactosamine, or N-isobutyrylgalactosamine.

In some embodiments, the nucleotide sequence of the sense strand of the dsRNA conjugate comprises or is selected from the group consisting of SEQ ID NO: 3, and the nucleotide sequence of the antisense strand comprises or is selected from the group consisting of the nucleotide sequence of any one of SEQ ID NO: 16, SEQ ID NO: 20, SEQ ID NO: 22 to SEQ ID NO: 33, and SEQ ID NO: 35.

In some embodiments, the nucleotide sequence of the sense strand of the dsRNA conjugate comprises or is selected from the group consisting of SEQ ID NO: 4, and the nucleotide sequence of the antisense strand comprises or is selected from the group consisting of the nucleotide sequence of any one of SEQ ID NO: 9 to SEQ ID NO: 20 and SEQ ID NO: 33 to SEQ ID NO: 35.

In some embodiments, the nucleotide sequence of the sense strand of the dsRNA conjugate comprises or is selected from the group consisting of SEQ ID NO: 5, and the nucleotide sequence of the antisense strand comprises or is selected from the group consisting of the nucleotide sequence of any one of SEQ ID NO: 16, SEQ ID NO: 20, and SEQ ID NO: 33.

In some embodiments, the dsRNA conjugate is as follows:
the nucleotide sequence of the sense strand comprises SEQ ID NO: 3, and the nucleotide sequence of the antisense strand comprises SEQ ID NO: 16.

In some embodiments, the dsRNA conjugate is as follows:
the nucleotide sequence of the sense strand is set forth in SEQ ID NO: 3, and the nucleotide sequence of the antisense strand is set forth in SEQ ID NO: 16.

In some embodiments, the dsRNA conjugate is the following structure or a pharmaceutically acceptable salt thereof:

sense strand

antisense strand

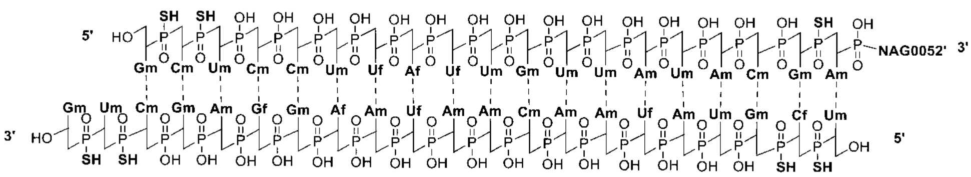

wherein Af = adenine 2'-F ribonucleoside; Cf = cytosine 2'-F ribonucleoside; Uf = uracil 2'-F ribonucleoside; Gf = guanine 2'-F ribonucleoside; Am = adenine 2'-OMe ribonucleoside; Cm = cytosine 2'-OMe ribonucleoside; Gm = guanine 2'-OMe ribonucleoside; Um = uracil 2'-OMe ribonucleoside.

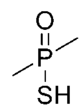

represents a phosphorothioate diester group;

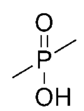

represents a phosphodiester group;

NAG0052' represents

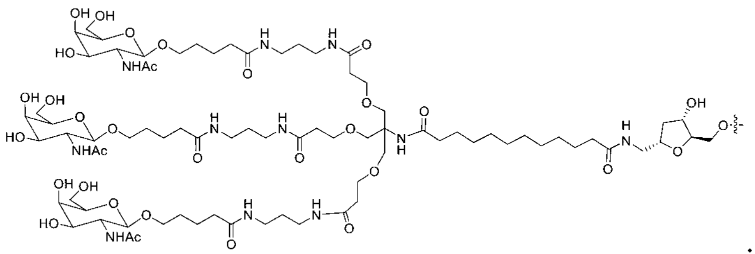

.

In some embodiments, the dsRNA conjugate is the following structure or a pharmaceutically acceptable salt thereof:

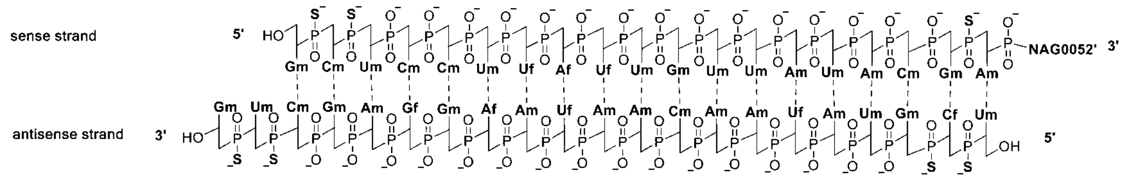

wherein Af = adenine 2'-F ribonucleoside; Cf = cytosine 2'-F ribonucleoside; Uf = uracil 2'-F ribonucleoside; Gf = guanine 2'-F ribonucleoside; Am = adenine 2'-OMe ribonucleoside; Cm = cytosine 2'-OMe ribonucleoside; Gm = guanine 2'-OMe ribonucleoside; Um = uracil 2'-OMe ribonucleoside.

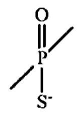

represents an anionic form of a phosphorothioate diester group;

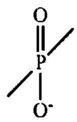

represents

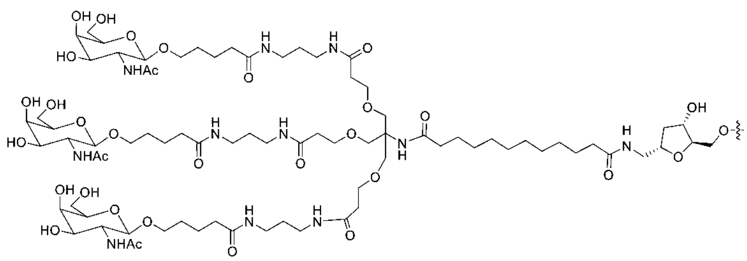

.

an anionic form of a phosphodiester group;

NAG0052' represents

In some embodiments, the pharmaceutically acceptable salt may be a conventional salt in the art, including but not limited to sodium salts, potassium salts, ammonium salts, amine salts, etc.

In some embodiments, the dsRNA conjugate is selected from the group consisting of any one of TJR100422, TJR100423, TJR100424, TJR100425, TJR100426, TJR100427, TJR100428, TJR100429, TJR100430, TJR100431, TJR100432, TJR100800, TJR100801, TJR100802, TJR100803, TJR100804, TJR100805, TJR100806, TJR100807, TJR100808, TJR100809, TJR100810, TJR100811, TJR101079, TJR101080, TJR101081, TJR101082, TJR101083, TJR101085,

TJR101086, TJR101087, TJR101088, TJR101084, TJR102134, and TJR102136.

In some embodiments, the dsRNA conjugate is TJR101079, which has a structure of:

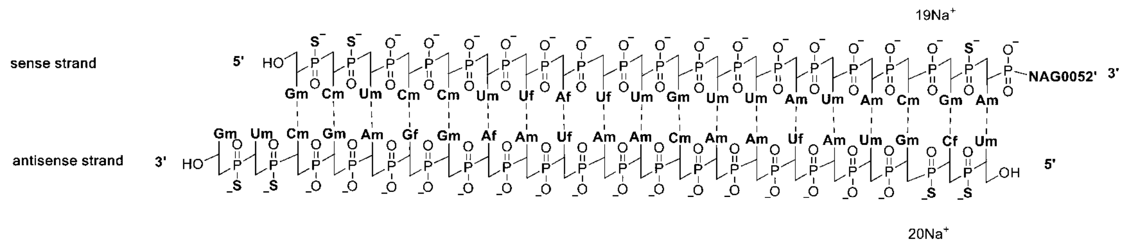

wherein Af = adenine 2'-F ribonucleoside; Cf = cytosine 2'-F ribonucleoside; Uf = uracil 2'-F ribonucleoside; Gf = guanine 2'-F ribonucleoside; Am = adenine 2'-OMe ribonucleoside; Cm = cytosine 2'-OMe ribonucleoside; Gm = guanine 2'-OMe ribonucleoside; Um = uracil 2'-OMe ribonucleoside.

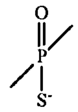

represents an anionic form of a phosphorothioate diester group;

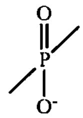

represents an anionic form of a phosphodiester group;

NAG0052' represents

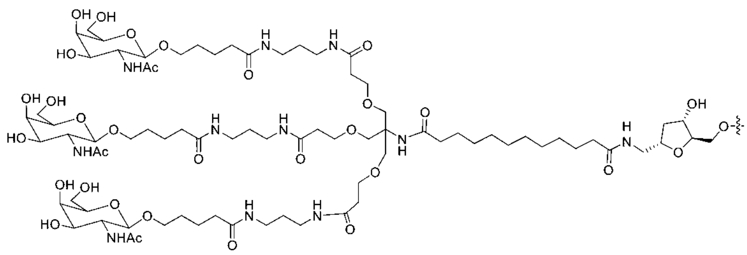

.

In another aspect, the present disclosure provides a composition comprising the dsRNA and/or the dsRNA conjugate described above, and one or more pharmaceutically acceptable excipients, such as a carrier, a vehicle, a diluent, and/or a delivery polymer. In the present disclosure, various delivery systems are known and can be used for the dsRNA and/or the dsRNA conjugate of the present disclosure, e.g., encapsulation in liposomes, microparticles, microcapsules, recombinant cells capable of expressing the compound, receptor-mediated endocytosis, and construction of a nucleic acid as part of a retroviral vector or other vectors.

In another aspect, the present disclosure provides use of the dsRNA and/or the dsRNA conjugate or the composition thereof described above in the manufacture of a medicament for treating a disease in a subject; in some embodiments, the disease is selected from the group consisting of a hepatic disease.

In another aspect, the present disclosure provides a method for treating a disease in a subject, and the method comprises administering to the subject the dsRNA and/or the dsRNA conjugate or the composition described above.

In another aspect, the present disclosure provides a method for inhibiting the expression of mRNA of LPA in a subject, and the method comprises administering to the subject the dsRNA and/or the dsRNA conjugate or the composition described above.

In another aspect, the present disclosure provides a method for delivering an expression-inhibiting oligomeric compound to the liver *in vivo,* and the method comprises administering to a subject the conjugate and/or the composition described above.

The dsRNA, dsRNA conjugate, composition, and methods disclosed herein can lower the level of a target mRNA in a cell, a cell population, a tissue, or a subject, which comprises: administering to the subject a therapeutically effective amount of the dsRNA described herein. The dsRNA is linked to a targeting ligand, thereby inhibiting the expression of the target mRNA in the subject.

In some embodiments, it has been previously identified that the subject has abnormal expression of the target gene in the targeted cell or tissue.

The subject described in the present disclosure refers to a subject having a disease or disorder that would benefit from the reduction or inhibition of the expression of the target mRNA.

Delivery can be accomplished by topical administration (e.g., direct injection, implantation, or topical application), systemic administration, or through subcutaneous, intravenous, intraperitoneal, or parenteral routes, including intracranial (e.g., intraventricular, intraparenchymal, and intrathecal), intramuscular, transdermal, airway (aerosol), nasal, oral, rectal, or topical (including buccal and sublingual) administration. In an optional embodiment, the pharmaceutical composition provided by the present disclosure may be administered by injection, e.g., intravenous, intramuscular, intradermal, subcutaneous, intraduodenal, or intraperitoneal injection.

In an optional embodiment, after the targeting ligand and the dsRNA are linked to form a conjugate, the conjugate can be packaged in a kit.

In another aspect, the present disclosure provides a pharmaceutical composition comprising the dsRNA and/or the dsRNA conjugate described in the present disclosure. In some embodiments, the pharmaceutical composition may further comprise a pharmaceutically acceptable auxiliary material and/or adjuvant, and the auxiliary material may be one or more of the various formulations or compounds conventionally used in the art. For example, the pharmaceutically acceptable auxiliary material may include at least one of a pH buffer, a protective agent, and an osmotic pressure regulator.

In some embodiments, when the dsRNA, the dsRNA conjugate, or the pharmaceutical composition thereof described in the present disclosure is in contact with a target gene-expressing cell, the dsRNA, the dsRNA conjugate, or the pharmaceutical composition described above inhibits the expression of the target gene by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the dsRNA, the dsRNA conjugate, or the pharmaceutical composition thereof described in the present disclosure is in contact with a target gene-expressing cell, the dsRNA, the dsRNA conjugate, or the pharmaceutical composition described above results in a remaining expression percentage of the target gene mRNA of no greater than 99%, no greater than 95%, no greater than 90%, no greater than 85%, no greater than 80%, no greater than 75%, no greater than 70%, no greater than 65%, no greater than 60%, no greater than 55%, no greater than 50%, no greater than 45%, no greater than 40%, no greater than 35%, no greater than 30%, no greater than 25%, no greater than 20%, no greater than 15%, or no greater than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the dsRNA, the dsRNA conjugate, or the pharmaceutical composition thereof described in the present disclosure is in contact with a target gene-expressing cell, the dsRNA, the dsRNA conjugate, or the pharmaceutical composition thereof, while retaining on-target activity, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the dsRNA, the dsRNA conjugate, or the pharmaceutical composition thereof described in the present disclosure is in contact with a target gene-expressing cell, the dsRNA, the dsRNA conjugate, or the pharmaceutical composition thereof, while reducing on-target activity by at most 20%, at most 19%, at most 15%, at most 10%, at most 5%, or more than 1%, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the dsRNA and/or the dsRNA conjugate or the pharmaceutical composition thereof described in the present disclosure is in contact with a target gene-expressing cell, the dsRNA, the dsRNA conjugate, or the pharmaceutical composition thereof, while increasing on-target activity by at least 1%, at least 5%, at least 10%, at least 15%, at least 20%, at least 25%, at least 30%, at least 35%, at least 40%, at least 45%, at least 50%, at least 55%, at

least 60%, at least 65%, at least 70%, at least 75%, or at least 80%, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In yet some other specific embodiments, when the dsRNA and/or the dsRNA conjugate or the pharmaceutical composition thereof described in the present disclosure is in contact with a target gene-expressing cell, the $IC_{50}$ is not greater than 1 nM, not greater than 0.9 nM, not greater than 0.8 nM, not greater than 0.7 nM, not greater than 0.6 nM, not greater than 0.5 nM, not greater than 0.4 nM, not greater than 0.3 nM, not greater than 0.2 nM, not greater than 0.19 nM, not greater than 0.18 nM, not greater than 0.17 nM, not greater than 0.16 nM, not greater than 0.15 nM, not greater than 0.14 nM, not greater than 0.13 nM, not greater than 0.12 nM, not greater than 0.11 nM, not greater than 0.10 nM, not greater than 0.09 nM, not greater than 0.08 nM, not greater than 0.07 nM, not greater than 0.06 nM, not greater than 0.05 nM, not greater than 0.04 nM, not greater than 0.03 nM, not greater than 0.02 nM, not greater than 0.01 nM, or a range between any two numerical values.

The present disclosure further provides a cell comprising the dsRNA and/or the dsRNA conjugate of the present disclosure.

The present disclosure further provides a kit comprising one or more containers, wherein the containers independently comprise the dsRNA and/or the dsRNA conjugate or the pharmaceutical composition thereof of the present disclosure.

The present disclosure further provides a method for silencing a target gene or mRNA of the target gene in a cell, and the method comprises a step of introducing into the cell the dsRNA and/or the dsRNA conjugate and/or the pharmaceutical composition according to the present disclosure.

The present disclosure further provides a method for silencing a target gene or mRNA of the target gene in a cell *in vivo* or *in vitro,* and the method comprises a step of introducing into the cell the dsRNA and/or the dsRNA conjugate and/or the pharmaceutical composition according to the present disclosure.

The present disclosure further provides a method for inhibiting the expression of a target gene or mRNA of the target gene, and the method comprises administering to a subject in need thereof an effective amount or effective dose of the dsRNA and/or the dsRNA conjugate and/or the pharmaceutical composition according to the present disclosure.

In some embodiments, administration is performed through administration modes including intramuscular, intrabronchial, intrapleural, intraperitoneal, intraarterial, lymphatic, intravenous, subcutaneous, cerebrospinal, or combinations thereof.

In some embodiments, the effective amount or effective dose of the dsRNA and/or the dsRNA conjugate and/or the pharmaceutical composition is about 0.001 mg/kg body weight to about 200 mg/kg body weight, about 0.01 mg/kg body weight to about 100 mg/kg body weight, or about 0.5 mg/kg body weight to about 50 mg/kg body weight.

In some embodiments, the target gene is LPA.

In another aspect, the present disclosure provides use of the dsRNA and/or the dsRNA conjugate or the pharmaceutical composition comprising the dsRNA and/or the dsRNA conjugate in the manufacture of a medicament.

In another aspect, the present disclosure provides the aforementioned dsRNA and/or pharmaceutical composition and/or dsRNA conjugate for use in treating and/or preventing a disease associated with elevated lipoprotein(a) and/or apolipoprotein(a) levels in a subject. In some embodiments, the disease associated with elevated lipoprotein(a) and/or apolipoprotein(a) levels is selected from the group consisting of a cardiovascular disease. In some embodiments, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

In another aspect, the present disclosure provides the aforementioned dsRNA and/or pharmaceutical composition and/or dsRNA conjugate for use in treating and/or preventing a disease, wherein the disease is selected from the group consisting of a cardiovascular disease. In some embodiments, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides the aforementioned dsRNA and/or pharmaceutical composition and/or dsRNA conjugate for use in lowering lipoprotein(a) and/or apolipoprotein(a) levels.

The present disclosure provides use of the aforementioned dsRNA and/or pharmaceutical composition and/or dsRNA conjugate in the manufacture of a medicament for inhibiting LPA expression.

The present disclosure provides use of the aforementioned dsRNA and/or pharmaceutical composition and/or dsRNA conjugate in the manufacture of a medicament for treating and/or preventing a disease associated with elevated lipoprotein(a) and/or apolipoprotein(a) levels in a subject. In some embodiments, the disease associated with elevated lipoprotein(a) and/or apolipoprotein(a) levels is selected from the group consisting of a cardiovascular disease. In some embodiments, the cardiovascular disease is selected from the group consisting of ischemic stroke, atherosclerosis,

thrombosis, coronary heart disease, lower extremity arterial disease or aortic stenosis, myocardial infarction, coronary stenosis, carotid stenosis, femoral artery stenosis, and heart failure. The present disclosure provides use of the aforementioned dsRNA and/or pharmaceutical composition and/or dsRNA conjugate in the manufacture of a medicament for treating and/or preventing a disease, wherein the disease is selected from the group consisting of a cardiovascular disease. In some embodiments, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides use of the aforementioned dsRNA and/or pharmaceutical composition and/or dsRNA conjugate in the manufacture of a medicament for lowering lipoprotein(a) and/or apolipoprotein(a) levels.

The present disclosure provides a method for inhibiting LPA expression, and the method comprises administering to a subject an effective amount or effective dose of the aforementioned dsRNA and/or pharmaceutical composition and/or dsRNA conjugate.

The present disclosure provides a method for treating and/or preventing a disease associated with elevated lipoprotein(a) and/or apolipoprotein(a) levels in a subject, and the method comprises administering to the subject an effective amount or effective dose of the aforementioned dsRNA and/or pharmaceutical composition and/or dsRNA conjugate. In some embodiments, the disease associated with elevated lipoprotein(a) and/or apolipoprotein(a) levels is selected from the group consisting of a cardiovascular disease; in some embodiments, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides a method for treating and/or preventing a disease, and the method comprises administering to a subject an effective amount or effective dose of the aforementioned dsRNA and/or pharmaceutical composition and/or dsRNA conjugate, wherein the disease is selected from the group consisting of a cardiovascular disease. In some embodiments, the cardiovascular disease is selected from the group consisting of ischemic stroke, atherosclerosis, thrombosis, coronary heart disease, lower extremity arterial disease or aortic stenosis, myocardial infarction, coronary stenosis, carotid stenosis, femoral artery stenosis, and heart failure.

The present disclosure provides a method for lowering lipoprotein(a) and/or apolipoprotein(a) levels, and the method comprises administering to a subject an effective amount or effective dose of the aforementioned dsRNA and/or pharmaceutical composition and/or dsRNA conjugate.

The present disclosure provides a method for delivering a dsRNA that inhibits LPA expression and/or replication to the liver *in vivo,* and the method comprises administering to a subject the aforementioned dsRNA and/or pharmaceutical composition and/or dsRNA conjugate.

In another aspect, the present disclosure further provides a method for preparing the dsRNA and/or the dsRNA conjugate or the pharmaceutical composition described in the present disclosure, and the method comprises: synthesizing the dsRNA and/or the dsRNA conjugate or the pharmaceutical composition described in the present disclosure.

The present disclosure further provides a dsRNA or a dsRNA conjugate, wherein one or more bases U, e.g., 1, 2, 3, 3, 5, 6, 7, 8, 9, or 10 bases U, of any dsRNA or dsRNA conjugate of the present disclosure are replaced with bases T. In some embodiments, all of the bases U in the present disclosure may be replaced with bases T.

The pharmaceutically acceptable salts of the compounds described in the present disclosure are selected from the group consisting of inorganic salts and organic salts. The compounds described in the present disclosure can react with acidic or basic substances to form corresponding salts.

The compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

In the chemical structures of the compounds of the present disclosure, the bond "" indicates an unspecified configuration; that is, if chiral isomers exist in the chemical structures, the bond "" may be "" or "", or includes both the configurations "" and "" simultaneously. In the chemical structures of the compounds of the present disclosure, the bond "" does not specify a configuration; that is, the configuration of the bond "" may be an E configuration or a Z configuration, or includes both the E configuration and the Z configuration simultaneously.

In the chemical structural formulas of the present disclosure, "" or "" may be linked to any group or groups in accordance with the scope of the invention described herein.

When the configuration is not specified, the compounds and intermediates of the present disclosure can also be present in different tautomeric forms, and all such forms are included within the scope of the present disclosure. The term "tautomer" or "tautomeric form" refers to structural isomers of different energies that can interconvert via a low-energy barrier.

The present disclosure also includes some isotopically labeled compounds of the present disclosure that are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Compared to non-deuterated drugs, deuterated drugs have the advantages of reduced toxic and side effects, increased drug stability, enhanced efficacy, prolonged biological half-lives, and the like. All isotopic variations of the compounds of the present disclosure, whether radioactive or not, are intended to be included within the scope of the present disclosure. Each available hydrogen atom attached to a carbon atom may be independently replaced with a deuterium atom, wherein the replacement with deuterium may be partial or complete. The partial replacement with deuterium refers to the replacement of at least one hydrogen atom with at least one deuterium atom.

Unless otherwise specified, in the compounds of the present disclosure, when a position is specifically assigned "deuterium" or "D", the position should be construed as the abundance of deuterium being at least 1000 times greater than the natural abundance of deuterium (which is 0.015%) (i.e., at least 15% deuterium incorporation). In some embodiments, the abundance of deuterium of each of the assigned deuterium atoms is at least 1000 times greater than the natural abundance of deuterium (i.e., at least 15% deuterium incorporation). In some embodiments, the abundance of deuterium of each of the assigned deuterium atoms is at least 2000 times greater than the natural abundance of deuterium (i.e., at least 30% deuterium incorporation). In some embodiments, the abundance of deuterium of each of the assigned deuterium atoms is at least 3000 times greater than the natural abundance of deuterium (i.e., at least 45% deuterium incorporation). In some embodiments, the abundance of deuterium of each of the assigned deuterium atoms is at least 3340 times greater than the natural abundance of deuterium (i.e., at least 50.1% deuterium incorporation). In some embodiments, the abundance of deuterium of each of the assigned deuterium atoms is at least 3500 times greater than the natural abundance of deuterium (i.e., at least 52.5% deuterium incorporation). In some embodiments, the abundance of deuterium of each of the assigned deuterium atoms is at least 4000 times greater than the natural abundance of deuterium (i.e., at least 60% deuterium incorporation). In some embodiments, the abundance of deuterium of each of the assigned deuterium atoms is at least 4500 times greater than the natural abundance of deuterium (i.e., at least 67.5% deuterium incorporation). In some embodiments, the abundance of deuterium of each of the assigned deuterium atoms is at least 5000 times greater than the natural abundance of deuterium (i.e., at least 75% deuterium incorporation). In some embodiments, the abundance of deuterium of each of the assigned deuterium atoms is at least 5500 times greater than the natural abundance of deuterium (i.e., at least 82.5% deuterium incorporation). In some embodiments, the abundance of deuterium of each of the assigned deuterium atoms is at least 6000 times greater than the natural abundance of deuterium (i.e., at least 90% deuterium incorporation). In some embodiments, the abundance of deuterium of each of the assigned deuterium atoms is at least 6333.3 times greater than the natural abundance of deuterium (i.e., at least 95% deuterium incorporation). In some embodiments, the abundance of deuterium of each of the assigned deuterium atoms is at least 6466.7 times greater than the natural abundance of deuterium (i.e., at least 97% deuterium incorporation). In some embodiments, the abundance of deuterium of each of the assigned deuterium atoms is at least 6600 times greater than the natural abundance of deuterium (i.e., at least 99% deuterium incorporation). In some embodiments, the abundance of deuterium of each of the assigned deuterium atoms is at least 6633.3 times greater than the natural abundance of deuterium (i.e., at least 99.5% deuterium incorporation). Those skilled in the art can synthesize the compounds in deuterated form by reference to the relevant literature. Commercially available deuterated starting materials can be used in preparing the deuterated compounds, or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

The terms "about" and "approximately" mean that a numerical value is within an acceptable margin of error for the specific value determined by those of ordinary skill in the art, and the numerical value depends in part on how the value is

measured or determined (i.e., the limitations of the measurement system). For example, "about" may mean a standard deviation within 1 or greater than 1. Alternatively, "about" or "substantially comprise" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes embodiments of the given number. Unless otherwise specified, when a specific value is provided in the present application and claims, the meaning of "about" or "substantially comprise" should be assumed to be within an acceptable margin of error for that specific value.

WO2022028462A, WO2023274395A, WO2023208023A, and WO2023109940A are incorporated in the present disclosure by reference in their entirety.

Terms and Definitions

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains.

Unless otherwise specified, in the context of the present disclosure, the terms "apolipoprotein(a) gene", Apo(a) gene, LPA, and Lp(a) are used interchangeably in the present disclosure. LPAs include, but are not limited to, human LPA, cynomolgus monkey LPA, mouse LPA, and rat LPA, the amino acids and complete coding sequences and mRNA sequences of which are readily accessible using publicly available databases, e.g., GenBank, UniProt, OMIM, and the Macaca genome project website.

The term "target sequence" refers to a contiguous portion of the nucleotide sequence of an mRNA molecule formed during the transcription of LPA, including mRNA that is a product of RNA processing of a primary transcription product. The targeted portion in the target sequence should be long enough to serve as a substrate for iRNA-directed cleavage. In one embodiment, the target sequence is within the protein encoding region of LPA.

As used herein, the sense strand (also referred to as SS or SS strand) of the dsRNA refers to a strand that comprises a sequence that is identical or substantially identical to a target mRNA sequence; the antisense strand (also referred to as AS or AS strand) of the dsRNA refers to a strand comprising a sequence that is at least partially complementary to a target mRNA sequence.

In the context of describing the sense strand of the dsRNA described herein, the term "at least 17 contiguous nucleotides of a sequence that differs from the nucleotide sequence of SEQ ID NO: 1 by no more than 3 nucleotides" is intended to mean that the sense strand of the dsRNA described herein comprises at least 17 contiguous nucleotides of the sense strand set forth in SEQ ID NO: 1, or a sequence that differs from at least 17 contiguous nucleotides of the sense strand set forth in SEQ ID NO: 1 by no more than 3 nucleotides, optionally by no more than 2 nucleotides, and optionally by 1 nucleotide. Optionally, the sense strand of the dsRNA described herein comprises at least 18 contiguous nucleotides from the sense strand set forth in SEQ ID NO: 1, or a sequence that differs from at least 18 contiguous nucleotides of the sense strand set forth in SEQ ID NO: 1 by no more than 3 nucleotides, optionally by no more than 2 nucleotides, and optionally by 1 nucleotide. In the context of describing the antisense strand of the dsRNA described herein, the term "at least 19 contiguous nucleotides of a sequence that differs from the antisense strand set forth in SEQ ID NO: 2 by no more than 3 nucleotides" is intended to mean at least 19 contiguous nucleotides of the antisense strand set forth in SEQ ID NO: 2 described herein, or a sequence that differs from at least 19 contiguous nucleotides of the antisense strand set forth in SEQ ID NO: 2 by no more than 3 nucleotides, optionally by no more than 2 nucleotides, and optionally by 1 nucleotide.

In the present disclosure, the "5' region", "5' end", and "5' terminus" of the sense or antisense strand are used interchangeably. For example, the nucleotides at positions 2 to 8 of the 5' region of the antisense strand may also be replaced with the nucleotides at positions 2 to 8 of the 5' end of the antisense strand. Likewise, the "3' region", "3' terminus", and "3' end" of the sense or antisense strand are also used interchangeably.

Unless otherwise specified, in the context of the present disclosure, "G", "C", "A", "T", and "U" each represent a nucleotide, and comprise the bases of guanine, cytosine, adenine, thymidine, and uracil, respectively. The lowercase letter m means that the upstream nucleotide adjacent to the letter m is a methoxy-modified nucleotide; the lowercase letter f means that the upstream nucleotide adjacent to the letter f is a fluoro-modified nucleotide; the lowercase letter s means that the two nucleotides directly adjacent to the letter s are linked by a phosphorothioate diester group.

As used in the present disclosure, the term "2'-fluoro (2'-F)-modified nucleotide" refers to a nucleotide in which the hydroxy group at the 2' position of the ribosyl group of the nucleotide is substituted with fluorine, and "non-fluoro-modified nucleotide" refers to a nucleotide or a nucleotide analog in which the hydroxy group at the 2' position of the ribosyl group of the nucleotide is substituted with a non-fluorine group.

As used in the present disclosure, the term "2'-methoxy (2'-OMe)-modified nucleotide" refers to a nucleotide in which the 2'-hydroxy group of the ribosyl group is substituted with a methoxy group.

In the context of the present disclosure, the "nucleotide difference" between one nucleotide sequence and another nucleotide sequence means that the former has a change in the base type of the nucleotide at the same position as

compared to the latter; for example, when one nucleotide base in the latter is A, in the case where the corresponding nucleotide base at the same position in the former is U, C, G, or T, it is considered that there is a nucleotide difference at that position between the two nucleotide sequences. In some embodiments, the replacement of a nucleotide at its original position with an abasic nucleotide or an equivalent thereof can also be considered as creating a nucleotide difference at that position.

As used herein, the terms "complementary" and "reverse complementary" are used interchangeably and have the meaning well-known to those skilled in the art; that is, in a double-stranded nucleic acid molecule, the bases of one strand are paired with the bases of the other strand in a complementary manner. In DNA, the purine base adenine is always paired with the pyrimidine base thymine (or uracil in RNA), and the purine base guanine is always paired with the pyrimidine base cytosine. Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

The term "dsRNA" refers to a double-stranded RNA molecule capable of RNA interference, comprising a sense strand and an antisense strand.

The term "chemical modification" or "modification" includes all changes made to a nucleotide by chemical means, such as the addition or removal of a chemical moiety, or the substitution of one chemical moiety for another.

The term "base" encompasses any known DNA and RNA bases and base analogs such as purines or pyrimidines, and also includes the natural compounds adenine, thymine, guanine, cytosine, uracil, inosine, and natural analogs.

The term "optionally" or "optional" means that the event or circumstance subsequently described may, but does not necessarily, occur, and this description includes instances where the event or circumstance occurs or does not occur. For example, "$C_{1-6}$ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

The term "alkyl" refers to a saturated aliphatic hydrocarbon group that is a straight-chain or branched-chain group containing 1 to 20 carbon atoms; in some embodiments, alkyl is selected from the group consisting of alkyl groups containing 1 to 12 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. In some embodiments, alkyl is selected from the group consisting of alkyl groups containing 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted, and when it is substituted, the substituent may be substituted at any accessible point of attachment; the substituent, in some embodiments, is selected from the group consisting of one or more of the following groups; the substituent is independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and a carboxylate group.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted, and when it is substituted, the substituent is preferably one or more of the following groups; the substituent is independently selected from the group consisting of halogen, hydroxy, oxo, cyano, amino, $C_{1-6}$ alkyl, $C_{1-6}$ alkoxy, 3- to 7-membered cycloalkyl, and 3- to 7-membered heterocycloalkyl, and the alkyl, alkoxy, cycloalkyl, or heterocycloalkyl is optionally substituted with halogen, hydroxy, nitro, cyano, or amino.

The term "alkylthio" refers to -S-(alkyl), wherein the alkyl is as defined above. Non-limiting examples of alkylthio include: methylthio, ethylthio, propylthio, and butylthio. Alkylthio may be optionally substituted or unsubstituted, and when it is substituted, the substituent is preferably one or more of the following groups; the substituent is independently selected from the group consisting of $C_{1-6}$ alkoxy, 3- to 6-membered cycloalkyl, 3- to 6-membered heterocycloalkyl, 3- to 6-membered cycloalkoxy, 3- to 6-membered heterocycloalkoxy, $C_{1-6}$ alkylthio, 3- to 6-membered cycloalkylthio, and 3- to 6-membered heterocycloalkylthio, and the alkoxy, cycloalkyl, heterocycloalkyl, cycloalkoxy, heterocycloxy, alkylthio,

cycloalkylthio, and heterocycloalkylthio are optionally substituted with halogen, hydroxy, cyano, or amino.

The term "alkenyl" refers to a straight-chain or branched-chain non-aromatic hydrocarbon group containing at least one carbon-carbon double bond and having 2-10 carbon atoms. Up to 5 carbon-carbon double bonds may be present in such groups. For example, "$C_2$-$C_6$" alkenyl is defined as an alkenyl group having 2-6 carbon atoms. Examples of alkenyl groups include, but are not limited to: ethenyl, propenyl, butenyl, and cyclohexenyl. The straight-chain, branched-chain, or cyclic portion of an alkenyl group may contain a double bond and is optionally mono-, di-, tri-, tetra-, or penta-substituted at any position as permitted by normal valency.

The term "alkynyl" refers to a straight-chain or branched-chain hydrocarbon group containing 2-10 carbon atoms and containing at least one carbon-carbon triple bond. Up to 5 carbon-carbon triple bonds may be present. Thus, "$C_2$-$C_6$ alkynyl" refers to an alkynyl group having 2-6 carbon atoms. Examples of alkynyl groups include, but are not limited to: ethynyl, 2-propynyl, and 2-butynyl. The straight-chain or branched-chain portion of an alkynyl group may contain a triple bond as permitted by normal valency and is optionally mono-, di-, tri-, tetra-, or penta-substituted at any position as permitted by normal valency.

The term "cycloalkyl" or "carbocycle" refers to a saturated or partially unsaturated, monocyclic or polycyclic hydrocarbon substituent; the cycloalkyl ring contains 3 to 20 carbon atoms and, in some embodiments, is selected from the group consisting of cycloalkyl rings containing 3 to 7 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups. Cycloalkyl may be substituted or unsubstituted, and when it is substituted, the substituent may be substituted at any accessible point of attachment; in some embodiments, the substituent is selected from the group consisting of one or more of the following groups; the substituent is independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, and the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano.

The cycloalkyl ring may be fused to an aryl or heteroaryl ring, wherein the ring attached to the parent structure is cycloalkyl; non-limiting examples include indanyl, tetrahydronaphthyl, benzocycloheptyl, etc. Cycloalkyl may be optionally substituted or unsubstituted, and when it is substituted, the substituent, in some embodiments, is selected from the group consisting of one or more of the following groups; the substituent is independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, and the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano.

The term "heterocycloalkyl" or "heterocycle" or "heterocyclyl" refers to a saturated or partially unsaturated, monocyclic or polycyclic hydrocarbon substituent containing 3 to 20 ring atoms, wherein one or more ring atoms are heteroatoms selected from the group consisting of nitrogen, oxygen, and $S(O)_m$ (where m is an integer from 0 to 2), but a ring moiety of -O-O-, -O-S-, or -S-S- is excluded, and the other ring atoms are carbon atoms. In some embodiments, heterocycloalkyl is selected from the group consisting of heterocycloalkyl groups containing 3 to 12 ring atoms, wherein 1-4 ring atoms are heteroatoms; in some embodiments, heterocycloalkyl is selected from the group consisting of heterocycloalkyl groups containing 3 to 7 ring atoms. Non-limiting examples of monocyclic heterocycloalkyl groups include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, etc. Polycyclic heterocycloalkyl groups include spiro-ring, fused-ring, and bridged-ring heterocycloalkyl groups. Non-limiting examples of "heterocycloalkyl" include:

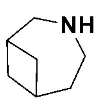 , 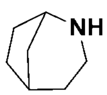 , 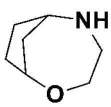 , 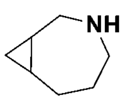 , 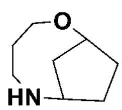 , 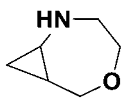 ,

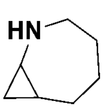 , 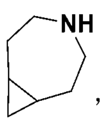 , 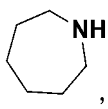 , 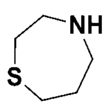 , 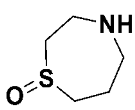 , 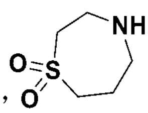 ,

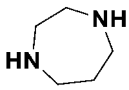, 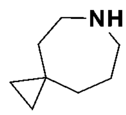, 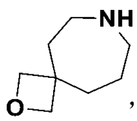, 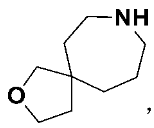, 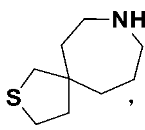, 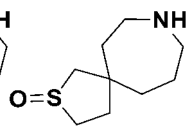,

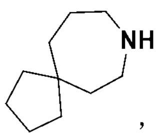, 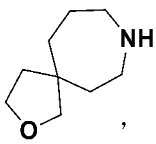, 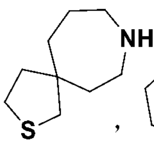, 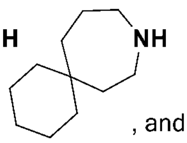, and 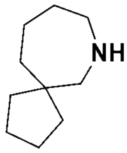,

etc.

The heterocycloalkyl ring may be fused to an aryl or heteroaryl ring, wherein the ring attached to the parent structure is heterocycloalkyl; its non-limiting examples include:

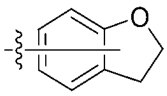, 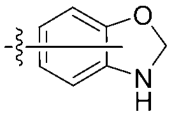, 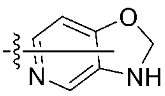, 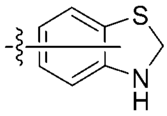,

etc.

Heterocycloalkyl may be optionally substituted or unsubstituted, and when it is substituted, the substituent, in some embodiments, is selected from the group consisting of one or more of the following groups; the substituent is independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, and the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano.

The term "aryl" refers to a 6- to 14-membered all-carbon monocyclic or fused polycyclic (i.e., rings sharing a pair of adjacent carbon atoms) group having a conjugated π-electron system; in some embodiments, aryl is selected from the group consisting of 6- to 12-membered aryl groups, such as phenyl and naphthyl. The aryl ring may be fused to a heteroaryl, heterocycloalkyl, or cycloalkyl ring, wherein the ring attached to the parent structure is the aryl ring; its non-limiting examples include:

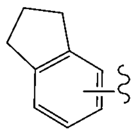, 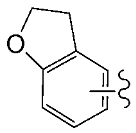, 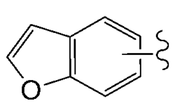, 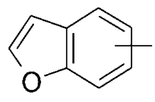, 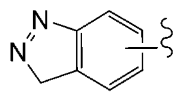, 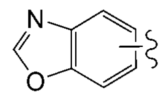,

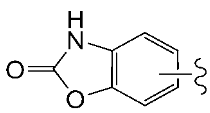, 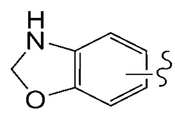, 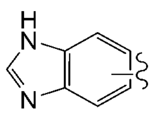, 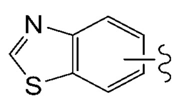, 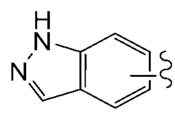,

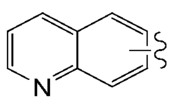, 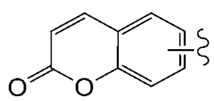, and 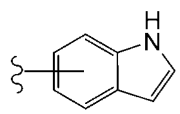.

Aryl may be substituted or unsubstituted, and when it is substituted, the substituent is preferably one or more of the following groups; the substituent is independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and a carboxylate group, preferably from the group consisting of phenyl.

The term "fused-ring aryl" may be an unsaturated aromatic fused-ring structure containing 8-14 ring atoms, preferably 8-12 ring atoms, formed by connecting two or more ring structures that share two adjacent atoms with each

other, including, for example, all unsaturated fused-ring aryl groups such as naphthalene and phenanthrene, as well as partially saturated fused-ring aryl groups such as benzo 3- to 8-membered saturated monocyclic cycloalkyl groups and benzo 3- to 8-membered partially saturated monocyclic cycloalkyl groups. "Fused aromatic ring" refers to the ring system in a fused-ring aryl group. Specific examples of fused-ring aryl groups include 2,3-dihydro-1H-indenyl, IH-indenyl, 1,2,3,4-tetrahydronaphthyl, 1,4-dihydronaphthyl, etc.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Preferably, heteroaryl is 5- to 12-membered, e.g., imidazolyl, furyl, thienyl, thiazolyl, pyrazolyl, oxazolyl, pyrrolyl, tetrazolyl, pyridyl, pyrimidinyl, thiadiazole, or pyrazinyl, preferably imidazolyl, pyrazolyl, pyrimidinyl, or thiazolyl; more preferably, heteroaryl is pyrazolyl or thiazolyl. The heteroaryl ring may be fused to an aryl, heterocyclyl, or cycloalkyl ring, wherein the ring attached to the parent structure is the heteroaryl ring. "Heteroaromatic ring" refers to the ring system in a heteroaryl group. Non-limiting examples of heteroaryl groups include:

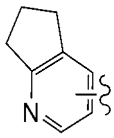, 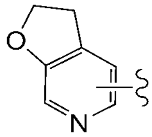, 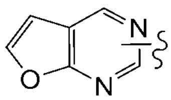, 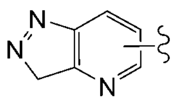, 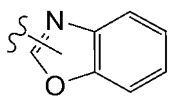, 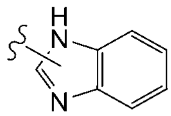,

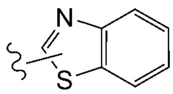, 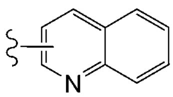, 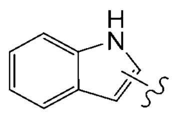, and 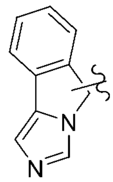.

Heteroaryl may be optionally substituted or unsubstituted, and when it is substituted, the substituent, in some embodiments, is selected from the group consisting of one or more of the following groups; the substituent is independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, and the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano.

The term "alkylamino" refers to a group having a structure of -NH(C1-C12 alkyl).

The term "hydroxyalkyl" refers to an alkyl group substituted with one or more hydroxy groups, wherein the alkyl group is as defined above.

The term "hydroxy" refers to the -OH group.

The term "halogen" refers to fluorine, chlorine, bromine, or iodine.

The term "haloalkyl" refers to an alkyl group substituted with halogen, wherein the alkyl group is as defined above.

The term "haloalkoxy" refers to an alkoxy group substituted with halogen, wherein the alkoxy group is as defined above.

The term "cyano" refers to -CN.

The term "nitro" refers to $-NO_2$.

The term "oxo" refers to the =O group; for example, a carbon atom is linked to the oxygen atom by a double bond, resulting in a keto or aldehyde group.

The term "amino" refers to $-NH_2$.

The term "carboxyl" refers to -C(O)OH.

The terms "blunt" and "blunt-ended" are used interchangeably and mean that there are no unpaired nucleotides or nucleotide analogs at a given end of a dsRNA, i.e., no nucleotide overhang. In most cases, a dsRNA whose both ends are blunt-ended will be double-stranded over its entire length.

In the context of the present disclosure, the phosphodiester group or the phosphodiester group with a modification in each modification group can be replaced with any group capable of linking it to an adjacent nucleotide; for example, the

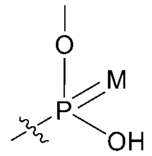

moiety in the chemical modification

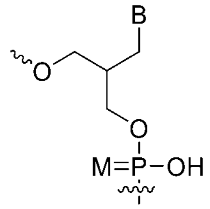

can be replaced with any group capable of linking to an adjacent nucleotide.

The term "link", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between.

The term "indirectly linked" means that a first compound or group is linked to a second compound or group by an intermediate group, a compound, or a molecule (e.g., a linking group).

The term "substituted" means that any one or more hydrogen atoms on the specified atom (usually a carbon, oxygen, or nitrogen atom) are replaced with any group as defined herein, provided that the normal valency of the specified atom is not exceeded and that the substitution results in a stable compound. Non-limiting examples of substituents include C1-C6 alkyl, C2-C6 alkenyl, C2-C6 alkynyl, cyano, hydroxy, oxo, carboxyl, cycloalkyl, cycloalkenyl, heterocyclyl, heteroaryl, aryl, ketone, alkoxycarbonyl, aryloxycarbonyl, heteroaryloxycarbonyl, or halogen (e.g., F, Cl, Br, or I). When the substituent is a ketone or oxo (i.e., =O), two (2) hydrogens on the atom are replaced.

"Substituted with one or more..." refers to substitution with a single substituent or multiple substituents. In the case of substitution with multiple substituents, there may be a plurality of identical substituents, or one or a group of a plurality of different substituents. "Pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity. "Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved as acceptable for use in humans or livestock animals. As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level may be any type of control level used in the art, such as a pre-dose baseline level or a level determined from a similar untreated or control (e.g., buffer-only control or inert agent control) treated subject, cell, or sample. For example, the remaining mRNA expression level can be used to characterize the degree of inhibition of target gene expression by the dsRNA; for example, the remaining expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition of the expression of a target gene can be measured using Dual-Glo® Luciferase Assay System: the Firefly chemiluminescence value and the Renilla chemiluminescence value are each read, and the relative value Ratio = Ren/Fir and the inhibition rate (%) = 1 - (Ratio + siRNA/Ratioreporter only) × 100% are calculated. In the present disclosure, the proportion of remaining mRNA expression (or residual activity%) = 100% - the inhibition rate (%).

Unless otherwise specified, the "compound", "ligand", "nucleic acid-ligand conjugate", "dsRNA conjugate", "nucleic acid", "conjugate", "chemical modification", "targeting ligand", "dsRNA", and "RNAi" of the present disclosure can each independently be present in the form of a salt, mixed salts, or a non-salt (e.g., a free acid or free base). When being present in the form of a salt or mixed salts, it may be a pharmaceutically acceptable salt.

The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts, and magnesium salts; sodium salts are preferred. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

"Effective amount" or "effective dose" includes an amount sufficient to ameliorate or prevent a symptom or sign of a medical disorder. An effective amount also means an amount sufficient to allow or facilitate diagnosis. The effective amount for a particular patient or veterinary subject may vary depending on factors such as the disorder to be treated, the general health of the patient, the method and route and dosage of administration, and the severity of side effects. An effective amount may be the maximum dose or administration regimen to avoid significant side effects or toxic effects.

As used herein, "object", "patient", "subject", and "individual" are used interchangeably and include human or non-human animals, e.g., mammals, e.g., humans or monkeys. The ordinals "first", "second", etc. in the present disclosure are not intended to limit the order or hierarchy, but are used only for distinguishing different features, molecules, steps, compositions, elements, etc.

The dsRNA provided by the present disclosure can be obtained using a preparation method conventional in the art (e.g., solid-phase synthesis and liquid-phase synthesis). Solid-phase synthesis has been commercially available as a customization service. A modified nucleotide group can be introduced into the dsRNA of the present disclosure using a nucleoside monomer with a corresponding modification. Methods of preparing a nucleoside monomer with a corresponding modification and introducing a modified nucleotide group into a dsRNA are also well known to those skilled in the art.

Some abbreviations in the present disclosure are defined as follows:

DMF: dimethylformamide;
DIPEA: N-ethyl diisopropylamine;
HBTU: benzotriazole-N,N,N',N'-tetramethyluronium hexafluorophosphate;
DMAP: 4-dimethylaminopyridine;
NMI: N-methylimidazole.

## BRIEF DESCRIPTION OF THE DRAWINGS

FIG. 1A shows the ratios of the serum Lp(a) concentrations on day 28 after administration of TJR101079 and TRD007790 to those before the administration.

FIG. 1B shows the ratios of the serum Lp(a) concentrations on day 28 after administration of TJR101079 and TJR102134 to those before the administration.

FIG. 1C shows the ratios of the serum Lp(a) concentrations on day 28 after administration of TJR101079 and TJR102136 to those before the administration.

## DETAILED DESCRIPTION

The present disclosure is further described below with reference to examples, but these examples are not intended to limit the scope of the present disclosure. Experimental procedures without specific conditions indicated in the examples of the present disclosure were generally conducted under conventional conditions or conditions recommended by the manufacturers of the starting materials or commercial products. Where the specific source of a reagent is not indicated, the reagent may be obtained from any supplier of reagents for molecular biology at a quality/purity level for application in molecular biology.

### Example 1: Chemical Modifications

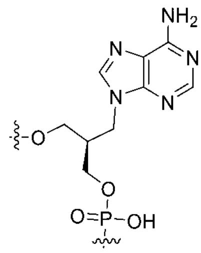

(-)hmpNA(A)

(-)hmpNA(A) was prepared with reference to the method in WO2022028462A. The absolute configuration of (-) hmpNA(A) was (R)-hmpNA(A).

### Example 2: Preparation of NAG0052, L96, and NAG25

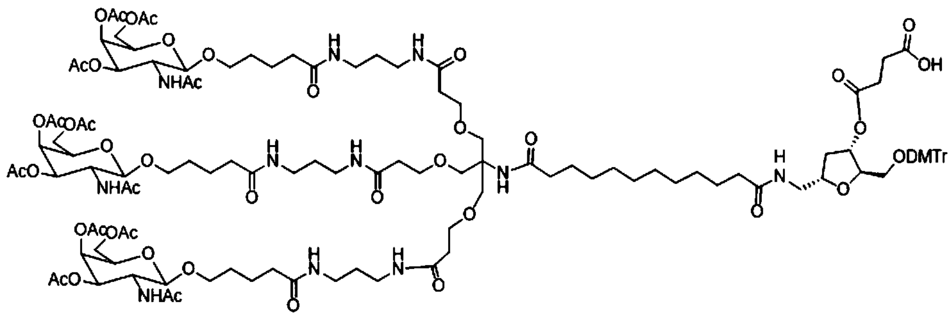

NAG0052, represented by the above structural formula, was prepared using the method described in patent application No. WO2023274395A.

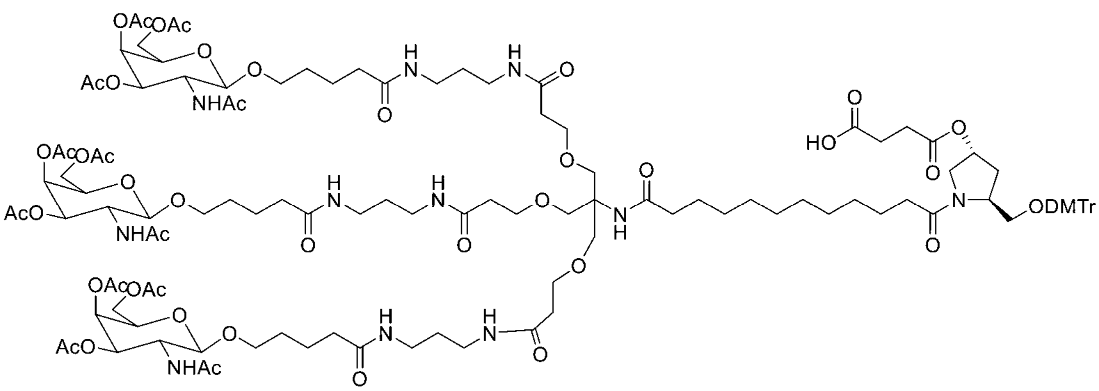

L96, represented by the above structural formula, was prepared using the method described in patent application

No. WO2014025805A.

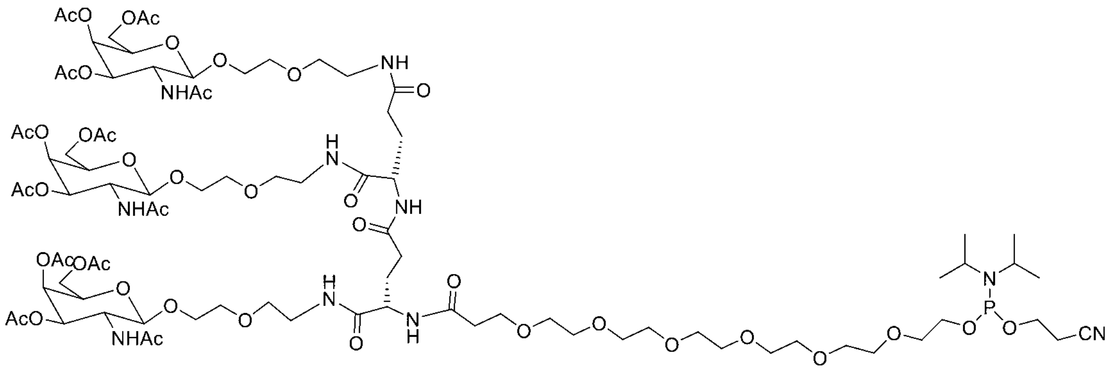

**NAG25,** represented by the above structural formula, was prepared using the method in patent application No. WO2017156012A.

**Example 3: Preparation of Deuterated 5'-End Chemically Modified Phosphoramidite**

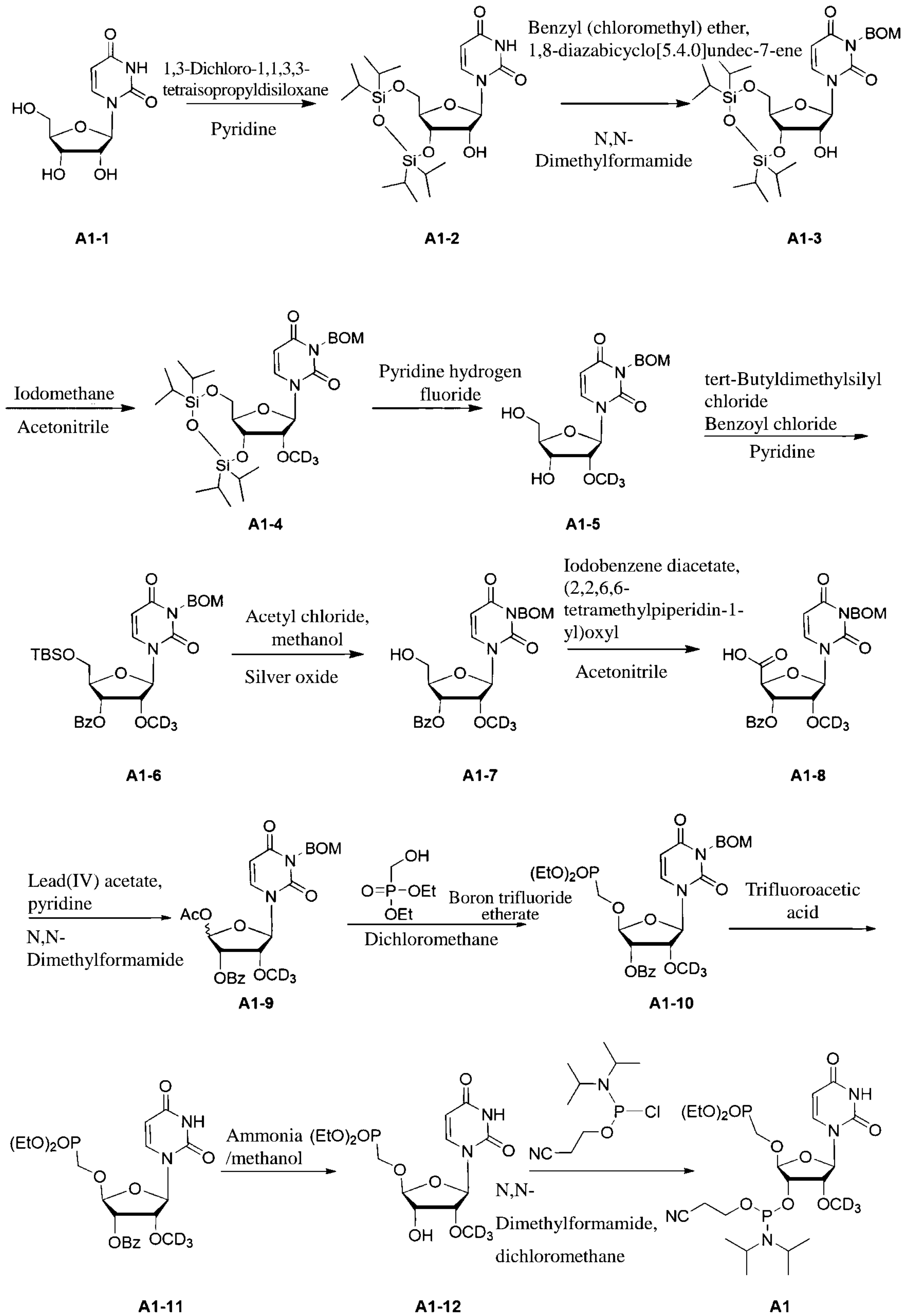


A1-1 A1-2 A1-3

A1-4 A1-5

A1-6 A1-7 A1-8

A1-9 A1-10

A1-11 A1-12 A1

Step 1

1-((6aR,8R,9R,9aS)-9-Hydroxy-2,2,4,4-tetraisopropyltetrahydro-6H-furo[3,2-f][1,3,5,2,4]trioxadisilocin-8-yl)pyrimidine-2,4(1H,3H)-dione **A1-2**

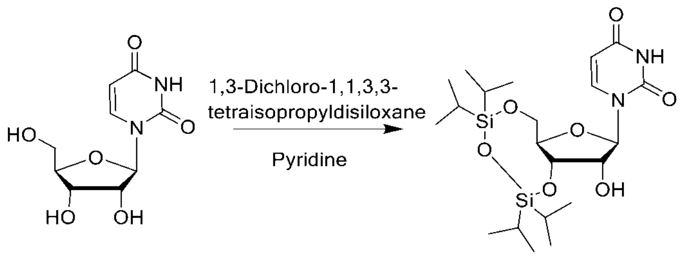


**A1-1** **A1-2**

In a nitrogen atmosphere, commercially available compound **A1-1** (100 g, 409 mmol) was dissolved in pyridine (1000 mL), and 1,3-dichloro-1,1,3,3-tetraisopropyldisiloxane ($TIPDSiCl_2$) (135.6 g, 430.0 mmol) was added at 0 °C. The mixture was left to react at 20 °C for 18 h. Ethyl acetate (1500 mL) and water (1000 mL) were added to the reaction mixture for extraction. The organic phase was washed three times with saturated brine (1000 mL × 3), then dried over anhydrous sodium sulfate, concentrated, and purified by column chromatography (petroleum ether:ethyl acetate = 2:1) to give compound **A1-2** (185 g, 380 mmol, yield: 92.8%).

Step 2

3-((Benzyloxy)methyl)-1-((6aR,8R,9R,9aS)-9-hydroxy-2,2,4,4-tetraisopropyltetrahydro-6H-furo[3,2-][1,3,5,2,4]trioxadisilocin-8-yl)pyrimidine-2,4(1H,3H)-dione **A1-3**

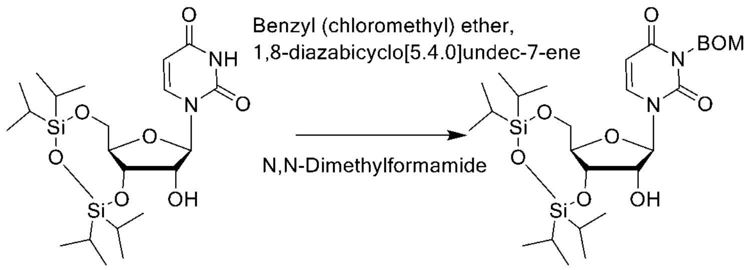


**A1-2** **A1-3**

Compound **A1-2** (185 g, 380.1 mmol) was dissolved in N,N-dimethylformamide (1850 mL), and benzyl chloromethyl ether (BOMCl) (89.29 g, 570.1 mmol) and 1,8-diazabicycloundec-7-ene (DBU) (115.7 g, 760.2 mmol) were then added. The mixture was left to react at 20 °C for 3 h. Ethyl acetate (3000 mL) and water (3000 mL) were added to the reaction mixture for extraction. The organic phase was washed three times with saturated brine (2000 mL × 3), then dried over anhydrous sodium sulfate, concentrated, and purified by column chromatography (petroleum ether:ethyl acetate = 3:1) to give compound **A1-3** (target compound, 172 g, 275.6 mmol, yield: 72.5%).

Step 3

3-((Benzyloxy)methyl)-1-((6aR,8R,9R,9aR)-2,2,4,4-tetraisopropyl-9-(methoxy-d3)tetrahydro-6H-furo[3,2-][1,3,5,2,4] trioxadisilocin-8-yl)pyrimidine-2,4(1H,3H)-dione **A1-4**

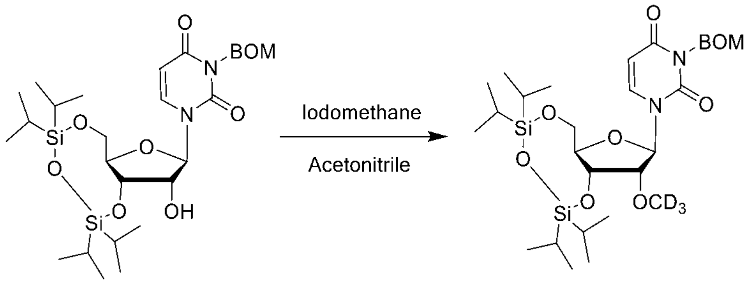


A1-3 A1-4

Compound **A1-3** (150 g, 247.2 mmol) was dissolved in acetonitrile (300 mL), and $CD_3I$ (107.5 g, 741.5 mmol) and silver oxide (114.5 g, 494.3 mmol) were added. The mixture was left to react at 55 °C for 24 h. The reaction mixture was filtered and concentrated under reduced pressure to give crude compound **A1-4** (target compound, 125 g, 200.3 mmol, yield: 81%).

Step 4

3-((Benzyloxy)methyl)-1-((2R,3R,4R,5R)-4-hydroxy-5-(hydroxymethyl)-3-(methoxy-d3)tetrahydrofuran-2-yl)pyrimidine-2,4(1H,3H)-dione **A1-5**

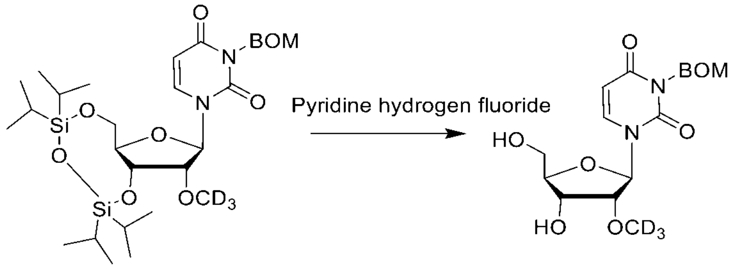


A1-4 A1-5

Compound **A1-4** (125 g, 200 mmol) was dissolved in tetrahydrofuran (1250 mL), and pyridine hydrogen fluoride (158.8 g, 1.60 mol) was then added. The mixture was left to react at 25 °C for 18 h. Ethyl acetate (1000 mL) and water (1000 mL) were added to the reaction mixture for extraction. The organic phase was washed once with saturated brine (1000 mL), then dried over anhydrous sodium sulfate, filtered, concentrated under reduced pressure, and purified by column chromatography (dichloromethane:methanol = 20:1) to give compound **A1-5** (target compound, 59 g, 154.7 mmol, yield: 77%).

Step 5

(2R,3R,4R,5R)-5-(3-((Benzyloxy)methyl)-2,4-dioxo-3,4-dihydropyrimidin-1(2H)-yl)-2-(((tert-butyldimethylsilyl)oxy)methyl)-4-(methoxy-d3)tetrahydrofuran-3-yl benzoate **A1-6**

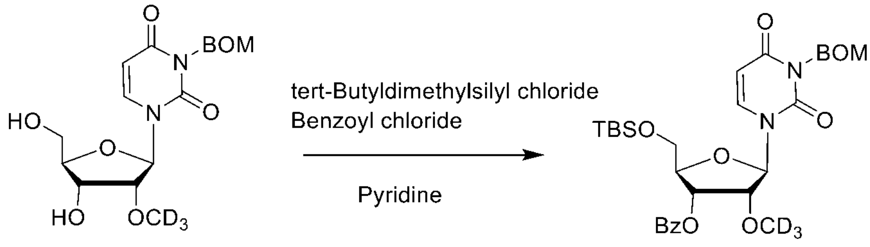


A1-5

A1-6

Compound **A1-5** (59 g, 154.7 mmol) was dissolved in pyridine (590 mL), and tert-butyldimethylsilyl chloride (TBSCl) (93.2 g, 618 mmol) was then added. The mixture was left to react at 25 °C for 18 h. Benzoyl chloride (32.62 g, 232.0 mmol) was then added, and the mixture was left to react at 25 °C for 3 h. Ethyl acetate (1000 mL) and water (1000 mL) were added to the reaction mixture for extraction. The organic phase was washed twice with saturated brine (1000 mL × 2), then dried over anhydrous magnesium sulfate, concentrated, and purified by column chromatography (petroleum ether:ethyl acetate = 5:1) to give compound **A1-6** (target compound, 60.0 g, 100.3 mmol, yield: 64.6%).

Step 6

(2R,3R,4R,5R)-5-(3-((Benzyloxy)methyl)-2,4-dioxo-3,4-dihydropyrimidin-1(2H)-yl)-2-hydroxymethyl-4-(methoxy-d3) tetrahydrofuran-3-yl benzoate A1-7

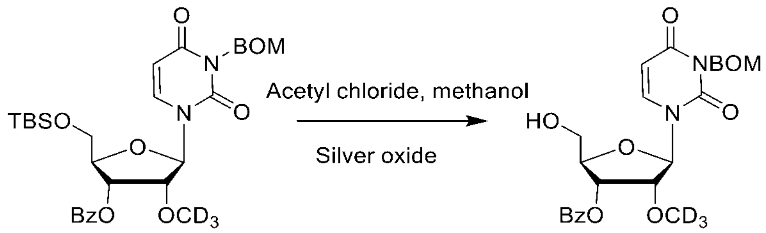


A1-6 A1-7

At 0 °C, compound A1-6 (60.0 g, 100.3 mmol) was dissolved in methanol (600 mL), and acetyl chloride (10.21 g, 130.1 mmol) was then added. The mixture was left to react at 20 °C for 2 h. Silver carbonate was then added, and the mixture was stirred at 20 °C for 1 h. The reaction mixture was filtered, and the filtrate was concentrated under reduced pressure to give compound **A1-7** (46.0 g, 94.7 mmol, yield: 94.7%).

Step 7

(2S,3S,4R,5R)-3-(Benzyloxy)-5-(3-((benzyloxy)methyl)-2,4-dioxo-3,4-dihydropyrimidin-1(2H)-yl)-4-(methoxy-d3)tetrahydrofuran-2-carboxylic acid **A1-8**

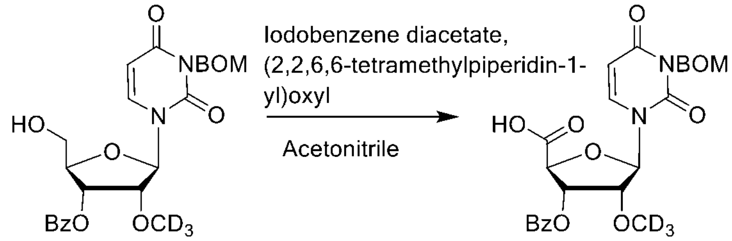


A1-7 A1-8

Compound **A1-7** (46.0 g, 94.75 mmol) and 4-carbonyl-tetramethylpiperidine oxide (TEMPO) (6.95 g, 44.49 mmol) were dissolved in $CH_3CN$ (230 mL) and $H_2O$ (230 mL), and 5(6)-amino-1-(4-aminophenyl)-1,3,3-trimethylindane (PIDA) (67.14 g, 208.4 mmol) was added. The mixture was left to react at 25 °C for 18 h. Ethyl acetate (500 mL) and water (300 mL) were added to the reaction mixture for extraction. The organic phase was dried over anhydrous sodium sulfate, then concentrated, purified by column chromatography (dichloromethane:methanol = 10:1), and concentrated under reduced pressure to give compound **A1-8** (target compound, 43.0 g, 86 mmol, yield: 90%).

Step 8

(3S,4R,5R)-2-Acetoxy-5-(3-((benzyloxy)methyl)-2,4-dioxo-3,4-dihydropyrimidin-1(2H)-yl)-4-(methoxy-d3)tetrahydrofuran-3-yl benzoate **A1-9**

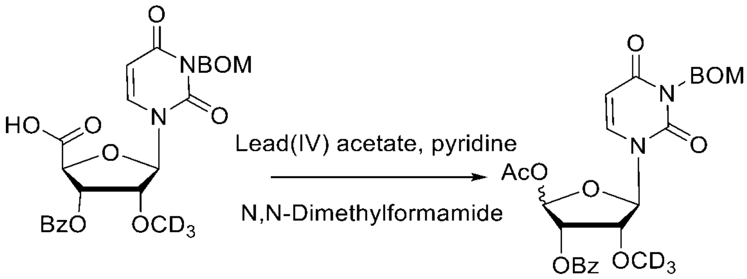


**A1-8** **A1-9**

Compound **A1-8** (5 g, 10.0 mmol) was added to a dry flask. After an argon purge, N,N-dimethylformamide (50 mL) was added. Pyridine (8 mL, 100 mmol) and lead acetate (9.7 mL, 50 mmol) were then sequentially added. The reaction mixture was kept away from light and stirred at room temperature for 48 h. The reaction mixture was quenched with water (200 mL) and diluted with ethyl acetate (200 mL). The resulting suspension was filtered using a pad of diatomaceous earth. The solid was rinsed with ethyl acetate. The organic layer was separated and concentrated *in vacuo.* The crude product was purified on a C18 reversed-phase column to give the title product **A1-9** (1.5 g, yield: 29%) as an $\alpha/\beta$ mixture.
MS (ESI): m/z = 514.2 $[M+H]^+$.

Step 9

(3S,4R,5R)-5-(3-((Benzyloxy)methyl)-2,4-dioxo-3,4-dihydropyrimidin-1(2H)-yl)-2-((diethoxyphosphoryl)methoxy)-4-(methoxy-d3)tetrahydrofuran-3-yl benzoate **A1-10**

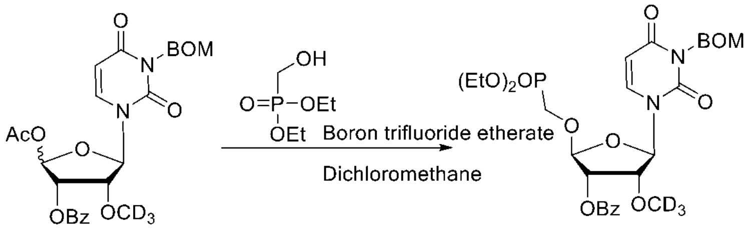


**A1-9** **A1-10**

In an argon atmosphere, diethyl (hydroxymethyl)phosphonate (2.5 g, 156.7 mmol) and boron trifluoride-diethyl ether coordination complex (5.7 mL, 43.8 mmol) were added to a solution of compound **A1-9** (1.5 g, 2.9 mmol) in anhydrous dichloromethane (15 mL). The reaction mixture was stirred at room temperature for 16 h. The reaction mixture was quenched with water and extracted with ethyl acetate. The organic layer was separated, washed with brine, dried over anhydrous sodium sulfate, and concentrated *in vacuo.* The crude product was purified on a C18 reversed-phase column to give the title compound **A1-10** (1 g, yield: 55%).
MS (ESI): m/z = 622.3 $[M+H]^+$.

Step 10

(3S,4R,5R)-2-((Diethoxyphosphoryl)methoxy)-5-(2,4-dioxo-3,4-dihydropyrimidin-1(2H)-yl)-4-(methoxy-d3)tetrahydrofuran-3-yl benzoate **A1-11**

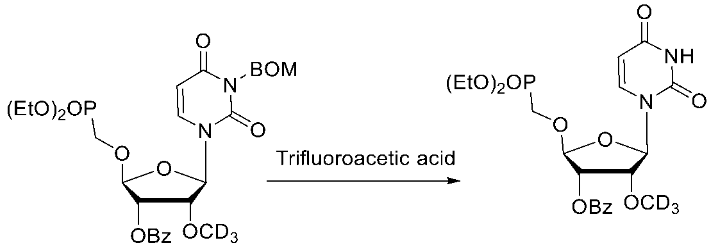


**A1-10** **A1-11**

A solution of compound **A1-10** (1 g, 14.6 mmol) in TFA (5 mL) was stirred at 80 °C for 30 min and then concentrated *in vacuo* to give the title compound **A1-11** (1 g, crude), which was directly used in the next step.
MS (ESI): m/z = 502.3 $[M+H]^+$.

Step 11

Diethyl ((((3S,4R,5R)-5-(2,4-dioxo-3,4-dihydropyrimidin-1(2H)-yl)-3-hydroxy-4-(methoxy-d3)tetrahydrofuran-2-yl)oxy)methyl)phosphonate A1-12

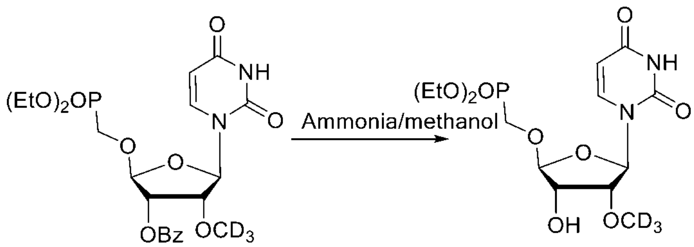


**A1-11** **A1-12**

A solution of compound **A1-11** (1 g, 2.0 mmol) in a solution of ammonia in methanol (7 N, 10 mL) was stirred at room temperature for 16 h. The reaction mixture was concentrated *in vacuo.* The crude product was purified on a C18 reversed-phase column to give the title compound **A1-12** (300 mg, yield: 38%).
MS (ESI): m/z = 398.2 $[M+H]^+$.

Step 12

2-Cyanoethyl ((2R,3S,4R,5R)-2-((diethoxyphosphoryl)methoxy)-5-(2,4-dioxo-3,4-dihydropyrimidin-1(2H)-yl)-4-(methoxy-d3)tetrahydrofuran-3-yl) diisopropylphosphoramidite **A1**

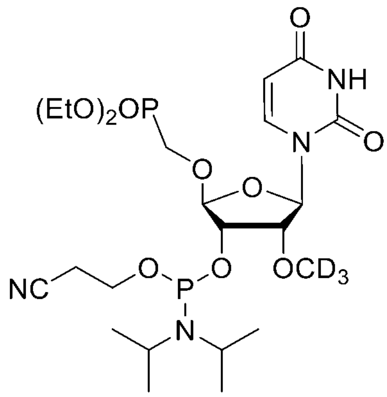

**A1**

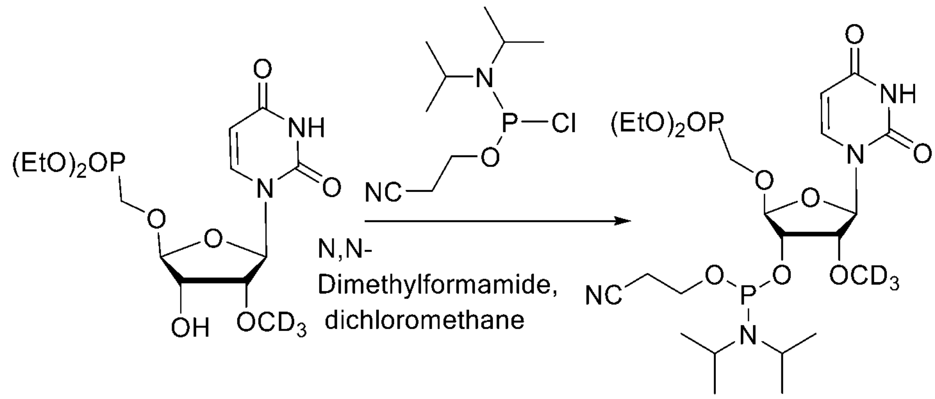


**A1-12** **A1**

DIPEA (58 mg, 0.45 mmol) was added to a solution of compound **A1-12** (100 mg, 0.252 mmol) in anhydrous dichloromethane (2 mL), and 3-[chloro-(diisopropylamino)phosphaneyl]oxypropanenitrile (83 mg, 0.35 mmol) was then added. The reaction mixture was stirred at room temperature for 2 h and then quenched with MeOH. The reaction mixture was diluted with ethyl acetate and washed with saturated sodium bicarbonate, water, and brine. The organic layer was concentrated *in vacuo.* The crude product was purified on a C18 reversed-phase column to give phosphoramidite monomer **A1** (80 mg, yield: 53%).

MS (ESI): m/z = 596.0 $[M-H]^+$.

$^1H$ NMR (400 MHz, $CD_3OD$) $\delta$ 7.74-7.70 (m, 1H), 6.33-6.30 (m, 1H), 5.84-5.82 (m, 1H), 5.25-5.18 (m, 1H), 4.51-4.44 (m, 1H), 4.27-3.67 (m, 11H), 2.82-2.79 (m, 2H), 1.41-1.36 (m, 6H), 1.29-1.23 (m, 12H).

$^{31}P$ NMR (400 MHz, $CD_3OD$) $\delta$ 151.95, 150.57, 21.29.

**Example 4: Synthesis of dsRNAs with Deuterated Chemical Modifications at 5' Ends**

The dsRNA synthesis was the same as the conventional phosphoramidite solid-phase synthesis. In synthesizing nucleotides with modifications at the ends of the antisense strands, the phosphoramidite monomer **A1** synthesized in Example 3 was used at the corresponding positions.

The synthesis process is briefly described below: Nucleoside phosphoramidite monomers were linked one by one according to a synthesis program on a Dr. Oligo48 synthesizer (Biolytic), with a Universal CPG support as a start. Apart from the nucleoside phosphoramidite monomer **A1** described in Example 3, 2'-F RNA, 2'-O-methyl RNA, and other nucleoside phosphoramidite monomers were purchased from Shanghai Hongene or Suzhou Genepharma. 5-Ethylthio-1H-tetrazole (ETT) was used as an activator (0.6 M in acetonitrile), and a 0.22 M solution of PADS in acetonitrile and collidine (1:1 by volume) (Suzhou Kroma) as a sulfurizing agent; phosphorothioate could be introduced to a designated position. An iodopyridine/water solution (Kroma) was used as an oxidant; oxophosphate could be introduced to a designated position.

After solid-phase synthesis, the 5'-end protecting groups needed to be removed using TMSI in certain examples. For example, after solid-phase synthesis using phosphoramidite monomer **A1**, the crude oligoribonucleotide product was

suspended in anhydrous acetonitrile, TMSI was added at room temperature, and after sufficient stirring, the ethyl (Et) groups in the oligoribonucleotide were removed, yielding an oligoribonucleotide with NA0127. Then, the oligoribonucleotide was further cleaved from the solid support under the following conditions: soaking in a 3:1 solution of 28% ammonia water and ethanol at 50 °C for 16 h. Centrifugation was then performed, and the supernatant was transferred to another centrifuge tube. After the supernatant was concentrated to dryness by evaporation, the residue was purified by C18 reversed-phase chromatography with 0.1 M TEAA and acetonitrile as the mobile phase, and DMTr was removed using a 3% trifluoroacetic acid solution. The target oligonucleotide was collected, then lyophilized, identified as the target product by LC-MS, and quantified by UV (260 nm).

The single-stranded oligonucleotides obtained were paired in an equimolar ratio in a complementary manner and annealed. The final dsRNAs were dissolved in 1× PBS, and the solutions were adjusted to the concentration required for the experiment so they were ready for use.

The sequences of sense and antisense strands with deuterated chemical modifications at their 5' ends, 2'-fluoro modifications, 2'-methoxy modifications, etc. are detailed in Table 1 and Table 2.

**Example 5: Synthesis of dsRNA Conjugates**

1. In-house preparation of resin with support

The carboxylic acid group-containing compound NAG0052 (157 mg, 0.062 mmol) was dissolved in anhydrous DMF (3 mL). After the substrate was completely dissolved, anhydrous acetonitrile (4 mL), DIEA (0.03 mL, 0.154 mmol, 2.5 eq), and HBTU (35 mg, 0.093 mmol, 1.5 eq) were sequentially added. After the reaction mixture was mixed well, macroporous aminomethyl resin (476 mg, blank loading 0.41 mmol/g, target loading 0.1 mmol/g) was added. The reaction mixture was shaken overnight on a shaker (temperature: 25 °C; rotational speed: 200 rpm). The reaction mixture was filtered, and the filter cake was washed with DCM and then with anhydrous acetonitrile. The solid was collected and dried overnight *in vacuo.*

The solid from the previous step was dispersed in anhydrous acetonitrile (5 mL), and pyridine (0.18 mL), DMAP (3 mg), NMI (0.12 mL), and CapB1 (2.68 mL) were sequentially added. The reaction mixture was shaken on a shaker (temperature: 25 °C; rotational speed: 200 rpm) for 2 h. The reaction mixture was filtered, and the filter cake was washed with anhydrous acetonitrile. The solid was collected and dried overnight *in vacuo* to give a resin with a support. The loading measured 0.1 mmol/g.

2. For NAG0052 that had been attached to the resin, the resin was used as a start, and nucleoside monomers were attached one by one in the 3'-5' direction in the order in which nucleotides were arranged. The linking of each nucleoside monomer involved four reactions: deprotection, coupling, capping, and oxidation or sulfurization. The procedure is conventional in the art.

Compound NAG0052 was attached to the sequence through solid-phase synthesis, and after aminolysis, some of the functional groups of the NAG0052 structure were removed, resulting in NAG0052'. 3. Related conjugates were synthesized according to the above steps using L96 in place of NAG0052. L96 was prepared using the method described in patent application No. WO2014025805A.

The prepared dsRNA conjugates had the sense and antisense strands shown in Table 1 and Table 2.

The positive control dsRNA conjugate TRD007790 was prepared using the method described in patent application No. WO2017059223A.

Table 1. A list of dsRNAs

| dsRNA No. | Sense strand No. | Antisense strand No. |
|---|---|---|
| TJR100396 | S0187 | A0258 |
| TJR100422 | | A0276 |
| TJR100423 | | A0277 |
| TJR100424 | | A0278 |
| TJR100425 | | A0279 |
| TJR100426 | | A0280 |
| TJR100427 | | A0281 |
| TJR100428 | | A0282 |
| TJR100429 | | A0283 |
| TJR100430 | | A0284 |
| TJR100431 | | A0285 |
| TJR100432 | | A0286 |
| TJR101079 | | A0552 |
| TJR101081 | | A0825 |
| TJR101085 | | A0556 |
| TJR101087 | | A0827 |
| TJR102136 | | A1764 |
| TJR100437 | S0277 | A0160 |
| TJR100800 | | A0545 |
| TJR100801 | | A0546 |
| TJR100802 | | A0547 |
| TJR100803 | | A0548 |
| TJR100804 | | A0549 |
| TJR100805 | | A0550 |
| TJR100806 | | A0551 |
| TJR100807 | | A0552 |
| TJR100808 | | A0553 |
| TJR100809 | | A0554 |
| TJR100810 | | A0555 |
| TJR100811 | | A0556 |
| TJR101080 | | A0825 |
| TJR101084 | | A0826 |
| TJR101086 | | A0827 |
| TJR101082 | S0736 | A0825 |
| TJR101083 | | A0552 |
| TJR101088 | | A0556 |
| TRD007790 | TJR014640S | TJR014643A |
| TJR102134 | S1757 | A0552 |

Table 2. The sense and antisense strands of dsRNA conjugates

| | Sense strand No. | SEQ ID NO | Sequence direction 5'-3' |
|---|---|---|---|
| Sense strand | S0187 | 3 | GmsCmsUmCmCmUmUfAfUfUmGmUmUm AmUm AmCmGmsAm-**NAG0052'** |
| | S0277 | 4 | GmsCmsUmCmCmUmUfAfUfUmGmUmUm AmUm AmCmGmAm-**NAG0052'** |
| | S0736 | 5 | GmsCmsUmCmCmUmUfAfUfUmGmUmUm AmUm AmCmGmAm-**L96'** |
| | S0187-1 | 6 | GmsCmsUmCmCmUmUfAfUfUmGmUmUm AmUm AmCmGmsAm |
| | S0277-1 | 7 | GmsCmsUmCmCmUmUfAfUfUmGmUmUm AmUm AmCmGmAm |
| | TJR014640S | 52 | **NAG25**s'-CmsAmGmCmCmCmCmUmUfAfUfUm GmUmUmAmUmAmCmGms(invdA) |
| | S1757 | 50 | GmsCmsUmsCmCmUmUfAfUfUmGmUmUm AmUmAmCmGmAm-**NAG0052'** |
| Antisense strand | A1764 | 51 | UmsCfsGmUfAmUfAmAmCmAmAmUfAmA fGmGmAmGmCmsUmsGm |
| | A0160 | 8 | UmsCfsGmUfAmUfAmAmCmAfAmUfAmAf GmGfAmGfCmsUmsGm |
| | A0545 | 9 | UmsCfsGmUfAmUfAmAmCmAfAmUfAmAf GmGfAmGmCmsUmsGm |
| | A0546 | 10 | UmsCfsGmUfAmUfAmAmCmAfAmUmAmA fGmGfAmGfCmsUmsGm |
| | A0547 | 11 | UmsCfsGmUfAmUfAmAmCmAmAmUfAmA fGmGfAmGfCmsUmsGm |
| | A0548 | 12 | UmsCfsGmUmAmUfAmAmCmAfAmUfAmA fGmGfAmGfCmsUmsGm |
| | A0549 | 13 | UmsCfsGmUmAmUfAmAmCmAmAmUmA mAfGmGfAmGmCmsUmsGm |
| | A0550 | 14 | UmsCfsGmUfAmUfAmAmCmAmAmUmAm AfGmGfAmGmCmsUmsGm |
| | A0551 | 15 | UmsCfsGmUmAmUfAmAmCmAfAmUmAm AfGmGfAmGmCmsUmsGm |

(continued)

| | Sense strand No. | SEQ ID NO | Sequence direction 5'-3' |
|---|---|---|---|
| | A0552 | 16 | UmsCfsGmUmAmUfAmAmCmAmAmUfAmAfGmGfAmGmCmsUmsGm |
| | A0553 | 17 | UmsCfsGmUmAmUmAmAmCmAmAmUmAmAfGmGmAmGmCmsUmsGm |
| | A0554 | 18 | UmsCfsGmUmAmUfAmAmCmAmAmUmAmAfGmGmAmGmCmsUmsGm |
| | A0555 | 19 | UmsCfsGmUmAmUmAmAmCmAmAmUmAmAfGmGfAmGmCmsUmsGm |
| | A0556 | 20 | UmsCfsGmUmAmUmAmAmCmAmAmUfAmAfGmGmAmGmCmsUmsGm |
| | A0258 | 21 | UmsCfsGmUfAmUf(-)hmpNA(A)AmCmAfAmUfAmAfGmGfAmGfCmsUmsGm |
| | A0276 | 22 | UmsCfsGmUfAmUf(-)hmpNA(A)AmCmAfAmUfAmAfGmGfAmGmCmsUmsGm |
| | A0277 | 23 | UmsCfsGmUfAmUf(-)hmpNA(A)AmCmAfAmUmAmAfGmGfAmGfCmsUmsGm |
| | A0278 | 24 | UmsCfsGmUfAmUf(-)hmpNA(A)AmCmAmAmUfAmAfGmGfAmGfCmsUmsGm |
| | A0279 | 25 | UmsCfsGmUmAmUf(-)hmpNA(A)AmCmAfAmUfAmAfGmGfAmGfCmsUmsGm |
| | A0280 | 26 | UmsCfsGmUmAmUf(-)hmpNA(A)AmCmAmAmUmAmAfGmGfAmGmCmsUmsGm |
| | A0281 | 27 | UmsCfsGmUfAmUf(-)hmpNA(A)AmCmAmAmUmAmAfGmGfAmGmCmsUmsGm |
| | A0282 | 28 | UmsCfsGmUmAmUf(-)hmpNA(A)AmCmAfAmUmAmAfGmGfAmGmCmsUmsGm |
| | A0283 | 29 | UmsCfsGmUmAmUf(-)hmpNA(A)AmCmAmAmUfAmAfGmGfAmGmCmsUmsGm |
| | A0284 | 30 | UmsCfsGmUmAmUm(-)hmpNA(A)AmCmAmAmUmAmAfGmGmAmGmCmsUmsGm |
| | A0285 | 31 | UmsCfsGmUmAmUf(-)hmpNA(A)AmCmAmAmUmAmAfGmGmAmGmCmsUmsGm |

(continued)

| | Sense strand No. | SEQ ID NO | Sequence direction 5'-3' |
|---|---|---|---|
| | A0286 | 32 | UmsCfsGmUmAmUm(-)hmpNA(A)AmCmA mAmUmAmAfGmGfAmGmCmsUmsGm |
| | A0825 | 33 | NA0127CfsGmUmAmUfAmAmCmAmAmUf AmAfGmGfAmGmCmsUmsGm |
| | A0826 | 34 | NA0149CfsGmUmAmUfAmAmCmAmAmUf AmAfGmGfAmGmCmsUmsGm |
| | A0827 | 35 | NA0127CfsGmUmAmUmAmAmCmAmAmU fAmAfGmGmAmGmCmsUmsGm |
| | TJR014643A | 53 | UmsCfsGmUfAmUfAmAmCmAmAmUfAmA fGmGfGmGfCmsUfsGm |

In the above table, the uppercase letters G, A, C, and U represent nucleotides containing guanine, adenine, cytosine, and uracil, respectively; the lowercase letter m represents a 2'-methoxy modification; the lowercase letter f represents a 2'-fluoro modification; the lowercase letter s means that the two nucleotides directly adjacent to the letter s are linked by a phosphorothioate diester group;

(-)hmpNA(A) represents

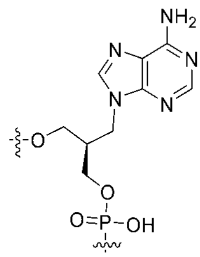

;

the structure of NAG0052' is:

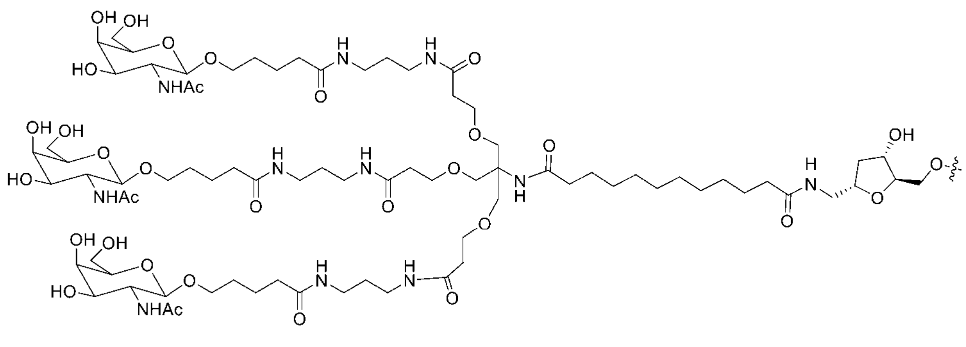

;

the structure of L96' is:

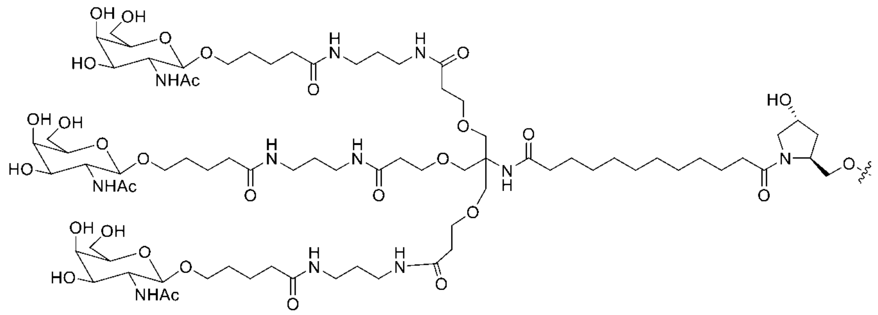

;

the structure of NAG25s' is:

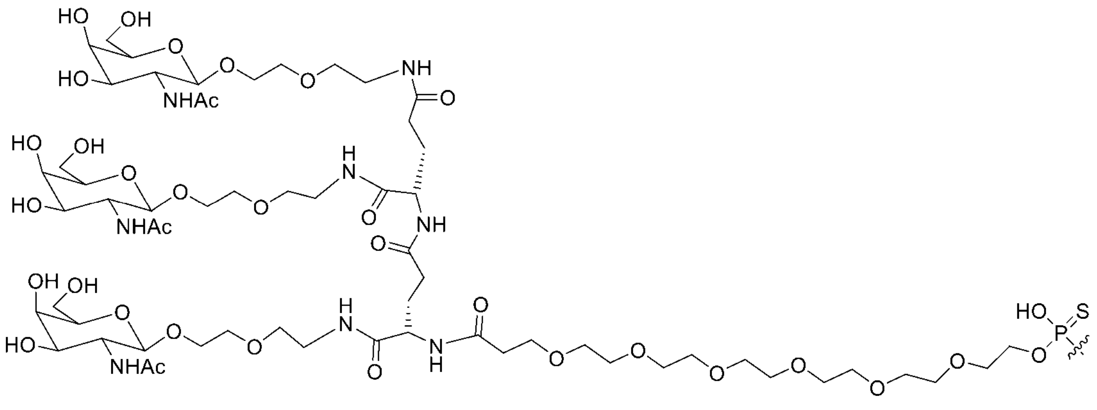

NA0127, NA0149, and InvdA represent:

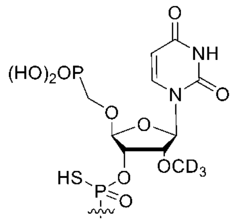

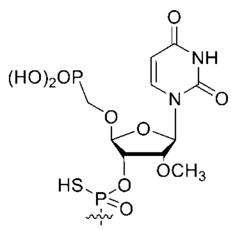

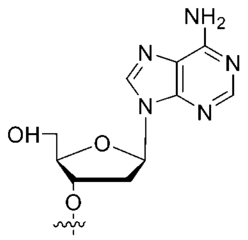

**NA0127** **NA0149** **InvdA**

NA0127 was introduced into dsRNAs using the method described in Example 4. NA0149 was introduced into dsRNAs using the synthesis method in US20190177729A. InvdA was introduced into dsRNAs using the synthesis method in WO2017059223A.

Table 3. Unmodified sequences corresponding to the sense and antisense strands of dsRNA conjugates

| | Chain No. corresponding to unmodified sequence | SEQ ID NO | Unmodified sequence, sequence direction 5'-3' |
|---|---|---|---|
| Sense strand | S0187, S0277, S0736, S1757 | 1 | GCUCCUUAUUGUUAUACGA |

(continued)

| | Chain No. corresponding to unmodified sequence | SEQ ID NO | Unmodified sequence, sequence direction 5'-3' |
|---|---|---|---|
| Antisense strand | A0258, A0276, A0277, A0278, A0279, A0280, A0281, A0282, A0283, A0284, A0285, A0286, A0160, A0545, A0546, A0547, A0548, A0549, A0550, A0551, A0552, A0553, A0554, A0555, A0556, A0825, A0826, A0827, A1764 | 2 | UCGUAUAACAAUAAGGAGCUG |

**Example 6: Design of Human LPA dsRNAs**

With human LPA (NM_005577.3) as a target gene, dsRNAs of 19/21nt were designed in accordance with the general rules for active dsRNAs. The sequences of unmodified sense and antisense strands are detailed in Table 4 and Table 5.

Table 4. The unmodified sense and antisense strands of a human LPA dsRNA

| Double strand No. of unmodified sequence | SEQ ID NO | Unmodified sense strand (5'-3') | SEQ ID NO | Unmodified antisense strand (5'-3') |
|---|---|---|---|---|
| TJR100373 | 1 | GCUCCUUAUUGUUAUACGA | 2 | UCGUAUAACAAUAAGGAGCUG |

Table 5. The unmodified sense and antisense strands of human LPA dsRNAs (control group)

| Double strand No. of unmodified sequence | SEQ ID NO | Unmodified sense strand (5'-3') | SEQ ID NO | Unmodified antisense strand (5'-3') |
|---|---|---|---|---|
| TJR100374 | 36 | CUCCUUAUUGUUAUACGAG | 37 | CUCGUAUAACAAUAAGGAGCU |
| TJR100375 | 38 | UCCUUAUUGUUAUACGAGG | 39 | CCUCGUAUAACAAUAAGGAGC |
| TJR100376 | 40 | AGCUCCUUAUUGUUAUACG | 41 | CGUAUAACAAUAAGGAGCUGC |
| TJR100377 | 42 | CAGCUCCUUAUUGUUAUAC | 43 | GUAUAACAAUAAGGAGCUGCC |
| TJR100378 | 44 | GCGCCUUAUUGUUAUACGA | 45 | UCGUAUAACAAUAAGGCGCUG |
| TJR100379 | 46 | GCACCUUAUUGUUAUACGA | 47 | UCGUAUAACAAUAAGGUGCUG |
| TJR100380 | 48 | CAGCCCCUUAUUGUUAUACGA | 49 | UCGUAUAACAAUAAGGGGCUG |

**Example 7: psiCHECK 9-Concentration-Point On-Target Activity of dsRNA Conjugates**

dsRNAs/dsRNA conjugates were subjected to an *in vitro* molecular-level simulation of on-target activity screening in HEK293A cells using 9 concentration gradients.

HEK293A cells were cultured at 37 °C with 5% $CO_2$ in a DMEM high glucose culture medium containing 10% fetal bovine serum. 24 h prior to transfection, the HEK293A cells were seeded into a 96-well plate at a density of $8 \times 10^3$ cells per well, with each well containing 100 μL of the culture medium.

The cells were co-transfected with the dsRNA conjugates and corresponding plasmids using Lipofectamine2000 (ThermoFisher, 11668019) according to the instructions, with 0.3 μL of Lipofectamine2000 used per well. The amount of plasmids for transfection was 40 ng per well. For on-target sequence plasmids, a total of 9 concentration points were set for the dsRNAs/dsRNA conjugates; the highest concentration point final concentration was 20 nM, and a 3-fold serial dilution was performed, resulting in 20 nM, 6.666666667 nM, 2.222222222 nM, 0.740740741 nM, 0.24691358 nM, 0.082304527 nM, 0.027434842 nM, 0.009144947 nM, and 0.003048316 nM. 24 h after transfection, the on-target levels were determined using a Dual-Luciferase Reporter Assay System (Promega, E2940).

The results are shown in Table 6.

Table 6. The psi-Check system 9-point activity of dsRNAs/dsRNA conjugates

| Remaining percentage of target gene mRNA expression (mean) | | | | | |
|---|---|---|---|---|---|
| Double strand code | 20 nM | 6.6667 nM | 2.2222 nM | 0.7407 nM | 0.2469 nM |
| TJR100373 | 16.0% | 11.8% | 13.6% | 9.2% | 14.5% |
| TJR100374 | 13.3% | 18.8% | 16.8% | 23.3% | 19.2% |
| TJR100375 | 22.8% | 16.8% | 16.2% | 25.0% | 35.5% |
| TJR100376 | 25.8% | 21.3% | 20.5% | 20.2% | 22.3% |
| TJR100377 | 27.5% | 16.7% | 24.1% | 30.5% | 30.2% |
| TJR100378 | 24.5% | 28.1% | 26.9% | 20.5% | 34.7% |
| TJR100379 | 23.8% | 21.6% | 22.9% | 19.6% | 23.0% |
| TJR100380 | 23.6% | 16.5% | 21.9% | 17.3% | 29.6% |
| TJR100807 | 10.8% | 11.1% | 15.1% | 25.4% | 48.2% |
| TJR101079 | 9.6% | 10.1% | 13.2% | 20.4% | 40.5% |
| TJR101080 | 8.4% | 9.4% | 9.5% | 11.4% | 15.6% |
| TJR101081 | 10.0% | 10.3% | 11.1% | 12.3% | 18.0% |
| TJR101082 | 9.1% | 9.7% | 10.8% | 12.7% | 20.0% |
| TJR101083 | 8.4% | 10.2% | 16.9% | 28.2% | 55.5% |
| TJR101084 | 8.8% | 8.7% | 10.1% | 12.2% | 19.3% |
| TJR100810 | 8.9% | 10.6% | 14.7% | 24.7% | 45.6% |
| TJR100811 | 9.4% | 11.2% | 15.6% | 28.0% | 55.5% |
| TJR101085 | 8.6% | 9.8% | 13.3% | 22.1% | 45.8% |
| TJR101086 | 8.5% | 8.3% | 8.9% | 10.3% | 15.4% |
| TJR101087 | 8.2% | 8.5% | 9.2% | 10.9% | 16.7% |
| TJR101088 | 9.3% | 11.7% | 17.4% | 30.8% | 57.0% |
| TJR100437 | 30.8% | 25.5% | 26.5% | 34.3% | 42.4% |
| TJR100800 | 28.2% | 22.5% | 28.7% | 38.6% | 52.4% |
| TJR100801 | 28.8% | 21.9% | 27.1% | 36.0% | 50.0% |
| TJR100802 | 32.2% | 31.0% | 29.3% | 32.3% | 49.9% |
| TJR100803 | 27.1% | 27.8% | 30.7% | 46.5% | 58.8% |
| TJR100804 | 31.3% | 27.4% | 33.4% | 47.8% | 61.3% |
| TJR100805 | 28.5% | 24.5% | 24.1% | 31.4% | 47.0% |
| TJR100806 | 24.3% | 23.0% | 24.5% | 34.6% | 50.7% |

(continued)

| Remaining percentage of target gene mRNA expression (mean) | | | | | |
|---|---|---|---|---|---|
| Double strand code | 20 nM | 6.6667 nM | 2.2222 nM | 0.7407 nM | 0.2469 nM |
| TJR100808 | 28.2% | 26.1% | 27.3% | 41.0% | 53.0% |
| TJR100809 | 30.9% | 28.9% | 30.5% | 41.3% | 56.7% |
| TJR100396 | 16.7% | 17.9% | 24.3% | 30.3% | 46.7% |
| TJR100422 | 20.2% | 28.8% | 27.9% | 42.1% | 67.7% |
| TJR100423 | 26.1% | 19.9% | 19.7% | 38.9% | 50.2% |
| TJR100424 | 19.0% | 19.6% | 21.0% | 32.5% | 56.7% |
| TJR100425 | 22.5% | 20.8% | 32.3% | 42.4% | 74.6% |
| TJR100426 | 19.1% | 21.6% | 32.1% | 44.2% | 76.3% |
| TJR100427 | 17.2% | 19.8% | 28.2% | 38.8% | 67.2% |
| TJR100428 | 18.6% | 17.3% | 27.3% | 42.4% | 67.9% |
| TJR100429 | 19.1% | 18.5% | 28.5% | 50.2% | 71.4% |
| TJR100430 | 19.8% | 23.3% | 37.2% | 59.4% | 80.8% |
| TJR100431 | 19.9% | 25.2% | 29.2% | 55.0% | 73.0% |
| TJR100432 | 16.0% | 18.8% | 30.2% | 50.5% | 67.9% |
| Double strand code | 0.0823 nM | 0.0274 nM | 0.0091 nM | 0.0030 nM | IC50 (nM) |
| TJR100373 | 21.8% | 28.8% | 49.0% | 85.5% | 0.009 |
| TJR100374 | 40.0% | 53.9% | 80.1% | 97.3% | 0.037 |
| TJR100375 | 46.8% | 56.8% | 88.3% | 80.0% | 0.068 |
| TJR100376 | 25.7% | 55.6% | 54.7% | 95.8% | 0.016 |
| TJR100377 | 58.7% | 85.1% | 143.4% | 86.7% | 0.103 |
| TJR100378 | 45.7% | 59.8% | 91.8% | 128.4% | 0.048 |
| TJR100379 | 55.3% | 98.9% | 91.1% | 100.7% | 0.091 |
| TJR100380 | 53.0% | 73.1% | 93.7% | 88.5% | 0.092 |
| TJR100807 | 74.7% | 90.7% | 89.2% | 101.6% | 0.230 |
| TJR101079 | 65.4% | 88.5% | 93.9% | 99.6% | 0.161 |
| TJR101080 | 30.4% | 55.8% | 81.8% | 102.9% | 0.033 |
| TJR101081 | 32.2% | 55.1% | 86.6% | 104.6% | 0.035 |
| TJR101082 | 37.6% | 65.3% | 96.4% | 104.9% | 0.050 |
| TJR101083 | 80.7% | 96.1% | 92.8% | 93.5% | 0.308 |
| TJR101084 | 34.7% | 71.3% | 92.2% | 102.9% | 0.052 |
| TJR100810 | 74.6% | 93.9% | 92.4% | 103.3% | 0.217 |
| TJR100811 | 82.4% | 104.5% | 108.6% | 115.9% | 0.288 |
| TJR101085 | 75.2% | 100.0% | 102.6% | 103.5% | 0.211 |
| TJR101086 | 27.3% | *55.5%* | 81.1% | 100.2% | 0.032 |
| TJR101087 | 31.6% | 64.3% | 93.0% | 102.9% | 0.043 |
| TJR101088 | 83.7% | 101.4% | 111.8% | 106.5% | 0.318 |
| TJR100437 | 74.9% | 91.8% | 110.3% | 112.7% | 0.195 |
| TJR100800 | 95.1% | 105.7% | 111.6% | 135.7% | 0.357 |

(continued)

| Double strand code | 0.0823 nM | 0.0274 nM | 0.0091 nM | 0.0030 nM | IC50 (nM) |
|---|---|---|---|---|---|
| TJR100801 | 72.7% | 85.4% | 92.0% | 90.5% | 0.258 |
| TJR100802 | 66.2% | 84.6% | 98.6% | 100.6% | 0.199 |
| TJR100803 | 93.1% | 89.6% | 96.7% | 105.2% | 0.491 |
| TJR100804 | 99.7% | 111.6% | 105.9% | 119.1% | 0.475 |
| TJR100805 | 70.5% | 81.6% | 96.9% | 102.0% | 0.200 |
| TJR100806 | 79.7% | 86.3% | 88.0% | 85.0% | 0.277 |
| TJR100808 | 67.3% | 74.1% | 88.9% | 99.1% | 0.270 |
| TJR100809 | 69.9% | 91.1% | 98.4% | 95.5% | 0.330 |
| TJR100396 | 78.9% | 93.3% | 98.2% | 103.3% | 0.240 |
| TJR100422 | 85.5% | 96.4% | 98.8% | 94.8% | 0.511 |
| TJR100423 | 77.8% | 90.4% | 97.3% | 102.5% | 0.279 |
| TJR100424 | 76.1% | 80.8% | 91.7% | 95.8% | 0.305 |
| TJR100425 | 89.5% | 116.0% | 99.7% | 104.9% | 0.574 |
| TJR100426 | 90.4% | 95.8% | 107.2% | 107.0% | 0.673 |
| TJR100427 | 95.4% | 109.3% | 100.5% | 95.6% | 0.467 |
| TJR100428 | 81.9% | 91.7% | 95.6% | 86.7% | 0.545 |
| TJR100429 | 104.4% | 101.1% | 101.3% | 94.0% | 0.684 |
| TJR100430 | 96.7% | 108.6% | 109.8% | 95.7% | 0.925 |
| TJR100431 | 87.9% | 96.7% | 93.0% | 94.1% | 0.813 |
| TJR100432 | 99.7% | 96.8% | 93.3% | 104.5% | 0.705 |

**Example 8: Inhibition of Human LPA in Primary Human Hepatocytes (PHHs) by dsRNAs - 7-Concentration-Point Inhibitory Activity**

dsRNA sequences were subjected to PHH activity screening using 7 concentration gradients in primary human hepatocytes (PHHs). The initial final concentration for transfection of each dsRNA sample was 20 nM, and a 5-fold serial dilution was performed to obtain 7 concentration points.

PHHs were cryopreserved in liquid nitrogen. 24 h prior to transfection, the primary human hepatocytes (PHHs) were thawed and then seeded into a 96-well plate at a density of 3 × 104 cells per well, with each well containing 80 μL of a culture medium.

The cells were transfected with the dsRNAs using Lipofectamine RNAi MAX (ThermoFisher, 13778150) according to the product instructions; the final gradient concentrations of the dsRNAs for transfection were 20 nM, 4 nM, 0.8 nM, 0.16 nM, 0.032 nM, 0.0064 nM, and 0.00128 nM. 24 h after treatment, total cell RNA extraction was performed using a high-throughput cell RNA extraction kit, and RNA reverse transcription and quantitative real-time PCR detection were performed to determine the mRNA level of human LPA. The mRNA level of human LPA was corrected based on the GAPDH internal reference gene level.

In the quantitative real-time PCR detection, probe Q-PCR detection was used; information on the primers is shown in Table 7 and Table 8.

After the Taqman probe Q-PCR detection was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2 - ΔΔCt, where ΔΔCt = [(target gene of Ct experimental group - internal reference of Ct experimental group) - (target gene of Ct control group - internal reference of Ct control group)].

$$\text{Inhibition rate (\%)} = (1 - \text{remaining level of target gene expression}) \times 100\%.$$

The results are expressed relative to the remaining percentage of human LPA mRNA expression in cells treated with the control dsRNA. The inhibition rate IC50 results are shown in Table 9.

The results show that TJR100373 had a significant advantage over TJR100374 to TJR100380 in activity, indicating that the target gene fragment targeted by TJR100373 is more suitable as a target.

Table 7. Taqman LPA probe primers

| Primer name | Primer sequence |
|---|---|
| hLPA-PF-MGB | TGGAGGGCCTCTGGTTTG(SEQ ID NO:54) |
| hLPA-PR-MGB | GGCGTGCACAGCCAAGAC(SEQ ID NO:55) |
| hLPA-P-MGB | 5`6-FAM- CATTTTACAAGGAGTCACTTC-3`MGB(SEQ ID NO:56) |

Table 8. A Taqman GAPDH probe primer

| Primer name | Brand | Cat. No. |
|---|---|---|
| Human GAPDH TaqMan Probe | Thermo | 4326317E |

Table 9. The multi-dose inhibitory activity of dsRNAs against human LPA in primary human hepatocytes (PHHs)

| Double strand No. | 20 nM | 4 nM | 0.8 nM | 0.16 nM | 0.032 nM | 0.0064 nM | 0.00128 nM | Primary human hepatocyte IC50 (nM) |
|---|---|---|---|---|---|---|---|---|
| TJR100373 | 29.1% | 36.6% | 31.9% | 39.7% | 45.6% | 71.4% | 79.8% | 0.026 |
| TJR100374 | 34.6% | 38.3% | 46.9% | 57.2% | 62.8% | 81.6% | 100.4% | 0.348 |
| TJR100375 | 51.2% | 51.7% | 52.7% | 59.8% | 78.9% | 96.5% | 114.6% | 2.710 |
| TJR100376 | 44.9% | 46.6% | 44.7% | 46.0% | 65.1% | 85.4% | 112.0% | 0.150 |
| TJR100377 | 40.1% | 44.5% | 54.3% | 55.6% | 69.5% | 100.5% | 115.7% | 0.479 |
| TJR100378 | 44.4% | 55.5% | 55.0% | 59.8% | 71.6% | 88.8% | 92.5% | 5.272 |
| TJR100379 | 41.7% | 59.9% | 56.4% | 62.0% | 73.6% | 80.1% | 104.4% | 7.244 |
| TJR100380 | 38.6% | 50.7% | 56.7% | 63.1% | 79.4% | 91.7% | 112.3% | 2.254 |

**Example 9: Assay for *In Vivo* Activity of dsRNA Conjugate in Humanized Mice (hu-Lp(a))**

The construction of the humanized mice (hu-Lp(a)) used in this example was entrusted by Tuojie Biotech (Shanghai) Co., Ltd. to Cyagen (Suzhou) Biosciences Co., Ltd.

The mice were evenly divided into 4 groups of 6 (2 males and 4 females) according to their serum Lp(a) protein content, and the groups were given normal saline, the positive control TRD007790, and the dsRNA conjugate TJR101079 of the present disclosure, respectively, via subcutaneous injection at a dose of 3 mg/kg and a volume of 10 μL/g. Before the administration on the administration day, 40 μL of serum was collected, and the determined concentrations of serum Lp(a) were used as basic data. The administration day was defined as day 1 (D1). On day 8 (D8), day 29 (D29), day 57 (D57), day 85 (D85), and day 99 (D99) after the administration, 40 μL of serum was collected each time, and the serum Lp(a) protein content was determined using an Abbott Ci4100 fully automatic biochemical immunoassay instrument. The inhibition of serum Lp(a) protein expression in humanized mice (hu-Lp(a)) by the dsRNA conjugate was calculated. The normalized serum Lp(a) protein ratio of each treatment group to the blank control group was calculated, and the results are shown in Table 10. Each group was statistically analyzed relative to the blank treatment group using one-way ANOVA.

It can be seen from the results in Table 10 that on day 99, there was no statistical difference between the blank control group and the positive control group TRD007790, while TJR101079 still significantly inhibited Lp(a) protein expression with statistical significance ($p < 0.01$), indicating that on day 99, TRD007790 no longer exhibited significant inhibitory activity, while TJR101079 still exhibited significant inhibitory activity.

This indicates that the dsRNA conjugate TJR101079 of the present disclosure has an excellent long-acting inhibitory effect on Lp(a) protein expression.

Table 10. The Lp(a) protein concentrations in the serum of humanized mice (hu-Lp(a))

| Group | Normalized serum Lp(a) protein ratio relative to blank | | | | |
|---|---|---|---|---|---|
| | Day 8 | Day 29 | Day 57 | Day 85 | Day 99 |
| Normal saline | 1±0.36 | 1±0.34 | 1±0.35 | 1±0.36 | 1±0.32 |
| TRD007790 | 0.04±0.02**** | 0.05±0.03**** | 0.24±0.11**** | 0.67±0.18* | 0.78±0.2 |
| TJR101079 | 0.04±0.04**** | 0.04±0.02**** | 0.15±0.06**** | 0.38±0.15*** | 0.5±0.18** |
| In Table 10, * represents $p \leq 0.05$; ** represents $p < 0.01$; *** represents $p < 0.001$; **** represents $p < 0.0001$. | | | | | |

**Example 10: Assay for *In Vivo* Activity of dsRNA Conjugate in Humanized Mice (hu-Lp(a))**

The construction of the humanized mice (hu-Lp(a)) used in this example was entrusted by Tuojie Biotech (Shanghai) Co., Ltd. to Cyagen (Suzhou) Biosciences Co., Ltd.

The mice were evenly divided into 4 groups of 6 according to their serum Lp(a) protein content, and the groups were given the dsRNA conjugates TRD007790, TJR101079, TJR102134, and TJR102136, respectively, via subcutaneous injection at a dose of 1 mg/kg and a volume of 10 μL/g. Before the administration on the administration day, 40 μL of serum was collected, and the determined concentrations of serum Lp(a) were used as basic data. The administration day was defined as day 1 (D1). On day 28 (D28) after the administration, 40 μL of serum was collected each time, and the serum Lp(a) protein content was determined using an Abbott Ci4100 fully automatic biochemical immunoassay instrument. The inhibition of serum Lp(a) protein expression in humanized mice (hu-Lp(a)) by the dsRNA conjugates was calculated. The normalized serum Lp(a) protein ratio of each treatment group to the blank control group was calculated, and the results are shown in FIG. 1A, FIG. 1B, and FIG. 1C. The D28 serum Lp(a) concentration of each group was compared with the serum Lp(a) concentration before administration to obtain a remaining serum Lp(a) ratio, and TJR101079 was statistically compared with TRD007790, TJR102134, and TJR102136 using Paired t tests.

It can be seen from the results in FIG. 1A, FIG. 1B, and FIG. 1C that the ratio of the day-28 serum Lp(a) concentration to the serum Lp(a) concentration before administration of TJR101079 significantly differed from those of TRD007790, TJR102134, and TJR102136. Between TJR101079 and TRD007790, "*" $p < 0.05$; between TJR101079 and TJR102134, "*" $p < 0.05$; between TJR101079 and TJR102136, "**" $p < 0.01$.

This indicates that the long-acting inhibitory effect of TJR101079 on Lp(a) protein expression was superior to those of the control group TRD007790, TJR102134, and TJR102136; that is, the long-acting inhibitory effect of the dsRNA conjugate of the present disclosure on Lp(a) protein expression was significantly better than those of the comparative conjugates.

## Claims

**1.** An LPA-targeting double-stranded ribonucleic acid (dsRNA), comprising a sense strand and an antisense strand that form a double-stranded region, wherein:

the unmodified nucleotide sequence of the sense strand comprises at least 17 contiguous nucleotides that differ from the nucleotide sequence of SEQ ID NO: 1 by no more than 3 nucleotides, and
the unmodified nucleotide sequence of the antisense strand comprises at least 19 contiguous nucleotides that differ from the nucleotide sequence of SEQ ID NO: 2 by no more than 3 nucleotides,
wherein in the direction from the 5' end to the 3' end,
the nucleotides at positions 7, 8, and 9 of the sense strand are 2'-fluoro-modified nucleotides, and the nucleotides at the other positions are 2'-methoxy-modified nucleotides;
the nucleotides at positions 2 and 14 of the antisense strand are 2'-fluoro-modified nucleotides, the nucleotides at positions 4, 6, 10, 12, 16, and 18 are independently 2'-methoxy-modified or 2'-fluoro-modified nucleotides, and the nucleotides at the other positions are 2'-methoxy-modified nucleotides;
the number of 2'-fluoro-modified nucleotides in the antisense strand is 2-7.

**2.** The dsRNA according to claim 1, wherein:
the unmodified nucleotide sequence of the sense strand comprises the nucleotide sequence set forth in SEQ ID NO: 1, and the unmodified nucleotide sequence of the antisense strand comprises the nucleotide sequence set forth in SEQ ID NO: 2.

**3.** The dsRNA according to claim 1 or 2, wherein the nucleotide at position 7 of the 5’ end of the antisense strand is a modified nucleotide, wherein:

- the modified nucleotide is a 2’-methoxy-modified nucleotide, or
- the modified nucleotide comprises a chemical modification represented by formula (I), (I-1), or (I-2) or a pharmaceutically acceptable salt thereof:

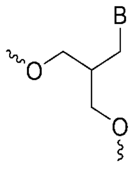 (I), 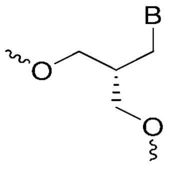 (I-1), or 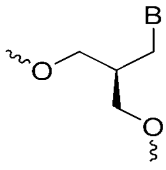 (I-2),

wherein B represents a base at a corresponding position of the nucleotide at position 7 of the 5’ end of the antisense strand.

**4.** The dsRNA according to any one of claims 1-3, wherein the nucleotide at position 1 of the 5’ end of the antisense strand is a modified nucleotide, wherein:

- the modified nucleotide is a 2’-methoxy-modified nucleotide, or
- the modified nucleotide is a chemically modified nucleotide represented by formula (II):

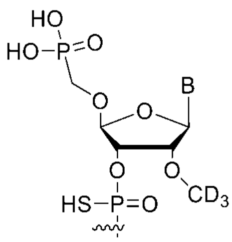 (II),

wherein B represents a base at a corresponding position of the nucleotide at position 1 of the 5’ end of the antisense strand.

**5.** The dsRNA according to any one of claims 1-4, wherein:

the sense strand comprises a nucleotide sequence represented by the following formula:

$$5'\text{-}N_aN_aN_aN_aN_aN_aN_bN_bN_bN_aN_aN_aN_aN_aN_aN_aN_aN_aN_a\text{-}3',$$

wherein $N_a$ is a 2’-methoxy-modified nucleotide, and $N_b$ is a 2’-fluoro-modified nucleotide.

**6.** The dsRNA according to any one of claims 1-5, wherein:

the antisense strand comprises a nucleotide sequence represented by the following formula:

$$5'\text{-}N_a'N_b'N_a'X'N_a'X'N_a'N_a'N_a'X'N_a'X'N_a'N_b'N_a'X'N_a'X'N_a'N_a'N_a'\text{-}3',$$

or

$$5'\text{-}N_a'N_b'N_a'X'N_a'X'W'N_a'N_a'X'N_a'X'N_a'N_b'N_a'X'N_a'X'N_a'N_a'N_a'\text{-}3',$$

or

$$5'\text{-}V'N_b'N_a'X'N_a'X'N_a'N_a'N_a'X'N_a'X'N_a'N_b'N_a'X'N_a'X'N_a'N_a'N_a'\text{-}3';$$

preferably, the antisense strand comprises a nucleotide sequence represented by the following formula:

$5'\text{-}N_a'N_b'N_a'N_a'N_a'N_b'N_a'N_a'N_a'N_a'N_a'N_b'N_a'N_b'N_a'N_b'N_a'N_a'N_a'N_a'N_a'\text{-}3'$,

$5'\text{-}\ N_a'N_b'N_a'N_b'N_a'Nb'N_a'N_a'N_a'N_b'N_a'N_b'N_a'N_b'N_a'N_b'N_a'N_a'N_a'N_a'N_a'\text{-}3'$,

$5'\text{-}N_a'N_b'N_a'N_b'N_a'N_b'N_a'N_a'N_a'N_b'N_a'N_a'N_a'N_b'N_a'N_b'N_a'N_b'N_a'N_a'N_a'\text{-}3'$,

$5'\text{-}N_a'N_b'N_a'N_b'N_a'N_b'N_a'N_a'N_a'N_a'N_a'N_b'N_a'N_b'N_a'N_b'N_a'N_b'N_a'N_a'N_a'\text{-}3'$,

$5'\text{-}N_a'N_b'N_a'N_a'N_a'N_b'N_a'N_a'N_a'N_b'N_a'N_b'N_a'N_b'N_a'N_b'N_a'N_b'N_a'N_a'N_a'\text{-}3'$,

$5'\text{-}N_a'N_b'N_a'N_a'N_a'N_b'N_a'N_a'N_a'N_a'N_a'N_a'N_a'N_b'N_a'N_b'N_a'N_a'N_a'N_a'N_a'\text{-}3'$,

$5'\text{-}N_a'N_b'N_a'N_b'N_a'N_b'N_a'N_a'N_a'N_a'N_a'N_a'N_a'N_b'N_a'N_b'N_a'N_a'N_a'N_a'N_a'\text{-}3'$,

$5'\text{-}N_a'N_b'N_a'N_a'N_a'N_b'N_a'N_a'N_a'N_b'N_a'N_a'N_a'N_b'N_a'N_b'N_a'N_a'N_a'N_a'N_a'\text{-}3'$,

$5'\text{-}N_a'N_b'N_a'N_a'N_a'N_a'N_a'N_a'N_a'N_a'N_a'N_a'N_a'N_b'N_a'N_a'N_a'N_a'N_a'N_a'N_a'\text{-}3'$,

$5'\text{-}N_a'N_b'N_a'N_a'N_a'N_b'N_a'N_a'N_a'N_a'N_a'N_a'N_a'N_8'N_a'N_a'N_a'N_a'N_a'N_a'N_a'\text{-}3'$,

$5'\text{-}N_a'N_b'N_a'N_a'N_a'N_a'N_a'N_a'N_a'N_a'N_a'N_a'N_a'N_b'N_a'N_b'N_a'N_a'N_a'N_a'N_a'\text{-}3'$,

$5'\text{-}N_a'N_b'N_a'N_a'N_a'N_a'N_a'N_a'N_a'N_a'N_a'N_b'N_a'N_b'N_a'N_a'N_a'N_a'N_a'N_a'N_a'\text{-}3'$,

$5'\text{-}N_a'N_b'N_a'N_b'N_a'N_b'W'N_a'N_a'N_b'N_a'N_b'N_a'N_b'N_a'N_b'N_a'N_a'N_a'N_a'N_a'\text{-}3'$,

$5'\text{-}N_a'N_b'N_a'N_b'N_a'N_b'W'N_a'N_a'N_b'N_a'N_a'N_a'N_b'N_a'N_b'N_a'N_b'N_a'N_a'N_a'\text{-}3'$,

$5'\text{-}N_a'N_b'N_a'N_b'N_a'N_d'W'N_a'N_a'N_a'N_a'N_b'N_a'N_b'N_a'N_b'N_a'N_b'N_a'N_a'N_a'\text{-}3'$,

$5'\text{-}N_a'N_b'N_a'N_a'N_a'N_b'W'N_a'N_a'N_b'N_a'N_b'N_a'N_b'N_a'N_b'N_a'N_b'N_a'N_a'N_a'\text{-}3'$,

$5'\text{-}N_a'N_b'N_a'N_a'N_a'N_b'W'N_a'N_a'N_a'N_a'N_a'N_a'N_b'N_a'N_b'N_a'N_a'N_a'N_a'N_a'\text{-}3'$,

$5'\text{-}N_a'N_b'N_a'N_b'N_a'N_b'W'N_a'N_a'N_a'N_a'N_a'N_a'N_b'N_a'N_b'N_a'N_a'N_a'N_a'N_a'\text{-}3'$,

$5'\text{-}N_a'N_b'N_a'N_a'N_a'N_b'W'N_a'N_a'N_b'N_a'N_a'N_a'N_b'N_a'N_b'N_a'N_a'N_a'N_a'N_a'\text{-}3'$,

$5'\text{-}N_a'N_b'N_a'N_a'N_a'N_b'W'N_a'N_a'N_a'N_a'N_b'N_a'N_b'N_a'N_b'N_a'N_a'N_a'N_a'N_a'\text{-}3'$,

$5'\text{-}N_a'N_b'N_a'N_a'N_a'N_a'W'N_a'N_a'N_a'N_a'N_a'N_a'N_d'N_a'N_a'N_a'N_a'N_a'N_a'N_a'\text{-}3'$,

$5'\text{-}N_a'N_b'N_a'N_a'N_a'N_b'W'N_a'N_a'N_a'N_a'N_a'N_a'N_b'N_a'N_a'N_a'N_a'N_a'N_a'N_a'\text{-}3'$,

$5'\text{-}N_a'N_b'N_a'N_a'N_a'N_a'W'N_a'N_a'N_a'N_a'N_a'N_a'N_b'N_a'N_b'N_a'N_a'N_a'N_a'N_a'\text{-}3'$,

$5'\text{-}V'N_b'N,'N,'N_a'N_b'N_a'N_a'N_a'N_a'N_a'N_b'N_a'N_b'N_a'N_b'N_a'N_a'N_a'N_a'N_a'\text{-}3'$,

or

$5'\text{-}V'N_b'N,'N,'N_a'N_a'N_a'N_a'N_a'N_a'N_a'N_b'N_a'N_b'N_a'N_a'N_a'N_a'N_a'N_a'N_a'\text{-}3'$,

wherein each X' is independently $N_a'$ or $N_b'$; $N_a'$ is a 2'-methoxy-modified nucleotide, and $N_b'$ is a 2'-fluoro-modified nucleotide; W' represents a nucleotide comprising a chemical modification represented by formula (I), (I-1), or (I-2) or a pharmaceutically acceptable salt thereof; V' represents a chemically modified nucleotide represented by formula (II).

7. The dsRNA according to any one of claims 1-6, wherein at least one phosphodiester group in the sense strand and/or the antisense strand is a phosphodiester group with a modification group, preferably a phosphorothioate diester group.

8. The dsRNA according to claim 7, wherein the phosphorothioate diester group is present at at least one of the following positions:

between the first and second nucleotides of the 5' end of the sense strand;
between the second and third nucleotides of the 5' end of the sense strand;
between the first and second nucleotides of the 3' end of the sense strand;
between the first and second nucleotides of the 5' end of the antisense strand;
between the second and third nucleotides of the 5' end of the antisense strand;
between the first and second nucleotides of the 3' end of the antisense strand; and
between the second and third nucleotides of the 3' end of the antisense strand;
preferably, the sense strand and/or the antisense strand comprise(s) a plurality of phosphorothioate diester groups, and the phosphorothioate diester groups are present:

between the first and second nucleotides of the 5' end of the sense strand; and
between the second and third nucleotides of the 5' end of the sense strand; and
between the first and second nucleotides of the 3' end of the sense strand; and
between the first and second nucleotides of the 5' end of the antisense strand; and
between the second and third nucleotides of the 5' end of the antisense strand; and
between the first and second nucleotides of the 3' end of the antisense strand; and
between the second and third nucleotides of the 3' end of the antisense strand; or
the sense strand and/or the antisense strand comprise(s) a plurality of phosphorothioate diester groups, and the phosphorothioate diester groups are present:

between the first and second nucleotides of the 5' end of the sense strand; and
between the second and third nucleotides of the 5' end of the sense strand; and
between the first and second nucleotides of the 5' end of the antisense strand; and
between the second and third nucleotides of the 5' end of the antisense strand; and
between the first and second nucleotides of the 3' end of the antisense strand; and
between the second and third nucleotides of the 3' end of the antisense strand.

9. The dsRNA according to any one of claims 1-8, wherein:

the sense strand comprises the nucleotide sequence set forth in SEQ ID NO: 6, and the antisense strand comprises the nucleotide sequence set forth in any one of SEQ ID NO: 16, SEQ ID NO: 20, SEQ ID NO: 22 to SEQ ID NO: 33, and SEQ ID NO: 35; or
the sense strand comprises the nucleotide sequence set forth in SEQ ID NO: 7, and the antisense strand comprises the nucleotide sequence set forth in any one of SEQ ID NO: 9 to SEQ ID NO: 20 and SEQ ID NO: 33 to SEQ ID NO: 35.

10. A dsRNA conjugate, comprising:

the dsRNA according to any one of claims 1 to 9, and
a targeting ligand linked to an end of the dsRNA,
wherein preferably, the targeting ligand is linked to the 3' end of the sense strand of the dsRNA.

11. The dsRNA conjugate according to claim 10, wherein:

the targeting ligand comprises at least one targeting moiety, and
the targeting moiety is independently selected from the group consisting of: galactose, galactosamine, N-formyl-galactosamine, N-acetyl-galactosamine, N-propionyl-galactosamine, N-n-butyryl-galactosamine, and N-isobutyryl-galactosamine;
preferably, the targeting moiety is N-acetyl-galactosamine;
more preferably, the targeting ligand comprises three identical or different targeting moieties.

**12.** The dsRNA conjugate according to claim 11, wherein the targeting ligand is the compound represented by formula (III-1) or (III-2) or a pharmaceutically acceptable salt thereof, wherein:

the formula (III-1) is:

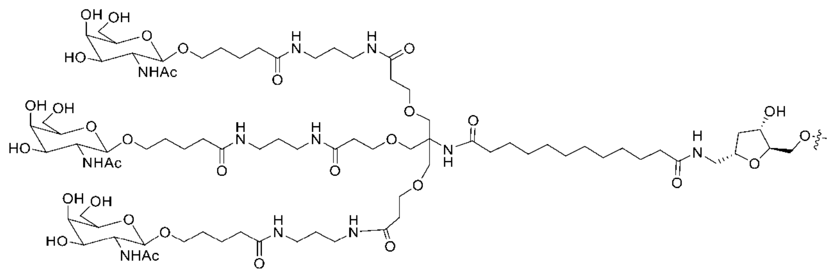

(III-1);

the formula (III-2) is:

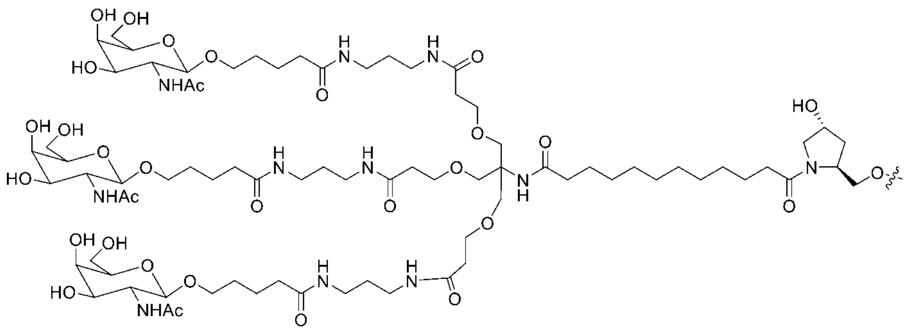

(III-2).

**13.** The dsRNA conjugate according to any one of claims 10-12, wherein the ligand is linked to the dsRNA by a phosphodiester group or a phosphorothioate diester group, preferably by a phosphodiester group.

**14.** The dsRNA conjugate according to any one of claims 10-13, wherein:

the sense strand comprises the nucleotide sequence set forth in SEQ ID NO: 3, and the antisense strand comprises the nucleotide sequence set forth in any one of SEQ ID NO: 16, SEQ ID NO: 20, SEQ ID NO: 22 to SEQ ID NO: 33, and SEQ ID NO: 35; or
the sense strand comprises the nucleotide sequence set forth in SEQ ID NO: 4, and the antisense strand comprises the nucleotide sequence set forth in any one of SEQ ID NO: 9 to SEQ ID NO: 20 and SEQ ID NO: 33 to SEQ ID NO: 35; or
the sense strand comprises the nucleotide sequence set forth in SEQ ID NO: 5, and the antisense strand comprises the nucleotide sequence set forth in any one of SEQ ID NO: 16, SEQ ID NO: 20, and SEQ ID NO: 33.

**15.** The dsRNA conjugate according to any one of claims 10-14, wherein the dsRNA conjugate is selected from the group consisting of the following structure and a pharmaceutically acceptable salt thereof:

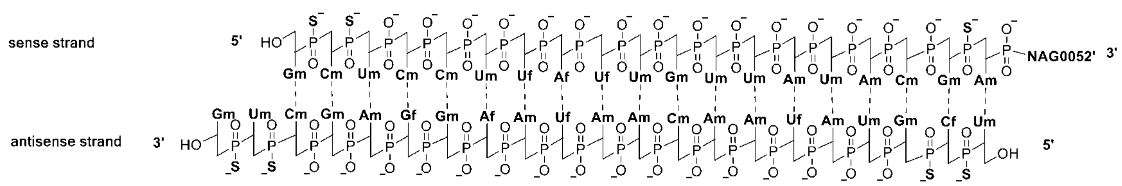

wherein:

Af = adenine 2'-F ribonucleoside;
Cf = cytosine 2'-F ribonucleoside;
Uf = uracil 2'-F ribonucleoside;

Gf = guanine 2'-F ribonucleoside;
Am = adenine 2'-OMe ribonucleoside;
Cm = cytosine 2'-OMe ribonucleoside;
Gm = guanine 2'-OMe ribonucleoside;
Um = uracil 2'-OMe ribonucleoside;

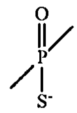

represents an anionic form of a phosphorothioate diester group;

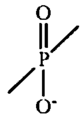

represents an anionic form of a phosphodiester group;
NAG0052' represents

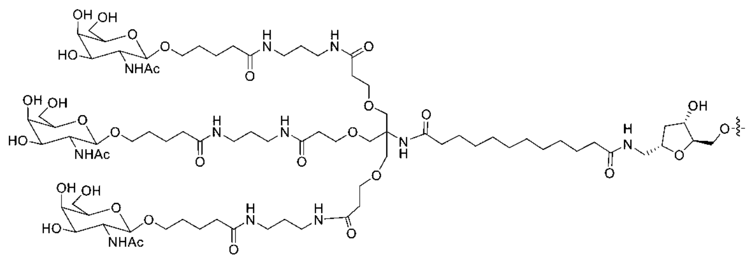

.

**16.** A pharmaceutical composition, comprising:

the dsRNA according to any one of claims 1-9 and/or the dsRNA conjugate according to any one of claims 10-15, wherein preferably, the pharmaceutical composition further comprises one or more pharmaceutically acceptable excipients.

**17.** Use of the dsRNA according to any one of claims 1-9 and/or the dsRNA conjugate according to any one of claims 10-15 and/or the pharmaceutical composition according to claim 16 in the manufacture of a medicament, wherein:

the medicament is used for preventing and/or treating a cardiovascular disease, or
the medicament is used for preventing and/or treating a disease associated with elevated lipoprotein(a) and/or apolipoprotein(a) levels;
preferably, the disease associated with elevated lipoprotein(a) and/or apolipoprotein(a) levels is selected from the group consisting of: a cardiovascular disease;
the cardiovascular disease is selected from the group consisting of: ischemic stroke, atherosclerosis, thrombosis, coronary heart disease, lower extremity arterial disease, aortic stenosis, myocardial infarction, coronary stenosis, carotid stenosis, femoral artery stenosis, and heart failure.

**18.** A method for inhibiting LPA expression, comprising administering to a subject an effective amount or effective dose of the dsRNA according to any one of claims 1-9 and/or the dsRNA conjugate according to any one of claims 10-15 and/or the pharmaceutical composition according to claim 16.

**19.** A method for delivering a dsRNA to the liver *in vivo* to inhibit LPA expression and/or replication, comprising administering to a subject an effective amount or effective dose of the dsRNA according to any one of claims 1-9 and/or the dsRNA conjugate according to any one of claims 10-15 and/or the pharmaceutical composition according to claim 16.

**20.** A cell, comprising the dsRNA according to any one of claims 1-9 and/or the dsRNA conjugate according to any one of

claims 10-15 and/or the pharmaceutical composition according to claim 16.

**21.** A kit, comprising the dsRNA according to any one of claims 1-9 and/or the dsRNA conjugate according to any one of claims 10-15 and/or the pharmaceutical composition according to claim 16.

**22.** A method for preparing a dsRNA, a dsRNA conjugate, or a pharmaceutical composition, comprising:

synthesizing the dsRNA according to any one of claims 1-9 and/or the dsRNA conjugate according to any one of claims 10-15; and/or
formulating the pharmaceutical composition according to claim 16.

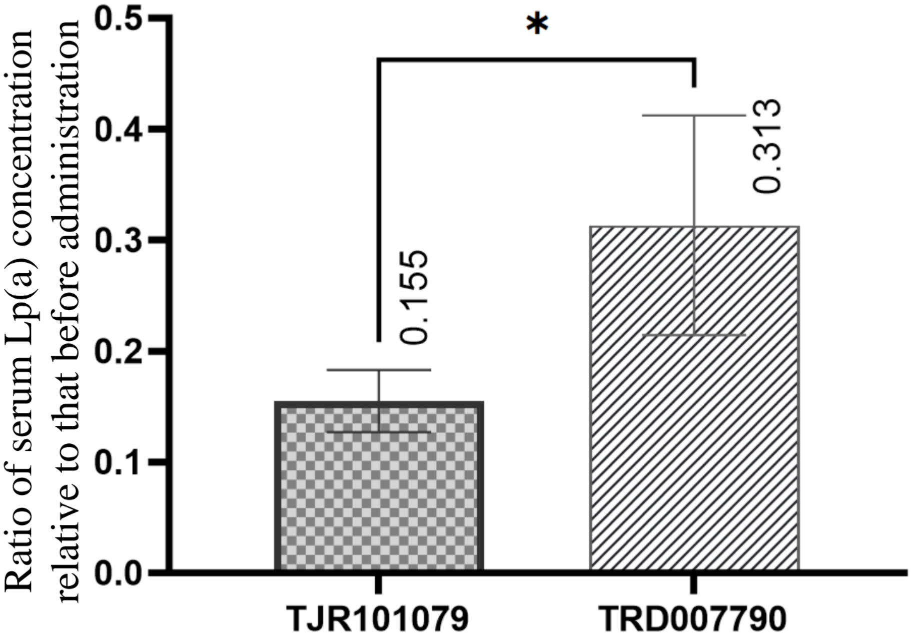
Ratio of serum Lp(a) concentration relative to that before administration
0.5
0.4
0.3
0.2
0.1
0.0
*
0.155
0.313
TJR101079
TRD007790

FIG. 1A

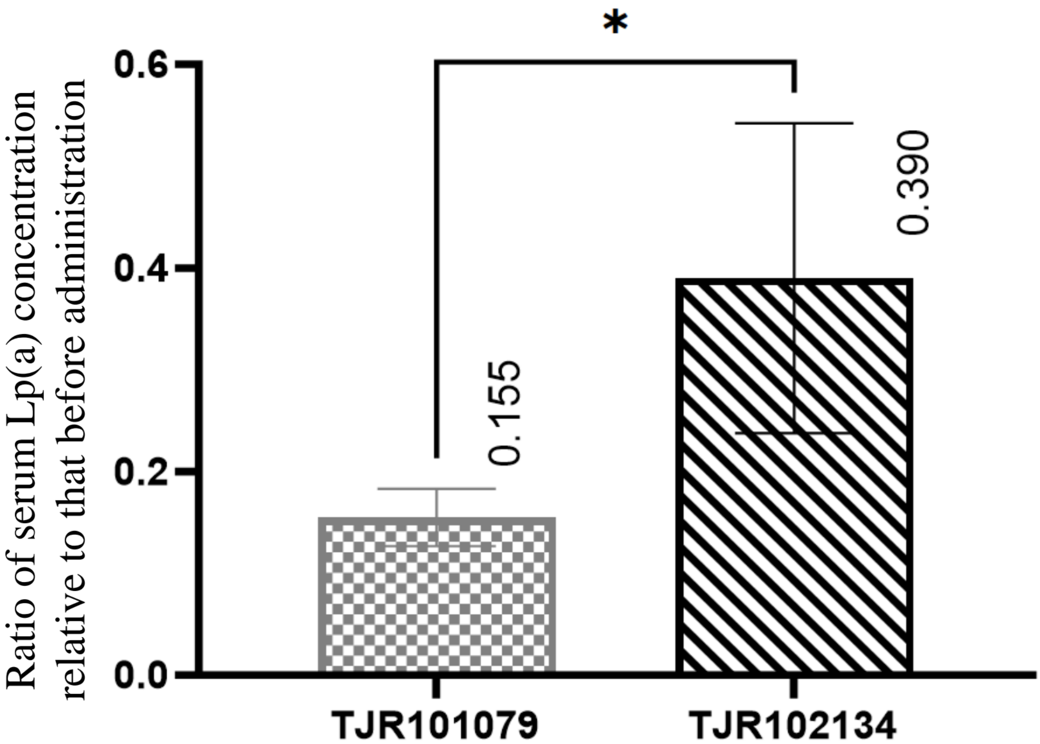
Ratio of serum Lp(a) concentration relative to that before administration
0.6
0.4
0.2
0.0
*
0.155
0.390
TJR101079
TJR102134

FIG. 1B

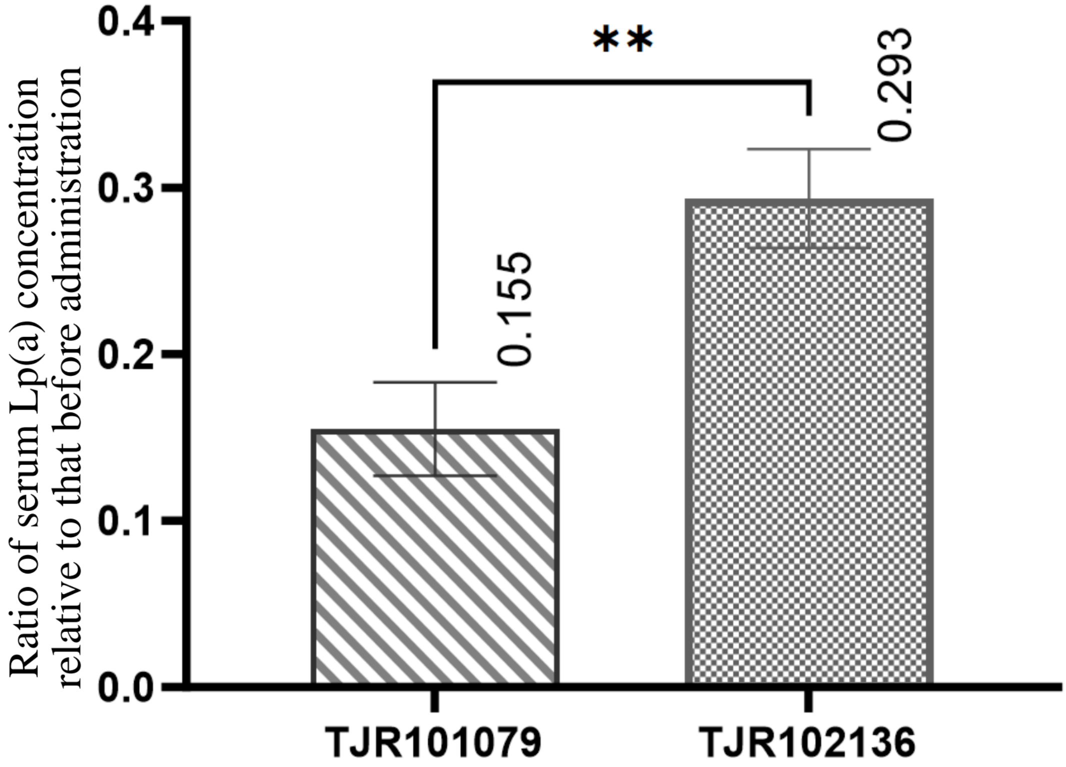
Ratio of serum Lp(a) concentration relative to that before administration
0.4
0.3
0.2
0.1
0.0
**
0.155
0.293
TJR101079
TJR102136

FIG. 1C

**INTERNATIONAL SEARCH REPORT**

International application No.
**PCT/CN2024/096731**

**A. CLASSIFICATION OF SUBJECT MATTER**

A61K 47/54 (2017.01)i; C12N15/113(2010.01)i; A61K31/713(2006.01)i; C12N15/11(2006.01)i; C07K14/47(2006.01)i

According to International Patent Classification (IPC) or to both national classification and IPC

**B. FIELDS SEARCHED**

Minimum documentation searched (classification system followed by classification symbols)

IPC: A61K,C12N,C07K

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, JPTXT, USTXT, EPTXT, 万方, WANFANG, CNKI, PubMed, GenBank, ISI web of knowledge, 中国专利序列数据库, National Sequence Database of Chinese Patent: 脂蛋白(a), 双链核糖核酸, 干扰, 表达, 复制, 2'-氟代修饰, 2'-甲氧基修饰, 硫代磷酸二酯基, Lipoprotein (a), double-stranded ribonucleic acid, interference, expression, replication, 2'-fluoro modification, 2'-methoxy modification, phosphorothioate group, LPA, dsRNA

**C. DOCUMENTS CONSIDERED TO BE RELEVANT**

| Category* | Citation of document, with indication, where appropriate, of the relevant passages | Relevant to claim No. |
|---|---|---|
| A | US 2021292756 A1 (ALNYLAM PHARMACEUTICALS, INC.) 23 September 2021 (2021-09-23) entire document | 1-22 |
| A | WO 2019100039 A1 (ALNYLAM PHARMACEUTICALS, INC.) 23 May 2019 (2019-05-23) entire document | 1-22 |
| A | CN 112876534 A (SUZHOU RIBO LIFE SCIENCE CO., LTD.) 01 June 2021 (2021-06-01) entire document | 1-22 |
| A | CN 112759620 A (SUZHOU RIBO LIFE SCIENCE CO., LTD.) 07 May 2021 (2021-05-07) entire document | 1-22 |
| A | WO 2017059223 A2 (ARROWHEAD PHARMACEUTICALS, INC.) 06 April 2017 (2017-04-06) entire document | 1-22 |
| A | CN 115955973 A (TUOJIE BIOTECH (SHANGHAI) CO., LTD.) 11 April 2023 (2023-04-11) entire document | 1-22 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 August 2024** | **22 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/096731**

**C. DOCUMENTS CONSIDERED TO BE RELEVANT**

| Category* | Citation of document, with indication, where appropriate, of the relevant passages | Relevant to claim No. |
|---|---|---|
| A | CN 108064162 A (IONIS PHARMACEUTICALS, INC.) 22 May 2018 (2018-05-22) entire document | 1-22 |

Form PCT/ISA/210 (second sheet) (July 2022)

**INTERNATIONAL SEARCH REPORT**

International application No.
**PCT/CN2024/096731**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/096731**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18-19**
because they relate to subject matter not required to be searched by this Authority, namely:

Claims 18-19 relate to a method for treatment or diagnosis of a living human or animal body, which falls within the cases set out in PCT Rule 39.1(iv) for which no international search is required. In the present report, a search is carried out on the basis of "the use in the preparation of a drug for inhibiting LPA expression and/or replication".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/096731**

| Patent document cited in search report | Publication date (day/month/year) | Patent family member(s) | Publication date (day/month/year) |
|---|---|---|---|
| US 2021292756 A1 | 23 September 2021 | IL 295496 A | 01 October 2022 |
| | | MX 2022010052 A | 05 September 2022 |
| | | CA 3171654 A1 | 26 August 2021 |
| | | JP 2023514336 A | 05 April 2023 |
| | | US 11162103 B2 | 02 November 2021 |
| | | EP 4107265 A1 | 28 December 2022 |
| | | BR 112022016324 A2 | 11 October 2022 |
| | | KR 20220143106 A | 24 October 2022 |
| | | WO 2021167841 A1 | 26 August 2021 |
| | | US 2022042014 A1 | 10 February 2022 |
| | | TW 202144571 A | 01 December 2021 |
| | | AU 2021224778 A1 | 29 September 2022 |
| WO 2019100039 A1 | 23 May 2019 | EP 3714054 A1 | 30 September 2020 |
| | | US 2020345758 A1 | 05 November 2020 |
| | | US 11229663 B2 | 25 January 2022 |
| | | US 2022305045 A1 | 29 September 2022 |
| CN 112876534 A | 01 June 2021 | None | |
| CN 112759620 A | 07 May 2021 | None | |
| WO 2017059223 A2 | 06 April 2017 | MX 2018003833 A | 18 June 2018 |
| | | HK 1259063 A1 | 22 November 2019 |
| | | MY 195796 A | 21 February 2023 |
| | | TN 2018000094 A1 | 08 July 2019 |
| | | HUE 055942 T2 | 28 January 2022 |
| | | UY 36926 A | 28 April 2017 |
| | | ZA 202106265 B | 30 August 2023 |
| | | CY 1125263 T1 | 24 March 2023 |
| | | CA 3000397 A1 | 06 April 2017 |
| | | EA 201890864 A1 | 28 September 2018 |
| | | EA 038478 B1 | 03 September 2021 |
| | | IL 300438 A | 01 April 2023 |
| | | TW 202332769 A | 16 August 2023 |
| | | TWI 836693 B | 21 March 2024 |
| | | PE 20181139 A1 | 17 July 2018 |
| | | CR 20180231 A | 31 May 2018 |
| | | ES 2896298 T3 | 24 February 2022 |
| | | IL 258333 A | 31 May 2018 |
| | | IL 258333 B | 01 March 2022 |
| | | US 2020263179 A1 | 20 August 2020 |
| | | WO 2017059223 A3 | 11 May 2017 |
| | | WO 2017059223 A9 | 17 August 2017 |
| | | HRP 20211410 T1 | 24 December 2021 |
| | | EP 3356529 A2 | 08 August 2018 |
| | | EP 3356529 A4 | 04 September 2019 |
| | | EP 3356529 B1 | 25 August 2021 |
| | | SG 10202008530 TA | 29 October 2020 |
| | | EP 4029941 A1 | 20 July 2022 |
| | | IL 290566 A | 01 April 2022 |
| | | IL 290566 B1 | 01 March 2023 |
| | | IL 290566 B2 | 01 July 2023 |
| | | RS 62523 B1 | 30 November 2021 |

Form PCT/ISA/210 (patent family annex) (July 2022)

**INTERNATIONAL SEARCH REPORT**
**Information on patent family members**

International application No.
**PCT/CN2024/096731**

| Patent document cited in search report | Publication date (day/month/year) | Patent family member(s) | Publication date (day/month/year) |
|---|---|---|---|
| | | US 2017096665 A1 | 06 April 2017 |
| | | US 9932586 B2 | 03 April 2018 |
| | | US 2018195070 A1 | 12 July 2018 |
| | | US 10662427 B2 | 26 May 2020 |
| | | KR 20180052703 A | 18 May 2018 |
| | | LT 3356529 T | 27 December 2021 |
| | | TW 201726918 A | 01 August 2017 |
| | | TWI 784934 B | 01 December 2022 |
| | | MA 43347 A | 08 August 2018 |
| | | MA 43347 B1 | 30 November 2021 |
| | | JP 2021087459 A | 10 June 2021 |
| | | JP 7116212 B2 | 09 August 2022 |
| | | UA 121998 C2 | 25 August 2020 |
| | | BR 112018006489 A2 | 09 October 2018 |
| | | CO 2018003678 A2 | 30 November 2018 |
| | | JP 2018529732 A | 11 October 2018 |
| | | JP 6991966 B2 | 03 February 2022 |
| | | MX 2022013010 A | 09 November 2022 |
| | | PH 12018500713 A1 | 15 October 2018 |
| | | JP 2024009262 A | 19 January 2024 |
| | | AU 2022283623 A1 | 02 February 2023 |
| | | SI 3356529 T1 | 31 January 2022 |
| | | JP 2022113835 A | 04 August 2022 |
| | | JP 7442574 B2 | 04 March 2024 |
| | | DK 3356529 T3 | 08 November 2021 |
| | | JOP 20210043 A1 | 16 June 2017 |
| | | PL 3356529 T3 | 20 December 2021 |
| | | AU 2016331084 A1 | 19 April 2018 |
| | | AU 2016331084 B2 | 08 September 2022 |
| | | PT 3356529 T | 04 November 2021 |
| | | NZ 741086 A | 24 November 2023 |
| | | JOP 20160211 B1 | 17 August 2021 |
| | | CL 2018000803 A1 | 31 August 2018 |
| CN 115955973 A | 11 April 2023 | EP 4194553 A1 | 14 June 2023 |
| | | TW 202214858 A | 16 April 2022 |
| | | JP 2023536320 A | 24 August 2023 |
| | | US 2023287418 A1 | 14 September 2023 |
| | | WO 2022028462 A1 | 10 February 2022 |
| | | KR 20230043195 A | 30 March 2023 |
| | | BR 112023002080 A2 | 25 April 2023 |
| | | CA 3190097 A1 | 10 February 2022 |
| CN 108064162 A | 22 May 2018 | RU 2019124314 A | 21 August 2019 |
| | | MX 2020004209 A | 13 August 2020 |
| | | MX 2019010441 A | 17 October 2019 |
| | | RS 60796 B1 | 30 October 2020 |
| | | JP 2016526874 A | 08 September 2016 |
| | | JP 6387084 B2 | 05 September 2018 |
| | | JP 2020058370 A | 16 April 2020 |
| | | WO 2014179620 A1 | 06 November 2014 |
| | | US 2016076032 A1 | 17 March 2016 |

Form PCT/ISA/210 (patent family annex) (July 2022)

**INTERNATIONAL SEARCH REPORT**
**Information on patent family members**

International application No.
**PCT/CN2024/096731**

| Patent document cited in search report | Publication date (day/month/year) | Patent family member(s) | Publication date (day/month/year) |
|---|---|---|---|
| | | US 9714421 B2 | 25 July 2017 |
| | | IL 261901 A | 31 October 2018 |
| | | IL 261901 B | 31 May 2020 |
| | | KR 20230006933 A | 11 January 2023 |
| | | ES 2778442 T3 | 10 August 2020 |
| | | EA 201891479 A1 | 30 November 2018 |
| | | EA 036584 B1 | 26 November 2020 |
| | | IL 263843 A | 31 January 2019 |
| | | IL 263843 B | 31 March 2020 |
| | | KR 20190084138 A | 15 July 2019 |
| | | KR 102212275 B1 | 05 February 2021 |
| | | US 2018273953 A1 | 27 September 2018 |
| | | US 10883104 B2 | 05 January 2021 |
| | | US 2016017323 A1 | 21 January 2016 |
| | | AU 2017200365 A1 | 23 February 2017 |
| | | AU 2017200365 B2 | 08 November 2018 |
| | | AU 2017200365 C1 | 18 April 2019 |
| | | EP 2991656 A2 | 09 March 2016 |
| | | EP 2991656 A4 | 22 February 2017 |
| | | EP 2991656 B1 | 18 December 2019 |
| | | HRP 20201378 T1 | 27 November 2020 |
| | | JP 2016523515 A | 12 August 2016 |
| | | JP 6456362 B2 | 23 January 2019 |
| | | JP 2021020901 A | 18 February 2021 |
| | | JP 7177127 B2 | 22 November 2022 |
| | | US 2023151365 A1 | 18 May 2023 |
| | | IL 242124 B | 28 February 2019 |
| | | US 2015176007 A1 | 25 June 2015 |
| | | US 9145558 B2 | 29 September 2015 |
| | | EP 3633039 A1 | 08 April 2020 |
| | | HUE 050394 T2 | 30 November 2020 |
| | | IL 296543 A | 01 November 2022 |
| | | PT 2992098 T | 05 July 2019 |
| | | US 2024247260 A1 | 25 July 2024 |
| | | AU 2014259759 A1 | 22 October 2015 |
| | | AU 2014259759 B2 | 18 June 2020 |
| | | IL 274064 A | 30 June 2020 |
| | | IL 274064 B | 30 June 2021 |
| | | US 2021395734 A1 | 23 December 2021 |
| | | IL 242132 B | 31 October 2018 |
| | | US 2021130823 A1 | 06 May 2021 |
| | | LT 2992098 T | 10 July 2019 |
| | | CA 2921162 A1 | 06 November 2014 |
| | | AU 2014259757 A1 | 22 October 2015 |
| | | AU 2014259757 B2 | 02 March 2017 |
| | | US 2018273952 A1 | 27 September 2018 |
| | | US 10927372 B2 | 23 February 2021 |
| | | IL 264241 A | 28 February 2019 |
| | | IL 264241 B | 30 April 2020 |
| | | HK 1221404 A1 | 02 June 2017 |

Form PCT/ISA/210 (patent family annex) (July 2022)

**INTERNATIONAL SEARCH REPORT**
**Information on patent family members**

International application No.
**PCT/CN2024/096731**

| Patent document cited in search report | Publication date (day/month/year) | Patent family member(s) | Publication date (day/month/year) |
|---|---|---|---|
| | | JP 2023113843 A | 16 August 2023 |
| | | AU 2020207820 A1 | 06 August 2020 |
| | | UA 120287 C2 | 11 November 2019 |
| | | JP 2020039355 A | 19 March 2020 |
| | | AU 2022202770 A1 | 19 May 2022 |
| | | AU 2014259755 A1 | 22 October 2015 |
| | | AU 2014259755 B2 | 30 August 2018 |
| | | KR 20210151260 A | 13 December 2021 |
| | | KR 102558571 B1 | 21 July 2023 |
| | | MY 178929 A | 23 October 2020 |
| | | CR 20190269 A | 13 September 2019 |
| | | US 2020224198 A1 | 16 July 2020 |
| | | US 2016076030 A1 | 17 March 2016 |
| | | US 9932580 B2 | 03 April 2018 |
| | | AU 2019200820 A1 | 28 February 2019 |
| | | AU 2019200820 B2 | 30 April 2020 |
| | | PT 3524680 T | 04 January 2021 |
| | | ES 2819213 T3 | 15 April 2021 |
| | | EP 2992009 A1 | 09 March 2016 |
| | | EP 2992009 A4 | 28 December 2016 |
| | | EP 2992009 B1 | 24 June 2020 |
| | | MX 2015015263 A | 16 December 2016 |
| | | HK 1221486 A1 | 02 June 2017 |
| | | AU 2019203674 A1 | 27 June 2019 |
| | | AU 2019203674 B2 | 25 March 2021 |
| | | SG 11201508800 WA | 27 November 2015 |
| | | CL 2015003217 A1 | 08 July 2016 |
| | | JP 2020007361 A | 16 January 2020 |
| | | JP 7429103 B2 | 07 February 2024 |
| | | AU 2018267625 A1 | 13 December 2018 |
| | | AU 2018267625 B2 | 10 September 2020 |
| | | NZ 740338 A | 29 April 2022 |
| | | MX 2020002184 A | 14 July 2020 |
| | | RU 2018136140 A | 17 December 2018 |
| | | RU 2018136140 A3 | 27 April 2022 |
| | | IL 264580 A | 28 February 2019 |
| | | IL 264580 B | 30 April 2020 |
| | | BR 112015027319 A2 | 26 September 2017 |
| | | BR 112015027319 A8 | 02 January 2018 |
| | | CY 1123369 T1 | 31 December 2021 |
| | | HUE 043697 T2 | 30 September 2019 |
| | | US 2021024923 A1 | 28 January 2021 |
| | | US 2022275365 A9 | 01 September 2022 |
| | | HK 1221475 A1 | 02 June 2017 |
| | | KR 20210014758 A | 09 February 2021 |
| | | DOP 2021000095 A | 15 September 2021 |
| | | IL 273184 A | 30 April 2020 |
| | | IL 273184 B | 29 July 2021 |
| | | PE 20152002 A1 | 21 January 2016 |
| | | EP 2992097 A2 | 09 March 2016 |

Form PCT/ISA/210 (patent family annex) (July 2022)

**INTERNATIONAL SEARCH REPORT**
**Information on patent family members**

International application No.
**PCT/CN2024/096731**

| Patent document cited in search report | Publication date (day/month/year) | Patent family member(s) | Publication date (day/month/year) |
|---|---|---|---|
| | | EP 2992097 A4 | 04 January 2017 |
| | | EP 2992097 B1 | 06 November 2019 |
| | | CL 2016002262 A1 | 09 June 2017 |
| | | IL 273205 A | 30 April 2020 |
| | | BR 112015027321 A2 | 26 September 2017 |
| | | BR 112015027321 A8 | 02 January 2018 |
| | | WO 2014179629 A2 | 06 November 2014 |
| | | WO 2014179629 A3 | 22 January 2015 |
| | | WO 2014179629 A8 | 02 June 2016 |
| | | EP 2991661 A1 | 09 March 2016 |
| | | EP 2991661 A4 | 15 February 2017 |
| | | EP 2991661 B1 | 13 March 2019 |
| | | RU 2015151203 A | 02 June 2017 |
| | | RU 2650510 C2 | 16 April 2018 |
| | | NZ 631512 A | 28 October 2016 |
| | | CY 1121879 T1 | 14 October 2020 |
| | | SG 11201508870 VA | 27 November 2015 |
| | | KR 20160002976 A | 08 January 2016 |
| | | KR 102315836 B1 | 22 October 2021 |
| | | EP 3828275 A1 | 02 June 2021 |
| | | IL 283660 A | 29 July 2021 |
| | | KR 20180051678 A | 16 May 2018 |
| | | KR 102138781 B1 | 28 July 2020 |
| | | AU 2017203436 A1 | 08 June 2017 |
| | | AU 2017203436 B2 | 18 October 2018 |
| | | PH 12015502493 A1 | 22 February 2016 |
| | | ES 2730015 T3 | 07 November 2019 |
| | | IL 242125 B | 28 February 2019 |
| | | HK 1221403 A1 | 02 June 2017 |
| | | KR 20220108195 A | 02 August 2022 |
| | | KR 102651423 B1 | 27 March 2024 |
| | | ME 03390 B | 20 January 2020 |
| | | RU 2015151199 A | 05 June 2017 |
| | | RU 2015151199 A3 | 27 March 2018 |
| | | RU 2697152 C2 | 12 August 2019 |
| | | PE 20161430 A1 | 06 January 2017 |
| | | DK 3524680 T3 | 14 December 2020 |
| | | MX 2015015234 A | 03 October 2016 |
| | | SG 10201801507 RA | 28 March 2018 |
| | | NZ 753018 A | 28 January 2022 |
| | | EP 2992098 A2 | 09 March 2016 |
| | | EP 2992098 A4 | 11 January 2017 |
| | | EP 2992098 B1 | 27 March 2019 |
| | | JP 2018027091 A | 22 February 2018 |
| | | JP 6592486 B2 | 16 October 2019 |
| | | WO 2014179627 A2 | 06 November 2014 |
| | | WO 2014179627 A9 | 26 February 2015 |
| | | WO 2014179627 A3 | 16 April 2015 |
| | | JP 2021107408 A | 29 July 2021 |
| | | JP 7339294 B2 | 05 September 2023 |

Form PCT/ISA/210 (patent family annex) (July 2022)

**INTERNATIONAL SEARCH REPORT**
**Information on patent family members**

International application No.
**PCT/CN2024/096731**

| Patent document cited in search report | Publication date (day/month/year) | Patent family member(s) | Publication date (day/month/year) |
|---|---|---|---|
| | | JP 2019056001 A | 11 April 2019 |
| | | JP 6639629 B2 | 05 February 2020 |
| | | JP 2016522817 A | 04 August 2016 |
| | | JP 6216444 B2 | 18 October 2017 |
| | | BR 112015027369 A2 | 26 September 2017 |
| | | BR 112015027369 A8 | 02 January 2018 |
| | | BR 112015027369 B1 | 08 June 2021 |
| | | US 2016090596 A1 | 31 March 2016 |
| | | US 9957504 B2 | 01 May 2018 |
| | | NZ 725538 A | 26 February 2021 |
| | | PL 2992098 T3 | 30 September 2019 |
| | | JP 2020074787 A | 21 May 2020 |
| | | DOP 2016000287 A | 15 February 2017 |
| | | DK 2992098 T3 | 17 June 2019 |
| | | JP 2016522683 A | 04 August 2016 |
| | | JP 6995478 B2 | 14 January 2022 |
| | | US 2018044676 A1 | 15 February 2018 |
| | | US 10683499 B2 | 16 June 2020 |
| | | WO 2014179626 A2 | 06 November 2014 |
| | | WO 2014179626 A3 | 26 February 2015 |
| | | CA 2921514 A1 | 06 November 2014 |
| | | CA 2921514 C | 24 October 2023 |
| | | SG 10201906382 QA | 27 August 2019 |
| | | RU 2018112167 A | 07 March 2019 |
| | | JP 2018183184 A | 22 November 2018 |
| | | JP 6652602 B2 | 26 February 2020 |
| | | AU 2021204244 A1 | 22 July 2021 |
| | | AU 2021204244 B2 | 19 October 2023 |
| | | SI 2992009 T1 | 30 October 2020 |
| | | US 2015126719 A1 | 07 May 2015 |
| | | US 9163239 B2 | 20 October 2015 |
| | | NZ 631552 A | 24 February 2017 |
| | | ZA 201507218 B | 27 September 2023 |
| | | IL 273312 A | 30 April 2020 |
| | | US 2016090595 A1 | 31 March 2016 |
| | | US 9932581 B2 | 03 April 2018 |
| | | WO 2014179625 A1 | 06 November 2014 |
| | | MY 198359 A | 28 August 2023 |
| | | NZ 712737 A | 27 August 2021 |
| | | JP 2023012548 A | 25 January 2023 |
| | | KR 20240042220 A | 01 April 2024 |
| | | US 2014343123 A1 | 20 November 2014 |
| | | US 9127276 B2 | 08 September 2015 |
| | | AU 2017200950 A1 | 02 March 2017 |
| | | AU 2017200950 B2 | 17 January 2019 |
| | | EP 3524680 A1 | 14 August 2019 |
| | | EP 3524680 B1 | 11 November 2020 |
| | | CA 2921518 A1 | 06 November 2014 |
| | | ZA 201507216 B | 30 August 2017 |
| | | NZ 728517 A | 24 December 2021 |

Form PCT/ISA/210 (patent family annex) (July 2022)

**INTERNATIONAL SEARCH REPORT**
**Information on patent family members**

International application No.
**PCT/CN2024/096731**

| Patent document cited in search report | Publication date (day/month/year) | Patent family member(s) | | | Publication date (day/month/year) |
|---|---|---|---|---|---|
| | | US | 2018002693 | A1 | 04 January 2018 |
| | | PH | 12019501191 | A1 | 01 March 2021 |
| | | MX | 2021008901 | A | 19 August 2021 |
| | | IL | 272617 | A | 31 March 2020 |
| | | ES | 2885174 | T3 | 13 December 2021 |
| | | US | 2015126720 | A1 | 07 May 2015 |
| | | US | 9181550 | B2 | 10 November 2015 |
| | | JP | 2020039354 | A | 19 March 2020 |
| | | JP | 6866459 | B2 | 28 April 2021 |
| | | AU | 2014259756 | A1 | 22 October 2015 |
| | | AU | 2014259756 | B2 | 23 February 2017 |
| | | MX | 2021008899 | A | 19 August 2021 |
| | | EP | 3690049 | A1 | 05 August 2020 |
| | | UA | 121017 | C2 | 25 March 2020 |
| | | BR | 112015027377 | A2 | 29 August 2017 |
| | | BR | 112015027377 | A8 | 03 October 2017 |
| | | BR | 112015027377 | B1 | 10 January 2023 |
| | | MX | 2015015264 | A | 12 August 2016 |
| | | US | 2019367914 | A1 | 05 December 2019 |
| | | US | 10844379 | B2 | 24 November 2020 |
| | | KR | 20210037752 | A | 06 April 2021 |
| | | JP | 2022017514 | A | 25 January 2022 |
| | | AU | 2024200296 | A1 | 08 February 2024 |
| | | JP | 2021074021 | A | 20 May 2021 |
| | | AU | 2020233603 | A1 | 01 October 2020 |
| | | CA | 2921167 | A1 | 06 November 2014 |
| | | MX | 2015015220 | A | 12 January 2016 |
| | | DK | 2992009 | T3 | 14 September 2020 |
| | | EA | 201592093 | A1 | 30 June 2016 |
| | | EA | 031393 | B1 | 28 December 2018 |
| | | PL | 2992009 | T3 | 30 November 2020 |
| | | MX | 2015015239 | A | 03 October 2016 |
| | | AU | 2020217347 | A1 | 27 August 2020 |
| | | IL | 242126 | B | 31 January 2019 |
| | | KR | 20200090966 | A | 29 July 2020 |
| | | RU | 2015151204 | A | 02 June 2017 |
| | | RU | 2015151204 | A3 | 27 March 2018 |
| | | RU | 2686080 | C2 | 24 April 2019 |
| | | AU | 2014259750 | A1 | 22 October 2015 |
| | | AU | 2014259750 | B2 | 28 February 2019 |
| | | HK | 1221485 | A1 | 02 June 2017 |
| | | JP | 2024010070 | A | 23 January 2024 |
| | | IL | 284593 | A | 31 August 2021 |
| | | IL | 284593 | B | 01 October 2022 |
| | | IL | 284593 | B2 | 01 February 2023 |
| | | KR | 20160002977 | A | 08 January 2016 |
| | | AU | 2019204784 | A1 | 25 July 2019 |
| | | AU | 2019204784 | B2 | 27 January 2022 |
| | | AU | 2019204784 | C1 | 03 November 2022 |
| | | KR | 20230113835 | A | 01 August 2023 |

Form PCT/ISA/210 (patent family annex) (July 2022)

**INTERNATIONAL SEARCH REPORT**
**Information on patent family members**

International application No.
**PCT/CN2024/096731**

| Patent document cited in search report | Publication date (day/month/year) | Patent family member(s) | Publication date (day/month/year) |
|---|---|---|---|
| | | KR 20160003723 A | 11 January 2016 |
| | | KR 102424855 B1 | 26 July 2022 |
| | | NZ 631537 A | 26 May 2017 |
| | | EP 4155403 A1 | 29 March 2023 |
| | | US 2015126718 A1 | 07 May 2015 |
| | | US 9181549 B2 | 10 November 2015 |
| | | BR 112015027322 A2 | 26 September 2017 |
| | | BR 112015027322 A8 | 02 January 2018 |
| | | PT 2992009 T | 21 September 2020 |
| | | RU 2015151202 A | 06 June 2017 |
| | | RU 2015151202 A3 | 27 March 2018 |
| | | RU 2670614 C2 | 24 October 2018 |
| | | RU 2670614 C9 | 23 November 2018 |
| | | DOP 2015000268 A | 30 November 2015 |
| | | SI 2992098 T1 | 28 June 2019 |
| | | AU 2019202598 A1 | 02 May 2019 |
| | | PH 12018501963 A1 | 20 July 2020 |
| | | CA 2921509 A1 | 06 November 2014 |
| | | CR 20150612 A | 03 March 2016 |
| | | LT 2992009 T | 10 November 2020 |
| | | RU 2019110030 A | 06 May 2019 |
| | | RS 58981 B1 | 30 August 2019 |
| | | IL 284000 A | 29 July 2021 |
| | | US 11299736 B1 | 12 April 2022 |
| | | RU 2015151200 A3 | 14 January 2019 |
| | | RU 2015151200 A | 11 September 2019 |
| | | US 2019055554 A1 | 21 February 2019 |
| | | KR 20160002975 A | 08 January 2016 |
| | | KR 101857707 B1 | 14 May 2018 |
| | | DK 2991656 T3 | 23 March 2020 |
| | | JP 2016526018 A | 01 September 2016 |
| | | JP 6769866 B2 | 14 October 2020 |
| | | KR 20210129257 A | 27 October 2021 |
| | | KR 102482890 B1 | 30 December 2022 |
| | | HRP 20190987 T1 | 20 September 2019 |
| | | US 2021087566 A1 | 25 March 2021 |
| | | US 11851655 B2 | 26 December 2023 |
| | | IL 270464 B | 29 July 2021 |
| | | KR 20160002974 A | 08 January 2016 |
| | | KR 102235678 B1 | 05 April 2021 |
| | | BR 122018009831 B1 | 21 December 2021 |
| | | MX 2019010443 A | 17 October 2019 |
| | | EP 3546579 A1 | 02 October 2019 |
| | | SG 10201801813 YA | 27 April 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- CN 202310628559
- CN 202311386740
- WO 2022028462 A
- WO 2023274395 A
- WO 2023208023 A
- WO 2023109940 A
- WO 2014025805 A
- WO 2017156012 A
- WO 2017059223 A
- US 20190177729 A

### Non-patent literature cited in the description

- **BERG K.** A new serum type system in man-the Lp system.. *Acta Pathol Microbiol Scand*, 1963, vol. 59, 369-82
- **SCHMIDT K** ; **NOUREEN A** ; **KRONENBERG F et al.** Structure, Function, and Genetics of Lipoprotein(a), [J. *Journal of Lipid Research*, 2016, vol. 57 (8), 1339
- **ENAS EA** ; **VARKEY B** ; **DHARMARAJAN T S et al.** Lipoprotein(a): An independent, genetic, and causal factor for cardiovascular disease and acute myocardial infarction[J. *Indian Heart Journal*, 2019, vol. 71 (2)
- **ALBERT YOUNGWOO JANG** ; **SEUNG HWAN HAN** ; **I1 SUK SOHN et al.** Lipoprotein(a) and Cardiovascular Diseases[J. *Circulation Journal*, 2020, vol. 84, 867-874